# EUROPEAN PATENT APPLICATION

(11) **EP 4 137 077 A2**
(43) Date of publication of application: **22.02.2023**
(21) Application number: 22190754.6
(22) Date of filing: 17.08.2022
(51) Int. Cl.: A61B 18/12, A61B 18/00, A61B 90/00

(54) **SYSTEMS FOR TREATING TISSUE BASED ON NAVIGATION INFORMATION**

(30) Priority: 18.08.2021 US 202163234474 P
(71) Applicant: Kardium Inc., Burnaby, BC V5J 0B6 (CA)
(72) Inventor: REINDERS, Daniel Martin, Richmond, V7A 3Y8 (CA); MICHAEL, Justin Aaron, Vancouver, V6Z 2Z5 (CA); GOERTZEN, Douglas Wayne, New Westminster, V3L 4H8 (CA)
(74) Representative: Laqua, Bernd Christian Kurt

(57) **Abstract**

A tissue ablation system may be configured to receive location information indicating locations of at least part of a transducer-based device in a bodily cavity; cause delivery of first tissue-ablative energy during a duration of a first particular time period in accordance with a first energy waveform parameter set at least in response to a first state in which at least part of the location information indicates at least a first rate of movement of the part of the transducer-based device in the bodily cavity; and cause delivery of second tissue-ablative energy during a duration of a second particular time period in accordance with a second energy waveform parameter set at least in response to a second state in which the at least part of the location information indicates at least a second rate of movement of the part of the transducer-based device in the bodily cavity.

## Description

### TECHNICAL FIELD

Aspects of this disclosure generally are related to systems and methods for treating tissue using location information indicating locations of a transducer-based device.

### BACKGROUND

Cardiac surgery was initially undertaken using highly invasive open procedures. A sternotomy, which is a type of incision in the center of the chest that separates the sternum, was typically employed to allow access to the heart. In the past several decades, more and more cardiac operations are performed using intravascular or percutaneous techniques, where access to inner organs or other tissue is gained via a catheter.

Intravascular or percutaneous surgeries benefit patients by reducing surgery risk, complications and recovery time. However, the use of intravascular or percutaneous technologies also raises some particular challenges. Medical devices used in intravascular or percutaneous surgery need to be deployed via catheter systems which significantly increase the complexity of the device structure. As well, doctors do not have direct visual contact with the medical devices once the devices are positioned within the body.

One example of where intravascular or percutaneous medical techniques have been employed is in the treatment of a heart disorder called atrial fibrillation. Atrial fibrillation is a disorder in which spurious electrical signals cause an irregular heartbeat. Atrial fibrillation has been treated with open heart methods using a technique known as the "Cox-Maze procedure". During this procedure, physicians create specific patterns of lesions in the left or right atria to block various paths taken by the spurious electrical signals. Such lesions were originally created using incisions, but are now typically created by ablating the tissue with various techniques including radio-frequency ("RF") energy, microwave energy, laser energy, and cryogenic techniques. Recently, pulsed field ablation ("PFA") techniques have been investigated in various tissue ablation procedures. In PFA, high voltage pulses with sub-second pulse durations are applied to target tissue. In some cases, the high voltage pulses form pores in cell membranes in a procedure sometimes referred to as electroporation. When the electroporation process is such that the formed pores are permanent in nature and consequently results in cell death, the process is referred to as irreversible electroporation by some. When the electroporation process is such that the formed pores are temporary in nature, and the cell survives the electroporation process, the process is referred to as reversible electroporation by some. Pulsed field ablation, because it refers to ablation of tissue, typically involves irreversible electroporation of target tissue. In some cases, PFA shows a specificity for certain tissues. That is, in some cases, PFA may ablate a certain tissue type, but not another tissue type.

The intravascular or percutaneous atrial fibrillation treatment procedure is performed with a high success rate, with or without PFA, despite the lack of direct vision that is provided in open procedures. However, it is relatively complex to perform, because of the difficulty in correctly positioning various catheter devices intravascularly or percutaneously to create the lesions in the correct locations. Various problems, potentially leading to severe adverse results, may occur if the lesions are placed incorrectly or are not formed correctly. For example, if tissue ablation (e.g., RF ablation) is attempted by a transducer in a state in which the transducer is not in sufficient contact with tissue, the ablation procedure may generate thermal coagulum in blood, which may lead to stroke or other harm to the patient. It also is particularly important to know the position of the various transducers which will be creating the lesions relative to various anatomical features (e.g., cardiac features such as the pulmonary veins and mitral valve of a cardiac chamber). The continuity, transmurality, and placement of the lesion patterns that are formed can impact the ability to block paths taken within the heart by spurious electrical signals and, consequently, can impact whether or not the procedure is successful.

Some conventional systems have attempted to address the problem of lack of visibility of an internal medical device associated with percutaneous or intravascular procedures. Some conventional systems rely on fluoroscopic imaging to view the location of an internal medical device or probe, but it has been recognized that such fluoroscopic imaging does not readily produce images of tissue within the bodily cavity in sufficient detail to assess the location or particular degree of tissue contact associated with a particular transducer or to identify particular anatomical landmarks within the bodily cavity. Some conventional systems generate a graphical model of a tissue surface defining a bodily cavity into which a medical device or probe is deployed based on data acquired from electric-potential-based navigation systems, electromagnetic-based navigation systems, or ultrasound-based navigation systems. Some of these conventional navigation systems rely on a three-dimensional ("3D") location of the medical device or probe located in the particular bodily cavity that is to be modeled. Some of these conventional navigation systems may incorporate a user interface employed to show a 3D graphical model of the bodily cavity, which, in some of these conventional systems, is generated via a medical practitioner moving a part of the medical device or probe (which moves a corresponding transducer) from point to point along the tissue wall. Some of these conventional systems may compile this sequence of points and, from such points, build the 3D graphical model of the bodily cavity. This model may be combined with real-time sensing of a location of the medical device or probe to provide the user with an awareness of the location of the medical device or probe in the bodily cavity with improved accuracy over, e.g., mere use of fluoroscopy.

In many procedures, it is desired to form a continuous lesion by positioning a transducer-based catheter device at multiple locations and ablating the tissue at each of the locations, e.g., in order to block a spurious electrophysiological signal from propagating through the tissue wall of the heart. In this regard, it is intended that individual lesions that are formed at the multiple locations combine to form a continuous, transmural lesion, else a spurious electrophysiological signal may escape through the lesion and limit successful treatment of atrial fibrillation.

The present inventors have recognized that positional limitations associated with typical navigation systems can create a tension between ensuring that sufficient ablation energy is transmitted at each of the multiple locations to adequately result in a lesion that is continuous and transmural, while minimizing or otherwise reducing the total energy applied to lower the risk of damage to various anatomical structures proximate the heart, such as the phrenic nerve or esophagus. This unwanted damage to anatomical structures proximate the heart, when it occurs, is typically associated with thermal ablation techniques (e.g., RF ablation). However, while these anatomical structures are conventionally thought to be more resistant to PFA pulses in pulse field ablation, the present inventors have recognized that the use of higher PFA voltage gradients can render these anatomical structures vulnerable. Accordingly, the present inventors have recognized that, even in the context of PFA, it can be important to find the right balance between ensuring the application of sufficient ablation energy to cause a continuous and transmural lesion, while not applying excessive energy that can increase risk of damage to anatomical structures proximate the heart.

For at least these and other reasons, the present inventors have recognized that a need in the art exists for improved methods of forming continuous and transmural tissue lesions.

### SUMMARY

At least the above-discussed need is addressed and technical solutions are achieved in the art by various embodiments of the present invention. According to some embodiments, a tissue ablation system may be summarized as including an input-output device system, a memory device system storing a program, and a data processing device system communicatively connected to the input-output device system and the memory device system. In some embodiments, the data processing device system may be configured at least by the program at least to receive, via the input-output device system, location information indicating locations of at least part of a transducer-based device in a bodily cavity. In some embodiments, the data processing device system may be configured at least by the program at least to cause, via the input-output device system and the transducer-based device, delivery of first tissue-ablative energy during a duration of a first particular time period in accordance with a first energy waveform parameter set at least in response to a first state in which at least part of the location information indicates at least a first rate of movement of the part of the transducer-based device in the bodily cavity. In some embodiments, the first tissue-ablative energy caused to be delivered during the duration of the first particular time period in accordance with the first energy waveform parameter set may be configured to cause tissue ablation. In some embodiments, the data processing device system may be configured at least by the program at least to cause, via the input-output device system and the transducer-based device, delivery of second tissue-ablative energy during a duration of a second particular time period in accordance with a second energy waveform parameter set at least in response to a second state in which the at least part of the location information indicates at least a second rate of movement of the part of the transducer-based device in the bodily cavity. In some embodiments, the second tissue-ablative energy caused to be delivered during the duration of the second particular time period in accordance with the second energy waveform parameter set may be configured to cause tissue ablation, the second energy waveform parameter set different than the first energy waveform parameter set. In some embodiments, the second rate of movement may be different than the first rate of movement.

In some embodiments, the data processing device system may be configured at least by the program at least to determine a rate of movement of the part of the transducer-based device in the bodily cavity based at least on an analysis of the locations indicated by the received location information. In some embodiments, the duration of the first particular time period may be the same as the duration of the second particular time period.

In some embodiments, in the first state, the part of the transducer-based device may move through at least some of the locations during the duration of the first particular time period. In some embodiments, in the second state, the part of the transducer-based device may move through at least some of the locations during the duration of the second particular time period. In some embodiments, in the first state, the part of the transducer-based device may move through at least some of the locations during the duration of the first particular time period with the first rate of movement. In some embodiments, in the second state, the part of the transducer-based device may move through at least some of the locations during the duration of the second particular time period with the second rate of movement.

According to some embodiments, at least in response to the first state, the data processing device system may be configured at least by the program at least to cause delivery of the first tissue-ablative energy via a first plurality of discrete energy application sets during the duration of the first particular time period. In some embodiments, at least in response to the second state, the data processing device system may be configured at least by the program at least to cause delivery of the second tissue-ablative energy via a second plurality of discrete energy application sets during the duration of the second particular time period. In some embodiments, the first energy waveform parameter set may define one or more first parameters applicable to each discrete energy application set in the first plurality of discrete energy application sets, and the second energy waveform parameter set may define one or more second parameters applicable to each discrete energy application set in the second plurality of discrete energy application sets. In some embodiments, each of at least one of the one or more first parameters is, or are, different than each of at least one of the one or more second parameters. In some embodiments, the duration of the first particular time period may be the same as the duration of the second particular time period.

In some embodiments, the first energy waveform parameter set may define a first plurality of discrete energy application sets to deliver the first tissue-ablative energy during the duration of the first particular time period. In some embodiments, the second energy waveform parameter set may define a second plurality of discrete energy application sets to deliver the second tissue-ablative energy during the duration of the second particular time period. In some embodiments, each discrete energy application set of the first plurality of discrete energy application sets and each discrete energy application set of the second plurality of discrete energy application sets may be configured to cause pulsed field ablation of tissue. In some embodiments, the first plurality of discrete energy application sets may include the same total number of discrete energy applications as the second plurality of discrete energy application sets.

In some embodiments, each discrete energy application set of at least one discrete energy application set in the first plurality of discrete energy application sets may include a different number of discrete energy applications compared to each discrete energy application set of at least one discrete energy application set in the second plurality of discrete energy application sets. In some embodiments, wherein each discrete energy application set in the first plurality of discrete energy application sets may include one or more discrete energy applications, and each discrete energy application set in the first plurality of discrete energy application sets may include the same total number of discrete energy applications as each of every other discrete energy application set in the first plurality of discrete energy application sets. In some embodiments, each discrete energy application set in the second plurality of discrete energy application sets may include one or more discrete energy applications. In some embodiments, each discrete energy application set in the second plurality of discrete energy application sets may include the same total number of discrete energy applications as each of every other discrete energy application set in the second plurality of discrete energy application sets.

In some embodiments, each discrete energy application set of at least one discrete energy application set in the first plurality of discrete energy application sets may include one or more discrete energy applications, each delivering a first particular amount of energy, and each discrete energy application set of at least one discrete energy application set in the second plurality of discrete energy application sets may include one or more discrete energy applications, each delivering a second particular amount of energy. According to various embodiments, the second particular amount of energy may be different than the first particular amount of energy.

In some embodiments, (a) movement of the part of the transducer-based device in the bodily cavity occurs at least between the delivery of at least two discrete energy application sets in the first plurality of discrete energy application sets, (b) movement of the part of the transducer-based device in the bodily cavity occurs at least between the delivery of at least two discrete energy application sets in the second plurality of discrete energy application sets, or each of (a) and (b). In some embodiments, in the event of (a), the first energy waveform parameter set may define that each discrete energy application set of the at least two discrete energy application sets in the first plurality of discrete energy application sets includes a respective one or more particular discrete energy applications, the respective one or more particular discrete energy applications of the at least the two discrete energy application sets in the first plurality of discrete energy application sets applied in an overlapping manner during the delivery of the first tissue-ablative energy. In some embodiments, in the event of (b), the second energy waveform parameter set may define that each discrete energy application set of the at least two discrete energy application sets in the second plurality of discrete energy application sets includes a respective one or more particular discrete energy applications, the respective one or more particular discrete energy applications of the at least two discrete energy application sets in the second plurality of discrete energy application sets applied in an overlapping manner during the delivery of the second tissue-ablative energy. In some embodiments, in the event of (a), the first energy waveform parameter set may define that the at least two discrete energy application sets in the first plurality of discrete energy application sets include at least three discrete energy application sets in the first plurality of discrete energy application sets. In some embodiments, in the event of (b), the second energy waveform parameter set may define that the at least two discrete energy application sets in the second plurality of discrete energy application sets include at least three discrete energy application sets in the second plurality of discrete energy application sets. In some embodiments, in the event of (a), each discrete energy application set of the at least two discrete energy application sets in the first plurality of discrete energy application sets may be configured at least by the first energy waveform parameter set to deliver a respective amount of energy insufficient to produce a transmural tissue lesion in the bodily cavity. In some embodiments, in the event of (b), each discrete energy application set of the at least two discrete energy application sets in the second plurality of discrete energy application sets may be configured at least by the second energy waveform parameter set to deliver a respective amount of energy insufficient to produce a transmural tissue lesion in the bodily cavity. In some embodiments, in the event of (a), at least the at least two discrete energy application sets in the first plurality of discrete energy application sets may be configured at least by the first energy waveform parameter set to collectively deliver energy sufficient to produce a transmural tissue lesion in the bodily cavity. In some embodiments, in the event of (b), at least the at least two discrete energy application sets in the second plurality of discrete energy application sets may be configured at least by the second energy waveform parameter set to collectively deliver energy sufficient to produce a transmural tissue lesion in the bodily cavity.

In some embodiments, each discrete energy application set in the first plurality of discrete energy application sets may be configured at least by the first energy waveform parameter set to deliver a respective amount of energy insufficient to produce a transmural tissue lesion in the bodily cavity, and the discrete energy application sets of the first plurality of discrete energy application sets may be configured at least by the first energy waveform parameter set to collectively deliver energy sufficient to produce a transmural tissue lesion in the bodily cavity. In some embodiments, each discrete energy application set in the second plurality of discrete energy application sets may be configured at least by the second energy waveform parameter set to deliver a respective amount of energy insufficient to produce a transmural tissue lesion in the bodily cavity, and the discrete energy application sets of the second plurality of discrete energy application sets may be configured at least by the second energy waveform parameter set to collectively deliver energy sufficient to produce a transmural tissue lesion in the bodily cavity.

According to some embodiments, each of the first tissue-ablative energy and the second tissue-ablative energy may be energy delivered via pulsed field ablation. In some embodiments, the location information may indicate the locations of the part of a transducer-based device relative to a tissue surface in the bodily cavity. In some embodiments, the location information may indicate the locations of the part of a transducer-based device relative to a reference device of a navigation system.

Combinations and sub-combinations of the systems described above may form other systems according to various embodiments.

According to various embodiments, a tissue ablation system may be summarized as including an input-output device system, a memory device system storing a program, and a data processing device system communicatively connected to the input-output device system and the memory device system. In some embodiments, the data processing device system may be configured at least by the program at least to receive, via the input-output device system, location information indicating a plurality of locations in a bodily cavity in response to movement of at least part of a transducer-based device in the bodily cavity. In some embodiments, the data processing device system may be configured at least by the program at least to determine, based at least on an analysis of at least part of the location information, target location information indicative of a target location set relative to a first particular location of the plurality of locations in the bodily cavity. In some embodiments, the data processing device system may be configured at least by the program at least to determine, based at least on an analysis of at least part of the location information, that at least a portion of the transducer-based device has reached a target location relative to the first particular location of the plurality of locations in the bodily cavity, the target location defined at least in part by the target location information and belonging to the target location set. In some embodiments, the data processing device system may be configured at least by the program at least to cause, in response to the determination that at least the portion of the transducer-based device has reached the target location relative to the first particular location of the plurality of locations in the bodily cavity, the transducer-based device to deliver particular tissue-ablative energy via a communicative connection between the input-output device system and the transducer-based device.

In some embodiments, the particular tissue-ablative energy may be energy delivered via pulsed field ablation.

In some embodiments, the data processing device system may be configured at least by the program at least to determine, as at least part of the determination that at least the portion of the transducer-based device has reached the target location relative to the first particular location of the plurality of locations in the bodily cavity, that at least the portion of the transducer-based device has reached a target distance from the first particular location of the plurality of locations in the bodily cavity. In some embodiments, the target location information may define a target distance from the first particular location of the plurality of locations in the bodily cavity. In some embodiments, the target location may be a second particular location of the plurality of particular locations in the bodily cavity spaced by at least the target distance from the first particular location of the plurality of locations in the bodily cavity. In some embodiments, the data processing device system may be configured at least by the program at least to determine the target location as a second particular location of the plurality of locations in the bodily cavity in response to the determination that at least the portion of the transducer-based device has reached the target distance from the first particular location of the plurality of locations in the bodily cavity.

According to some embodiments, the portion of the transducer-based device may be the part of the transducer-based device. In some embodiments, the target location information may define the target location set as a plurality of possible target locations, each of the possible target locations spaced from the first particular location of the plurality of locations in the bodily cavity by a target radius.

In some embodiments, the data processing device system may be configured at least by the program at least to determine, as at least part of the determination that at least the portion of the transducer-based device has reached the target location relative to the first particular location of the plurality of locations in the bodily cavity, a presence of contact between the transducer-based device and a tissue surface in the bodily cavity. In some embodiments, the data processing device system may be configured at least by the program at least to cause the transducer-based device to deliver the particular tissue-ablative energy also in response to determining the presence of the contact between the transducer-based device and of the tissue surface in the bodily cavity.

In some embodiments, the first particular location of the plurality of locations may be a location of a previously ablated tissue region. In some embodiments, the first particular location may be one of the plurality of locations in the bodily cavity corresponding to a previous delivery of tissue ablation energy by the transducer-based device prior to delivery of the particular tissue-ablative energy. In some embodiments, the first particular location is one of the plurality of locations in the bodily cavity corresponding to a previous delivery of tissue ablation energy by the portion of the transducer-based device prior to delivery of the particular tissue-ablative energy.

In some embodiments, the data processing device system may be configured at least by the program at least to determine that at least the portion of the transducer-based device has reached a target distance from a location of at least the part of the transducer-based device during a previous delivery of tissue ablation energy. In some embodiments, the portion of the transducer-based device may be the part of the transducer-based device.

In some embodiments, the target location may be a second particular location of the plurality of locations in the bodily cavity, the second particular location other than the first particular location. In some embodiments, the data processing device system may be configured at least by the program at least to cause the transducer-based device to deliver the particular tissue-ablative energy at the target location in response to determining that at least the portion of the transducer-based device has reached the target location.

In some embodiments, the data processing device system may be configured at least by the program at least to cause the transducer-based device to deliver the particular tissue-ablative energy via a discrete energy application set. In some embodiments, the discrete energy application set may be configured to cause pulsed field ablation of tissue. In some embodiments the portion of the transducer-based device may be a first portion of the transducer-based device, the target location may be a first target location, and the discrete energy application set may be a first discrete energy application set. In some embodiments, the data processing device system may be configured at least by the program at least to determine, after at least the first portion of the transducer-based device has reached the first target location, and based at least on an analysis of at least part of the location information, that at least a second portion of the transducer-based device has reached a second target location relative to the first target location. In some embodiments, the data processing device system may be configured at least by the program at least to cause, in response to determining that at least the second portion of the transducer-based device has reached the second target location relative to the first target location, the transducer-based device to deliver a second discrete energy application set via the communicative connection between the input-output device system and the transducer-based device. In some embodiments, the second portion of the transducer-based device may be the first portion of the transducer-based device. In some embodiments, the part of the transducer-based device may be the second portion of the transducer-based device, which may also be the first portion of the transducer-based device. In some embodiments, the data processing device system may be configured at least by the program at least to cause the transducer-based device to deliver the first discrete energy application set at the first target location in response to determining that at least the first portion of the transducer-based device has reached the first target location. In some embodiments, the data processing device system may be configured at least by the program at least to cause the transducer-based device to deliver the second discrete energy application set at the second target location in response to determining that at least the second portion of the transducer-based device has reached the second target location. In some embodiments, the data processing device system may be configured at least by the program at least to cause the transducer-based device to deliver the first discrete energy application set during a first time interval, and to cause the transducer-based device to deliver the second discrete energy application set during a second time interval. In some embodiments, a duration of the second time interval may be the same as a duration of the first time interval.

In some embodiments, the first discrete energy application set may include a different total number of discrete energy applications than the second discrete energy application set. In some embodiments, the first discrete energy application set may include one or more discrete energy applications, each delivering a first particular amount of energy. In some embodiments, the second discrete energy application set may include one or more discrete energy applications, each delivering a second particular amount of energy. In some embodiments, the second particular amount of energy may be the same as the first particular amount of energy. In some embodiments, the second particular amount of energy may be different than the first particular amount of energy.

In some embodiments, each of the first discrete energy application set and the second discrete energy application set may include one or more respective particular discrete energy applications, and the one or more respective particular discrete energy applications of the first discrete energy application set and the one or more respective particular discrete energy applications of the second discrete energy application set may be applied to the same particular tissue region. In some embodiments, each of the first discrete energy application set and the second discrete energy application set may form a respective part of a group of discrete energy application sets. in some embodiments, each discrete energy application set in the group of discrete energy application sets may be configured to deliver a respective amount of energy insufficient to produce a transmural tissue lesion in the bodily cavity. In some embodiments, the discrete energy application sets in the group of the discrete energy application sets may be configured to collectively deliver energy sufficient to produce a transmural tissue lesion in the bodily cavity. In some embodiments, the data processing device system may be configured at least by the program at least to cause the transducer-based device to deliver each of the first discrete energy application set and the second discrete energy application set to form at least part of a circumferential ablated tissue region in the bodily cavity. In some embodiments, the data processing device system may be configured at least by the program at least to cause the transducer-based device to deliver a third discrete energy application set to form at least part of the circumferential ablated tissue region. In some embodiments, the data processing device system may be configured at least by the program at least to cause the transducer-based device to deliver the first discrete energy application set to start formation of the circumferential ablated tissue region in the bodily cavity, and to cause the transducer-based device to deliver the third discrete energy application set to conclude formation of the circumferential ablated tissue region in the bodily cavity. In some embodiments, the first discrete energy application set may include a different total number of discrete energy applications than the third discrete energy application set. In some embodiments, the first discrete energy application set may include one or more discrete energy applications, each delivering a first particular amount of energy, and the third discrete energy application set may include one or more discrete energy applications, each delivering a second particular amount of energy. In some embodiments, the second particular amount of energy may be different than the first particular amount of energy.

Combinations and sub-combinations of the systems described above may form other systems according to various embodiments.

In some embodiments, a pulsed field ablation system may be summarized as including an input-output device system, a memory device system storing a program, and a data processing device system communicatively connected to the input-output device system and the memory device system. In some embodiments, the data processing device system may be configured at least by the program at least to receive, via the input-output device system, location information indicating movement of at least part of a transducer-based device through a plurality of locations in a bodily cavity during a particular time period. In some embodiments, the data processing device system may be configured at least by the program at least to cause the transducer-based device, via a communicative connection between the input-output device system and the transducer-based device, to deliver pulsed field ablation energy at least during part of the particular time period. In some embodiments, the data processing device system may be configured at least by the program at least to determine, based at least on an analysis of at least part of the location information, a rate of movement of at least the part of the transducer-based device. In some embodiments, the data processing device system may be configured at least by the program at least to provide, via the input-output device system, a first user-feedback indication in response to a first state in which the determined rate of movement of at least the part of the transducer-based device exceeds a first rate of movement threshold.

In some embodiments, the data processing device system may be configured at least by the program at least to provide, via the input-output device system, a second user-feedback indication in response to a second state in which the determined rate of movement of at least the part of the transducer-based device is below a second rate of movement threshold. In some embodiments, the data processing device system may be configured at least by the program at least to cause, during the movement, the transducer-based device to deliver the pulsed field ablation energy via the communicative connection between the input-output device system and the transducer-based device. In some embodiments, the data processing device system may be configured at least by the program at least to control or modify, via the communicative connection between the input-output device system and the transducer-based device, the delivery of the pulsed field ablation energy in response to a state in which the determined rate of movement indicates a change in rate of movement beyond a threshold.

In some embodiments, the data processing device system may be configured at least by the program at least to provide, via the input-output device system and at least in response to the first state in which the determined rate of movement of at least the part of the transducer-based device exceeds the first rate of movement threshold, a user re-ablate indication indicating that a tissue region should be re-ablated.

Combinations and sub-combinations of the systems described above may form other systems according to various embodiments.

In some embodiments, a pulsed field ablation system may be summarized as including an input-output device system, a memory device system storing a program, and a data processing device system communicatively connected to the input-output device system and the memory device system. In some embodiments, the data processing device system may be configured at least by the program at least to receive, via the input-output device system, location information indicating movement of at least part of a transducer-based device through a plurality of locations in a bodily cavity during a particular time period. In some embodiments, the data processing device system may be configured at least by the program at least to cause the transducer-based device, via a communicative connection between the input-output device system and the transducer-based device, to deliver pulsed field ablation energy at least during part of the particular time period. In some embodiments, the data processing device system may be configured at least by the program at least to determine, based at least on an analysis of the location information, a rate of movement of at least the part of the transducer-based device. In some embodiments, the data processing device system may be configured at least by the program at least to provide, via the input-output device system, a user-feedback indication indicating the determined rate of movement.

In some embodiments, a tissue ablation system may be summarized as including an input-output device system, a memory device system storing a program, and a data processing device system communicatively connected to the input-output device system and the memory device system. In some embodiments, the data processing device system may be configured at least by the program at least to receive, via the input-output device system, location information indicating locations of at least part of a transducer-based device relative to a tissue surface in a bodily cavity. In some embodiments, the data processing device system may be configured at least by the program at least to determine a rate of movement of at least the part of the transducer-based device relative to the tissue surface in the bodily cavity based at least on an analysis of the locations indicated by the received location information. In some embodiments, the data processing device system may be configured at least by the program at least to vary an energy waveform parameter set based at least on the determined rate of movement of at least the part of the transducer-based device in the bodily cavity. In some embodiments, the data processing device system may be configured at least by the program at least to cause, via the input-output device system and the transducer-based device, delivery of tissue-ablative energy in accordance with the varied energy waveform parameter set, wherein the tissue-ablative energy is configured to cause tissue ablation.

Various embodiments of the present invention may include systems, devices, or machines that are or include combinations or subsets of any one or more of the systems, devices, or machines and associated features thereof summarized above or otherwise described herein (which should be deemed to include the figures).

Further, all or part of any one or more of the systems, devices, or machines summarized above or otherwise described herein or combinations or sub-combinations thereof may implement or execute all or part of any one or more of the processes or methods described herein or combinations or sub-combinations thereof.

For example, in some embodiments, a method is executed by a data processing device system according to a program stored by a communicatively connected memory device system, the data processing device system also communicatively connected to an input-output device system, and the method including receiving, via the input-output device system, location information indicating locations of at least part of a transducer-based device; causing, via a communicative connection between the input-output device system and the transducer-based device, delivery of first tissue-ablative energy during a duration of a first particular time period in accordance with a first energy waveform parameter set at least in response to a first state in which at least part of the location information indicates at least a first rate of movement of the part of the transducer-based device, wherein the first tissue-ablative energy caused to be delivered during the duration of the first particular time period in accordance with the first energy waveform parameter set is configured to cause tissue ablation; and causing, via a communicative connection between the input-output device system and the transducer-based device, delivery of second tissue-ablative energy during a duration of a second particular time period in accordance with a second energy waveform parameter set at least in response to a second state in which the at least part of the location information indicates at least a second rate of movement of the part of the transducer-based device, wherein the second tissue-ablative energy caused to be delivered during the duration of the second particular time period in accordance with the second energy waveform parameter set is configured to cause tissue ablation, the second energy waveform parameter set different than the first energy waveform parameter set, and the second rate of movement different than the first rate of movement.

For another example, in some embodiments, a method is executed by a data processing device system according to a program stored by a communicatively connected memory device system, the data processing device system also communicatively connected to an input-output device system, and the method includes receiving, via the input-output device system, location information indicating a plurality of locations in response to movement of at least part of a transducer-based device; determining, based at least on an analysis of at least part of the location information, target location information indicative of a target location set relative to a first particular location of the plurality of locations; determining, based at least on an analysis of at least part of the location information, that at least a portion of the transducer-based device has reached a target location relative to the first particular location of the plurality of locations, the target location defined at least in part by the target location information and belonging to the target location set; and causing, in response to the determination that at least the portion of the transducer-based device has reached the target location relative to the first particular location of the plurality of locations, the transducer-based device to deliver particular tissue-ablative energy via a communicative connection between the input-output device system and the transducer-based device.

For another example, in some embodiments, a method is executed by a data processing device system according to a program stored by a communicatively connected memory device system, the data processing device system also communicatively connected to an input-output device system, and the method including receiving, via the input-output device system, location information indicating movement of at least part of a transducer-based device through a plurality of locations during a particular time period; causing the transducer-based device, via a communicative connection between the input-output device system and the transducer-based device, to deliver pulsed field ablation energy at least during part of the particular time period; determining, based at least on an analysis of at least part of the location information, a rate of movement of at least the part of the transducer-based device; and providing, via the input-output device system, a first user-feedback indication in response to a first state in which the determined rate of movement of at least the part of the transducer-based device exceeds a first rate of movement threshold.

For another example, in some embodiments, a method is executed by a data processing device system according to a program stored by a communicatively connected memory device system, the data processing device system also communicatively connected to an input-output device system, and the method including receiving, via the input-output device system, location information indicating movement of at least part of a transducer-based device through a plurality of locations during a particular time period; causing the transducer-based device, via a communicative connection between the input-output device system and the transducer-based device, to deliver pulsed field ablation energy at least during part of the particular time period; determining, based at least on an analysis of the location information, a rate of movement of at least the part of the transducer-based device; and providing, via the input-output device system, a user-feedback indication indicating the determined rate of movement.

It should be noted that various embodiments of the present invention include variations of the methods or processes summarized above or otherwise described herein (which should be deemed to include the figures) and, accordingly, are not limited to the actions described or shown in the figures or their ordering, and not all actions shown or described are required according to various embodiments. According to various embodiments, such methods may include more or fewer actions and different orderings of actions. Any of the features of all or part of any one or more of the methods or processes summarized above or otherwise described herein may be combined with any of the other features of all or part of any one or more of the methods or processes summarized above or otherwise described herein.

In addition, a computer program product may be provided that includes program code portions for performing some or all of any one or more of the methods or processes and associated features thereof described herein, when the computer program product is executed by a computer or other computing device or device system. Such a computer program product may be stored on one or more computer-readable storage mediums, also referred to as one or more computer-readable data storage mediums or a computer-readable storage medium system.

For example, in some embodiments, one or more computer-readable storage mediums may store a program executable by a data processing device system communicatively connected to an input-output device system, the program including reception instructions configured to cause reception, via the input-output device system, of location information indicating locations of at least part of a transducer-based device in a bodily cavity; first delivery instructions configured to cause, via a communicative connection between the input-output device system and the transducer-based device, delivery of first tissue-ablative energy during a duration of a first particular time period in accordance with a first energy waveform parameter set at least in response to a first state in which at least part of the location information indicates at least a first rate of movement of the part of the transducer-based device in the bodily cavity, wherein the first tissue-ablative energy caused to be delivered during the duration of the first particular time period in accordance with the first energy waveform parameter set is configured to cause tissue ablation; and second delivery instructions configured to cause, via a communicative connection between the input-output device system and the transducer-based device, delivery of second tissue-ablative energy during a duration of a second particular time period in accordance with a second energy waveform parameter set at least in response to a second state in which the at least part of the location information indicates at least a second rate of movement of the part of the transducer-based device in the bodily cavity, wherein the second tissue-ablative energy caused to be delivered during the duration of the second particular time period in accordance with the second energy waveform parameter set is configured to cause tissue ablation, the second energy waveform parameter set different than the first energy waveform parameter set, and the second rate of movement different than the first rate of movement.

For another example, in some embodiments, one or more computer-readable storage mediums may store a program executable by a data processing device system communicatively connected to an input-output device system, the program including reception instructions configured to cause reception, via the input-output device system, of location information indicating a plurality of locations in a bodily cavity in response to movement of at least part of a transducer-based device in the bodily cavity; first determination instructions configured to cause a determination, based at least on an analysis of at least part of the location information, of target location information indicative of a target location set relative to a first particular location of the plurality of locations in the bodily cavity; second determination instructions configured to cause a determination, based at least on an analysis of at least part of the location information, that at least a portion of the transducer-based device has reached a target location relative to the first particular location of the plurality of locations in the bodily cavity, the target location defined at least in part by the target location information and belonging to the target location set; and delivery instructions configured to cause, in response to the determination that at least the portion of the transducer-based device has reached the target location relative to the first particular location of the plurality of locations in the bodily cavity, the transducer-based device to deliver particular tissue-ablative energy via a communicative connection between the input-output device system and the transducer-based device.

For another example, in some embodiments, one or more computer-readable storage mediums may store a program executable by a data processing device system communicatively connected to an input-output device system, the program including reception instructions configured to cause reception, via the input-output device system, of location information indicating movement of at least part of a transducer-based device through a plurality of locations in a bodily cavity during a particular time period; delivery instructions configured to cause the transducer-based device, via a communicative connection between the input-output device system and the transducer-based device, to deliver pulsed field ablation energy at least during part of the particular time period; determination instructions configured to cause determination, based at least on an analysis of at least part of the location information, of a rate of movement of at least the part of the transducer-based device; and user-feedback instructions configured to cause provision, via the input-output device system, of a first user-feedback indication in response to a first state in which the determined rate of movement of at least the part of the transducer-based device exceeds a first rate of movement threshold.

For another example, in some embodiments, one or more computer-readable storage mediums may store a program executable by a data processing device system communicatively connected to an input-output device system, the program including reception instructions configured to cause reception, via the input-output device system, of location information indicating movement of at least part of a transducer-based device through a plurality of locations in a bodily cavity during a particular time period; delivery instructions configured to cause the transducer-based device, via a communicative connection between the input-output device system and the transducer-based device, to deliver pulsed field ablation energy at least during part of the particular time period; determination instructions configured to cause determination, based at least on an analysis of the location information, of a rate of movement of at least the part of the transducer-based device; and user-feedback instructions configured to cause provision, via the input-output device system, of a user-feedback indication indicating the determined rate of movement.

In some embodiments, each of any of one or more or all of the computer-readable data storage mediums or medium systems (also referred to as processor-accessible memory device systems) described herein is a non-transitory computer-readable (or processor-accessible) data storage medium or medium system (or memory device system) including or consisting of one or more non-transitory computer-readable (or processor-accessible) storage mediums (or memory devices) storing the respective program(s) which may configure a data processing device system to execute some or all of any of one or more of the methods or processes described herein.

Further, any of all or part of one or more of the methods or processes and associated features thereof discussed herein may be implemented or executed on or by all or part of a device system, apparatus, or machine, such as all or a part of any of one or more of the systems, apparatuses, or machines described herein or a combination or sub-combination thereof.

### BRIEF DESCRIPTION OF THE DRAWINGS

It is to be understood that the attached drawings are for purposes of illustrating aspects of various embodiments and may include elements that are not to scale.
Figure 1 includes a schematic representation of a tissue ablation system according to various example embodiments, the tissue ablation system including a data processing device system, an input-output device system, and a memory device system.
Figure 2 includes a partially schematic representation of some particular implementations of a catheter navigation system implementing an electric-field-based location system, according to various example embodiments.
Figure 3 includes a partially schematic representation of some particular implementations of a catheter navigation system implementing a magnetic-field-based location system, according to various example embodiments.
Figure 4 includes a cutaway diagram of a heart showing a catheter navigation system including a reference device and a transducer-based device of a tissue ablation system, the reference device part of a catheter-device-location tracking system and percutaneously placed at least proximate a heart cavity, and the transducer-based device percutaneously placed in a left atrium of the heart, according to various example embodiments.
Figure 5 includes a partially schematic representation of at least a portion of a tissue ablation system according to various example embodiments, the tissue ablation system including a data processing device system, an input-output device system, a memory device system, and a transducer-based device, the transducer-based device including a plurality of transducers and an expandable structure shown in a delivery or unexpanded configuration, according to various example embodiments.
Figure 6 includes the representation of the portion of the tissue ablation system of Figure 5 with the expandable structure shown in a deployed or expanded configuration, according to various example embodiments.
Figure 7 includes a schematic representation of a transducer-based device of a tissue ablation system that includes a flexible circuit structure, according to various example embodiments.
Figures 8A, 8B, and 8C illustrate program configurations or methods of ablating tissue, according to various example embodiments.
Figures 9A, 9B, 9C, and 9D illustrate various tissue ablation dosing configurations, according to various example embodiments.
Figures 10A and 10B illustrate various tissue ablation dosing configurations, according to various example embodiments.
Figures 11A and 11B illustrate various tissue ablation dosing configurations with respect to one or more target locations, according to various example embodiments.
Figure 11C illustrates a circumferential tissue ablation dosing configuration, according to various example embodiments.

### DETAILED DESCRIPTION

At least some embodiments of the present invention improve upon safety, efficiency, and effectiveness of various tissue ablation systems. In some embodiments, the tissue ablation systems include thermal ablation systems (e.g., RF ablation systems). In some embodiments, the tissue ablation systems include pulsed field ablation ("PFA") systems. According to some embodiments, location information (e.g., provided by a navigation system) is employed to deliver more uniform energy delivery to tissue by reducing overlapping of excessive deliveries of doses of ablation energy. Application of excessive doses of ablation energy may raise the risk of non-specific damage to extra-cardiac structures (e.g., esophagus, phrenic nerve, etc.). In some embodiments, one or more energy-delivery waveform parameters are controlled based at least on a rate of movement of at least a portion of a transducer-based device, which, among other things, may be utilized to control such overlapping. In some embodiments, energy delivery is controlled based at least on whether or not a portion of a transducer-based device has reached a target location relative to a previous location of the portion of the transducer-based device, which, among other things, may be utilized to control such overlapping. It should be noted, however, that the invention is not limited to these, or any other embodiments, or examples provided herein, which are referred to for purposes of illustration only. In this regard, for example, while addressing potential undesired thermal effects or avoiding overlapping of excessive deliveries of doses of ablation energy may provide benefits according to some embodiments of the present invention, such embodiments may have other benefits or goals, and other embodiments may also have at least some of the same or different benefits or goals.

In this regard, in the descriptions herein, certain specific details are set forth in order to provide a thorough understanding of various embodiments of the invention. However, one skilled in the art will understand that the invention may be practiced at a more general level without one or more of these details. In other instances, well known structures have not been shown or described in detail to avoid unnecessarily obscuring descriptions of various embodiments of the invention.

Any reference throughout this specification to "one embodiment", "an embodiment", "an example embodiment", "an illustrated embodiment", "a particular embodiment", and the like means that a particular feature, structure or characteristic described in connection with the embodiment is included in at least one embodiment. Thus, any appearance of the phrase "in one embodiment", "in an embodiment", "in an example embodiment", "in this illustrated embodiment", "in this particular embodiment", or the like in this specification is not necessarily always referring to one embodiment or a same embodiment. Furthermore, the particular features, structures or characteristics of different embodiments may be combined in any suitable manner to form one or more other embodiments.

Unless otherwise explicitly noted or required by context, the word "or" is used in this disclosure in a non-exclusive sense. In addition, unless otherwise explicitly noted or required by context, the word "set" is intended to mean one or more. For example, the phrase, "a set of objects" means one or more of the objects. In some embodiments, the word "subset" is intended to mean a set having the same or fewer elements of those present in the subset's parent or superset. In other embodiments, the word "subset" is intended to mean a set having fewer elements of those present in the subset's parent or superset. In this regard, when the word "subset" is used, some embodiments of the present invention utilize the meaning that "subset" has the same or fewer elements of those present in the subset's parent or superset, and other embodiments of the present invention utilize the meaning that "subset" has fewer elements of those present in the subset's parent or superset.

Further, the phrase "at least" is or may be used herein at times merely to emphasize the possibility that other elements may exist besides those explicitly listed. However, unless otherwise explicitly noted (such as by the use of the term "only") or required by context, non-usage herein of the phrase "at least" nonetheless includes the possibility that other elements may exist besides those explicitly listed. For example, the phrase, 'based at least on A' includes A as well as the possibility of one or more other additional elements besides A. In the same manner, the phrase, 'based on A' includes A, as well as the possibility of one or more other additional elements besides A. However, the phrase, 'based only on A' includes only A. Similarly, the phrase 'configured at least to A' includes a configuration to perform A, as well as the possibility of one or more other additional actions besides A. In the same manner, the phrase 'configured to A' includes a configuration to perform A, as well as the possibility of one or more other additional actions besides A. However, the phrase, 'configured only to A' means a configuration to perform only A.

The word "device", the word "machine", the word "system", and the phrase "device system" all are intended to include one or more physical devices or sub-devices (e.g., pieces of equipment) that interact to perform one or more functions, regardless of whether such devices or sub-devices are located within a same housing or different housings. However, it may be explicitly specified according to various embodiments that a device or machine or device system resides entirely within a same housing to exclude embodiments where the respective device, machine, system, or device system resides across different housings. The word "device" may equivalently be referred to as a "device system" in some embodiments, and the word "system" may equivalently be referred to as a "device system" in some embodiments.

Further, the phrase "in response to" may be used in this disclosure. For example, this phrase may be used in the following context, where an event A occurs in response to the occurrence of an event B. In this regard, such phrase includes, for example, that at least the occurrence of the event B causes or triggers the event A.

The phrase "thermal ablation" as used in this disclosure refers, in some embodiments, to an ablation method in which destruction of tissue occurs by hyperthermia (elevated tissue temperatures) or hypothermia (depressed tissue temperatures). Thermal ablation may include radiofrequency ("RF") ablation, microwave ablation, or cryo-ablation by way of non-limiting example. Thermal ablation energy waveforms can take various forms. For example, in some thermal ablation embodiments, energy (e.g., RF energy) is provided in the form of a continuous waveform. In some thermal ablation embodiments, energy (e.g., RF energy) is provided in the form of discrete energy applications (e.g., in the form of a duty cycled waveform).

The phrase "pulsed field ablation" ("PFA") as used in this disclosure refers, in some embodiments, to an ablation method that employs high voltage pulse delivery in a unipolar or bipolar fashion in proximity to target tissue. In some embodiments, each high voltage pulse may be referred to as a discrete energy application. In some embodiments, a grouped plurality of high voltages pulses may be referred to as a discrete energy application. Each high voltage pulse can be a monophasic pulse including a single polarity, or a biphasic pulse including a first component having a first particular polarity and a second component having a second particular polarity opposite the first particular polarity. In some embodiments, the second component of the biphasic pulse follows immediately after the first component of the biphasic pulse. In some embodiments, the first and second components of the biphasic pulse are temporally separated by a relatively small time interval. The electric field applied by the high voltage pulses in PFA physiologically changes the tissue cells to which the energy is applied (e.g., puncturing or perforating the cell membrane to form various pores therein). If a lower field strength is established, the formed pores may close in time and cause the cells to maintain viability (e.g., a process sometimes referred to as reversible electroporation). If the field strength that is established is greater, then permanent, and sometimes larger, pores form in the tissue cells, the pores allowing leakage of cell contents, eventually resulting in cell death (e.g., a process sometimes referred to as irreversible electroporation).

The word "fluid" as used in this disclosure should be understood to include any fluid that can be contained within a bodily cavity or can flow into or out of, or both into and out of a bodily cavity via one or more bodily openings positioned in fluid communication with the bodily cavity. In the case of cardiac applications, fluid such as blood will flow into and out of various intracardiac cavities (e.g., a left atrium or a right atrium).

The words "bodily opening" as used in this disclosure should be understood to include a naturally occurring bodily opening or channel or lumen; a bodily opening or channel or lumen formed by an instrument or tool using techniques that can include, but are not limited to, mechanical, thermal, electrical, chemical, and exposure or illumination techniques; a bodily opening or channel or lumen formed by trauma to a body; or various combinations of one or more of the above. Various elements having respective openings, lumens, or channels and positioned within the bodily opening (e.g., a catheter sheath) may be present in various embodiments. These elements may provide a passageway through a bodily opening for various devices employed in various embodiments.

The words "bodily cavity" as used in this disclosure should be understood to mean a cavity in a body. The bodily cavity may be a cavity or chamber provided in a bodily organ (e.g., an intracardiac cavity of a heart).

The word "tissue" as used in some embodiments in this disclosure should be understood to include any surface-forming tissue that is used to form a surface of a body or a surface within a bodily cavity, a surface of an anatomical feature or a surface of a feature associated with a bodily opening positioned in fluid communication with the bodily cavity. The tissue can include part, or all, of a tissue wall or membrane that defines a surface of the bodily cavity. In this regard, the tissue can form an interior surface of the cavity that surrounds a fluid within the cavity. In the case of cardiac applications, tissue can include tissue used to form an interior surface of an intracardiac cavity such as a left atrium or a right atrium. In some embodiments, the word tissue can refer to a tissue having fluidic properties (e.g., blood) and may be referred to as fluidic tissue.

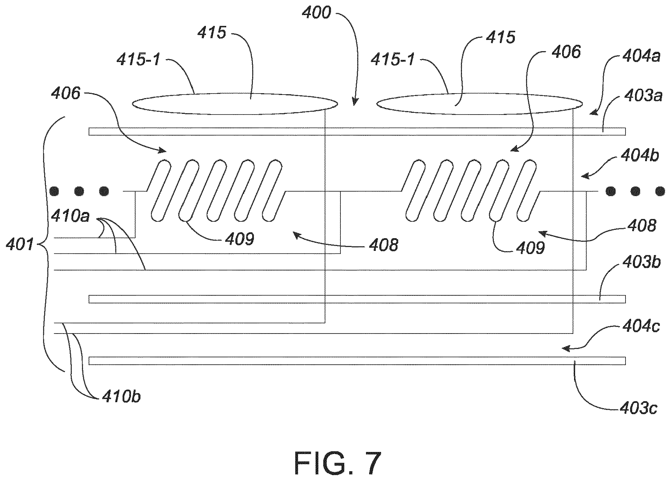

The term "transducer" as used in this disclosure should be interpreted broadly as any device capable of transmitting or delivering energy, distinguishing between fluid and tissue, sensing temperature, creating heat, ablating tissue, sensing, sampling or measuring electrical activity of a tissue surface (e.g., sensing, sampling or measuring intracardiac electrograms, or sensing, sampling or measuring intracardiac voltage data), stimulating tissue, or any combination thereof. A transducer may convert input energy of one form into output energy of another form. Without limitation, a transducer may include an electrode that functions as, or as part of, a sensing device included in the transducer, an energy delivery device included in the transducer, or both a sensing device and an energy delivery device included in the transducer. A transducer may be constructed from several parts, which may be discrete components or may be integrally formed. In this regard, although transducers, electrodes, or both transducers and electrodes are referenced with respect to various embodiments, it is understood that other transducers or transducer elements may be employed in other embodiments. It is understood that a reference to a particular transducer in various embodiments may also imply a reference to an electrode, as an electrode may be part of the transducer as shown, e.g., at least with Figure 7 discussed below.

The term "activation" as used in this disclosure should be interpreted broadly as making active a particular function as related to various transducers disclosed in this disclosure. Particular functions may include, but are not limited to, tissue ablation (e.g., PFA), sensing, sampling or measuring electrophysiological activity (e.g., sensing, sampling or measuring intracardiac electrogram information or sensing, sampling or measuring intracardiac voltage data), sensing, sampling or measuring temperature and sensing, sampling or measuring electrical characteristics (e.g., tissue impedance or tissue conductivity). For example, in some embodiments, activation of a tissue ablation function of a particular transducer is initiated by causing energy sufficient for tissue ablation from an energy source device system to be delivered to the particular transducer. Also, in this example, the activation can last for a duration of time concluding when the ablation function is no longer active, such as when energy sufficient for the tissue ablation is no longer provided to the particular transducer. In some contexts, however, the word "activation" can merely refer to the initiation of the activating of a particular function, as opposed to referring to both the initiation of the activating of the particular function and the subsequent duration in which the particular function is active. In these contexts, the phrase or a phrase similar to "activation initiation" may be used.

In the following description, some embodiments of the present invention may be implemented at least in part by a data processing device system or a controller system configured by a software program. Such a program may equivalently be implemented as multiple programs, and some, or all, of such software program(s) may be equivalently constructed in hardware. In this regard, reference to "a program" should be interpreted to include one or more programs.

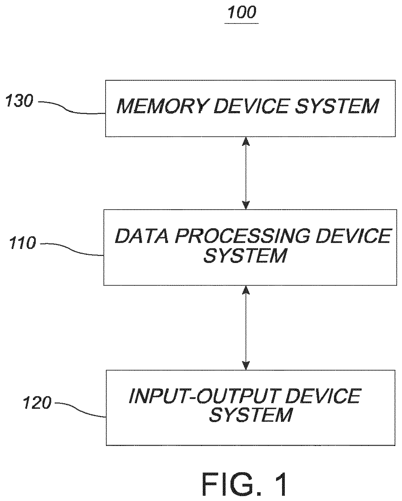

The term "program" in this disclosure should be interpreted as a set of instructions or modules that can be executed by one or more components in a system, such as a controller system or a data processing device system, in order to cause the system to perform one or more operations. The set of instructions or modules may be stored by any kind of memory device, such as those described subsequently with respect to the memory device system 130 or 330 shown in at least Figures 1, 5 and 6, respectively. In addition, this disclosure sometimes describes that the instructions or modules of a program are configured to cause the performance of a function. The phrase "configured to" in this context is intended to include at least (a) instructions or modules that are presently in a form executable by one or more data processing devices to cause performance of the function (e.g., in the case where the instructions or modules are in a compiled and unencrypted form ready for execution), and (b) instructions or modules that are presently in a form not executable by the one or more data processing devices, but could be translated into the form executable by the one or more data processing devices to cause performance of the function (e.g., in the case where the instructions or modules are encrypted in a non-executable manner, but through performance of a decryption process, would be translated into a form ready for execution). The word "module" can be defined as a set of instructions. In some instances, this disclosure describes that the instructions or modules of a program perform a function. Such descriptions should be deemed to be equivalent to describing that the instructions or modules are configured to cause the performance of the function.

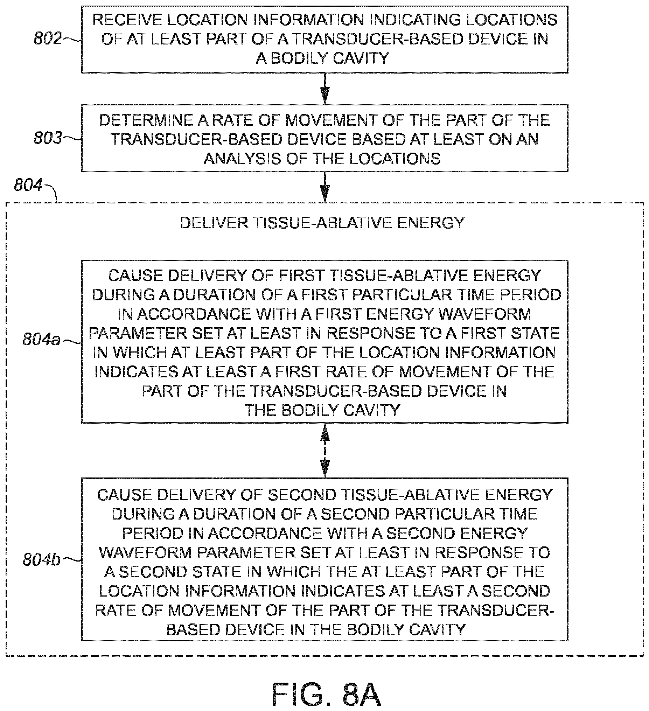

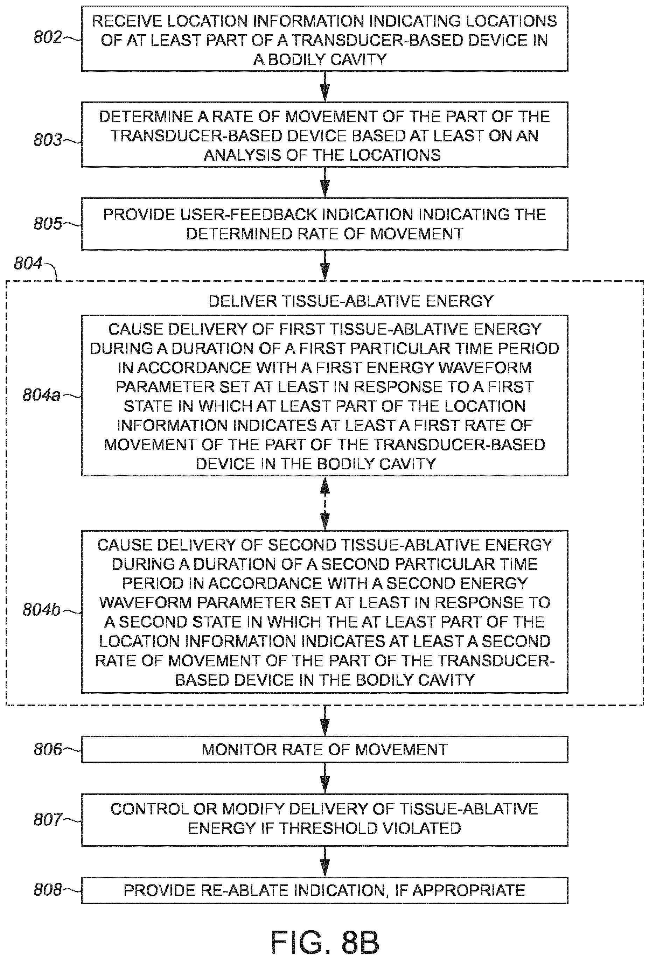

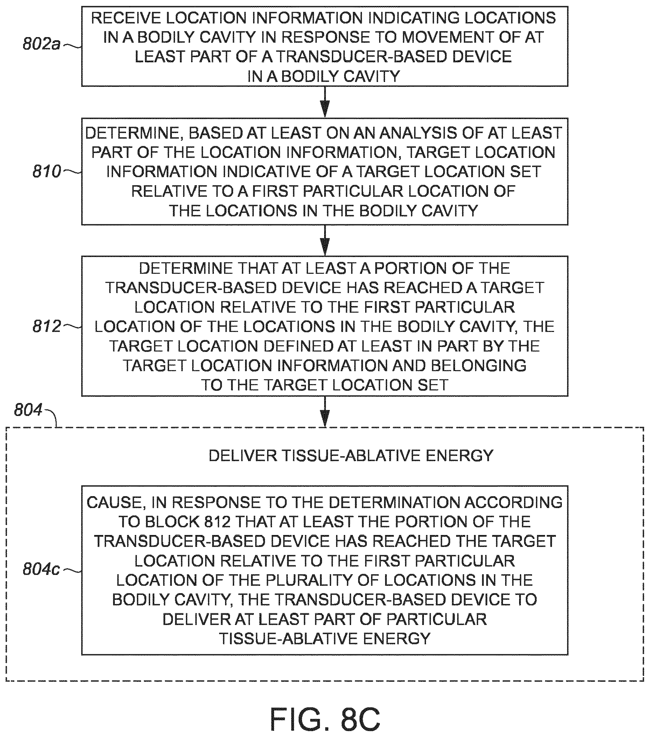

Example methods are described herein with respect to Figures 8A, 8B, and 8C. Such figures include blocks associated with actions, computer-executable instructions, or both, according to various embodiments. It should be noted that the respective instructions associated with any such blocks therein need not be separate instructions and may be combined with other instructions to form a combined instruction set. The same set of instructions may be associated with more than one block. In this regard, the block arrangement shown in each of the method figures herein is not limited to an actual structure of any program or set of instructions or required ordering of method tasks, and such method figures, according to some embodiments, merely illustrate the tasks that instructions are configured to perform, for example, upon execution by a data processing device system in conjunction with interactions with one or more other devices or device systems.

Each of the phrases "derived from" or "derivation of' or "derivation thereof' or the like may be used herein to mean to come from at least some part of a source, be created from at least some part of a source, or be developed as a result of a process in which at least some part of a source forms an input. For example, a data set derived from some particular portion of data may include at least some part of the particular portion of data, or may be created from at least part of the particular portion of data, or may be developed in response to a data manipulation process in which at least part of the particular portion of data forms an input. In some embodiments, a data set may be derived from a subset of the particular portion of data. In some embodiments, the particular portion of data is analyzed to identify a particular subset of the particular portion of data, and a data set is derived from the subset. In various ones of these embodiments, the subset may include some, but not all, of the particular portion of data. In some embodiments, changes in least one part of a particular portion of data may result in changes in a data set derived at least in part from the particular portion of data.

In this regard, each of the phrases "derived from" or "derivation of' or "derivation thereof' or the like may be used herein merely to emphasize the possibility that such data or information may be modified or subject to one or more operations. For example, if a device generates first data for display, the process of converting the generated first data into a format capable of being displayed may alter the first data. This altered form of the first data may be considered a derivative or derivation of the first data. For instance, the first data may be a one-dimensional array of numbers, but the display of the first data may be a color-coded bar chart representing the numbers in the array. For another example, if the above-mentioned first data is transmitted over a network, the process of converting the first data into a format acceptable for network transmission or understanding by a receiving device may alter the first data. As before, this altered form of the first data may be considered a derivative or derivation of the first data. For yet another example, generated first data may undergo a mathematical operation, a scaling, or a combining with other data to generate other data that may be considered derived from the first data. In this regard, it can be seen that data is commonly changing in form or being combined with other data throughout its movement through one or more data processing device systems, and any reference to information or data herein is intended in some embodiments to include these and like changes, regardless of whether or not the phrase "derived from" or "derivation of' or "derivation thereof' or the like is used in reference to the information or data. As indicated above, usage of the phrase "derived from" or "derivation of' or "derivation thereof' or the like merely emphasizes the possibility of such changes. Accordingly, in some embodiments, the addition of or deletion of the phrase "derived from" or "derivation of' or "derivation thereof' or the like should have no impact on the interpretation of the respective data or information. For example, the above-discussed color-coded bar chart may be considered a derivative of the respective first data or may be considered the respective first data itself.

In some embodiments, the term "adjacent", the term "proximate", and the like refer at least to a sufficient closeness between the objects or events defined as adjacent, proximate, or the like, to allow the objects or events to interact in a designated way. For example, in the case of physical objects, if object A performs an action on an adjacent or proximate object B, objects A and B would have at least a sufficient closeness to allow object A to perform the action on object B. In this regard, some actions may require contact between the associated objects, such that if object A performs such an action on an adjacent or proximate object B, objects A and B would be in contact, for example, in some instances or embodiments where object A needs to be in contact with object B to successfully perform the action. In some embodiments, the term "adjacent", the term "proximate", and the like additionally or alternatively refer to objects or events that do not have another substantially similar object or event between them. For example, object or event A and object or event B could be considered adjacent or proximate (e.g., physically or temporally) if they are immediately next to each other (with no other object or event between them) or are not immediately next to each other but no other object or event that is substantially similar to object or event A, object or event B, or both objects or events A and B, depending on the embodiment, is between them. In some embodiments, the term "adjacent", the term "proximate", and the like additionally or alternatively refer to at least a sufficient closeness between the objects or events defined as adjacent, proximate, and the like, the sufficient closeness being within a range that does not place any one or more of the objects or events into a different or dissimilar region or time period, or does not change an intended function of any one or more of the objects or events or of an encompassing object or event that includes a set of the objects or events. Different embodiments of the present invention adopt different ones or combinations of the above definitions. Of course, however, the term "adjacent", the term "proximate", and the like are not limited to any of the above example definitions, according to some embodiments. In addition, the term "adjacent" and the term "proximate" do not have the same definition, according to some embodiments.

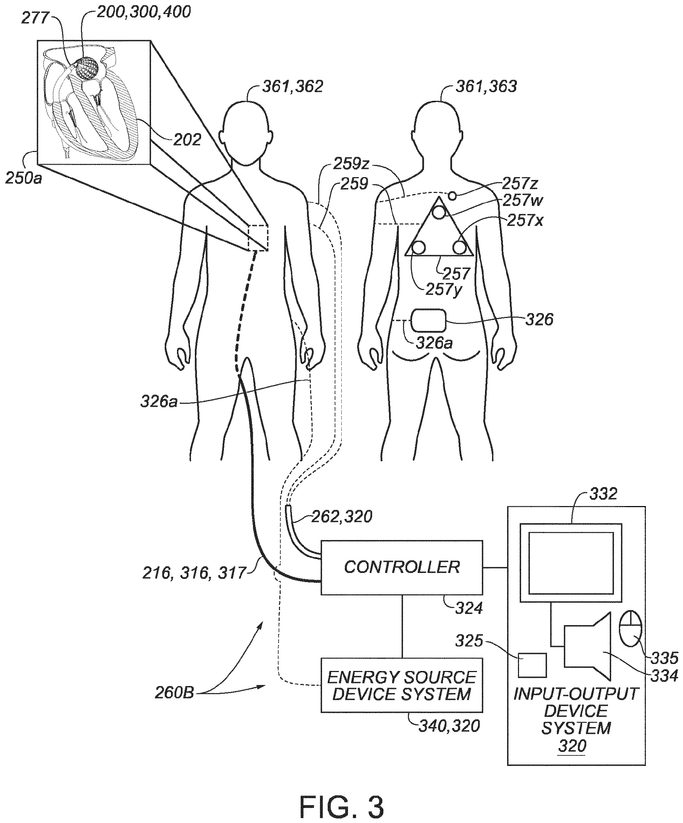

Figure 1 schematically illustrates a portion of a tissue ablation system or controller system thereof 100 that may be employed to at least select, control, activate, or monitor a function or activation of a number of electrodes or ablation transducers (e.g., ablation transducers configured to cause thermal ablation or ablation transducers configured to cause PFA), according to some embodiments. The system 100 includes a data processing device system 110, an input-output device system 120, and a processor-accessible memory device system 130. The processor-accessible memory device system 130 and the input-output device system 120 are communicatively connected to the data processing device system 110. According to some embodiments, various components such as data processing device system 110, input-output device system 120, and processor-accessible memory device system 130 form at least part of a controller system (e.g., controller system 324 shown in Figure 3).

The data processing device system 110 includes one or more data processing devices that implement or execute, in conjunction with other devices, such as those in the system 100, various methods and functions described herein, including those described with respect to methods exemplified in Figures 8A, 8B, and 8C. Each of the phrases "data processing device", "data processor", "processor", "controller", "computing device", "computer" and the like is intended to include any data or information processing device, such as a central processing unit (CPU), a control circuit, a desktop computer, a laptop computer, a mainframe computer, a tablet computer, a personal digital assistant, a cellular or smart phone, and any other device for processing data, managing data, or handling data, whether implemented with electrical, magnetic, optical, quantum, or biological components, or otherwise.

The memory device system 130 includes one or more processor-accessible memory devices configured to store one or more programs and information, including the program(s) and information needed to execute the methods or functions described herein, including those described with respect to method Figures 8A, 8B, and 8C. The memory device system 130 may be a distributed processor-accessible memory device system including multiple processor-accessible memory devices communicatively connected to the data processing device system 110 via a plurality of computers and/or devices. However, the memory device system 130 need not be a distributed processor-accessible memory system and, consequently, may include one or more processor-accessible memory devices located within a single data processing device or housing.

Each of the phrases "processor-accessible memory" and "processor-accessible memory device" and the like is intended to include any processor-accessible data storage device or medium, whether volatile or nonvolatile, electronic, magnetic, optical, or otherwise, including but not limited to, registers, hard disk drives, Compact Discs, DVDs, flash memories, ROMs, and RAMs. In some embodiments, each of the phrases "processor-accessible memory" and "processor-accessible memory device" is intended to include or be a processor-accessible (or computer-readable) data storage medium. In some embodiments, each of the phrases "processor-accessible memory" and "processor-accessible memory device" is intended to include or be a non-transitory processor-accessible (or computer-readable) data storage medium. In some embodiments, the processor-accessible memory device system 130 may be considered to include or be a non-transitory processor-accessible (or computer-readable) data storage medium system. In some embodiments, the memory device system 130 may be considered to include or be a non-transitory processor-accessible (or computer-readable) storage medium system or data storage medium system including or consisting of one or more non-transitory processor-accessible (or computer-readable) storage or data storage mediums.

The phrase "communicatively connected" is intended to include any type of connection, whether wired or wireless, between devices, data processors, or programs between which data may be communicated. Further, the phrase "communicatively connected" is intended to include a connection between devices or programs within a single data processor or computer, a connection between devices or programs located in different data processors or computers, and a connection between devices not located in data processors or computers at all. In this regard, although the memory device system 130 is shown separately from the data processing device system 110 and the input-output device system 120, one skilled in the art will appreciate that the memory device system 130 may be located completely or partially within the data processing device system 110 or the input-output device system 120. Further in this regard, although the input-output device system 120 is shown separately from the data processing device system 110 and the memory device system 130, one skilled in the art will appreciate that such system may be located completely or partially within the data processing system 110 or the memory device system 130, for example, depending upon the contents of the input-output device system 120. Further still, the data processing device system 110, the input-output device system 120, and the memory device system 130 may be located entirely within the same device or housing or may be separately located, but communicatively connected, among different devices or housings. In the case where the data processing device system 110, the input-output device system 120, and the memory device system 130 are located within the same device, the system 100 of Figure 1 can be implemented by a single application-specific integrated circuit (ASIC) in some embodiments.

The input-output device system 120 may include a mouse, a keyboard, a touch screen, another computer, or any device or combination of devices from which a desired selection, desired information, instructions, or any other data is input to the data processing device system 110. The input-output device system 120 may include a user-activatable control system that is responsive to a user action. The user-activatable control system may include at least one control element that may be activated or deactivated on the basis of a particular user action. The input-output device system 120 may include any suitable interface for receiving information, instructions or any data from other devices and systems described in various ones of the embodiments. In this regard, the input-output device system 120 may include various ones of other systems described in various embodiments. For example, the input-output device system 120 may include at least a portion of a transducer-based device system. The phrase "transducer-based device system" is intended to include one or more physical systems that include various transducers. The phrase "transducer-based device" is intended to include one or more physical devices that include various transducers. A PFA device system that includes one or more transducers may be considered a transducer-based device or device system, according to some embodiments.

The input-output device system 120 also may include an image generating device system, a display device system, a speaker or audio output device system, a computer, a processor-accessible memory device system, a network-interface card or network-interface circuitry, or any device or combination of devices to which information, instructions, or any other data is output by the data processing device system 110. In this regard, the input-output device system 120 may include various other devices or systems described in various embodiments. The input-output device system 120 may include any suitable interface for outputting information, instructions, or data to other devices and systems described in various ones of the embodiments. If the input-output device system 120 includes a processor-accessible memory device, such memory device may, or may not, form part, or all, of the memory device system 130. The input-output device system 120 may include any suitable interface for outputting information, instructions, or data to other devices and systems described in various ones of the embodiments. In this regard, the input-output device system 120 may include various other devices or systems described in various embodiments.

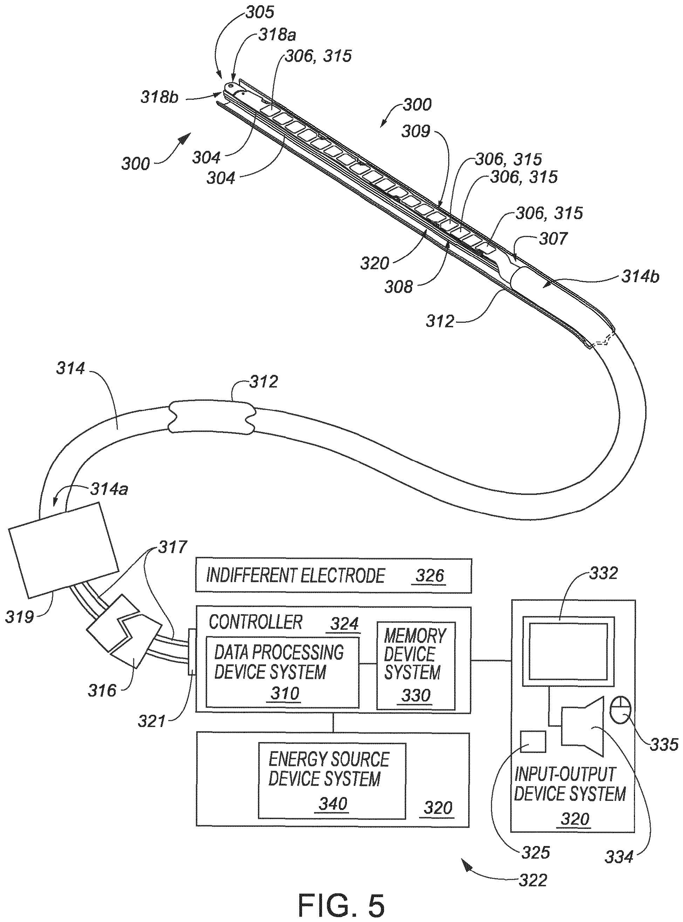

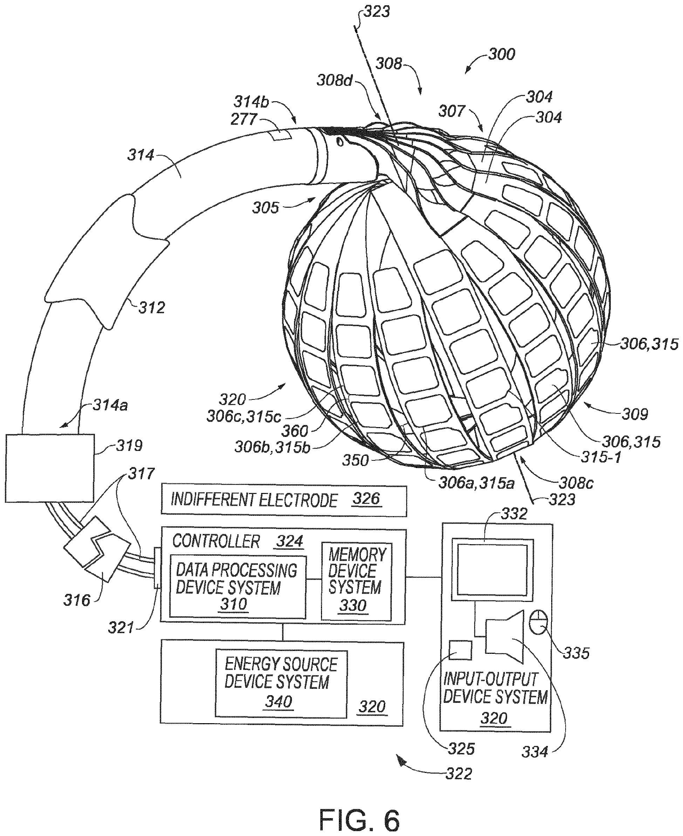

Various embodiments of transducer-based devices are described herein in this disclosure. Some of the described devices are PFA devices that are percutaneously or intravascularly deployed. Some of the described devices are movable between a delivery or unexpanded configuration (e.g., Figure 5 discussed below) in which a portion of the device is sized for passage through a bodily opening leading to a bodily cavity, and an expanded or deployed configuration (e.g., Figure 6 discussed below) in which the portion of the device has a size too large for passage through the bodily opening leading to the bodily cavity. An example of an expanded or deployed configuration, in some embodiments, is when the portion of the transducer-based device is in its intended-deployed-operational state, which may be inside the bodily cavity when, e.g., performing a therapeutic or diagnostic procedure for a patient, or which may be outside the bodily cavity when, e.g., performing testing, quality control, or other evaluation of the device. Another example of the expanded or deployed configuration, in some embodiments, is when the portion of the transducer-based device is being changed from the delivery configuration to the intended-deployed-operational state to a point where the portion of the device now has a size too large for passage through the bodily opening leading to the bodily cavity.

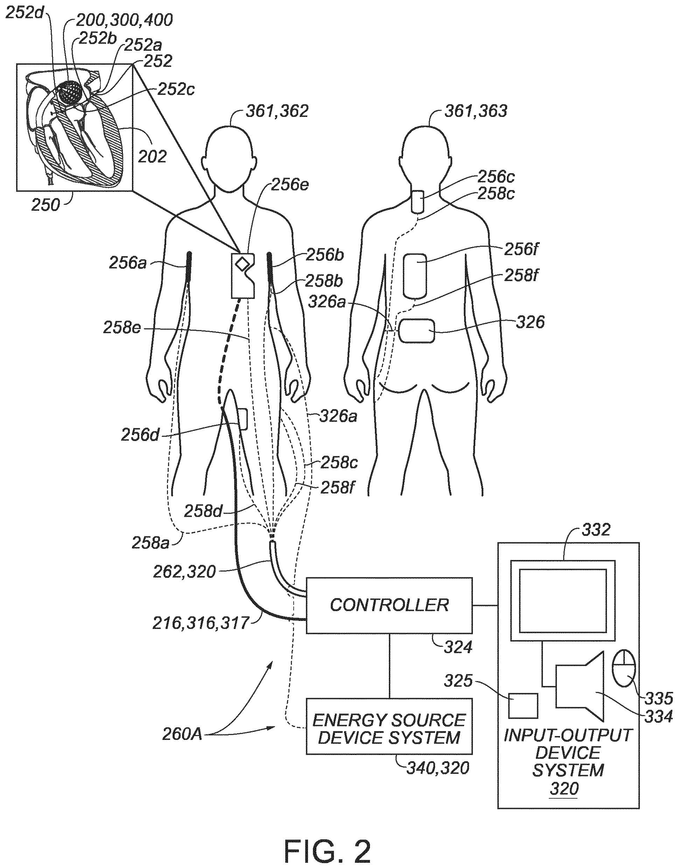

In some example embodiments, the device includes transducers that sense characteristics (e.g., convective cooling, permittivity, force) that distinguish between fluid, such as a fluidic tissue (e.g., blood), and tissue forming an interior surface of the bodily cavity. Such sensed characteristics can allow a medical system to map the cavity, for example, using positions of openings or ports into and out of the cavity to determine a position or orientation (e.g., pose), or both of the portion of the device in the bodily cavity. In some example embodiments, the described systems employ a navigation system or electro-anatomical mapping system (e.g., as described below with respect to at least Figures 2 or 3, according to some embodiments) including electromagnetic-based systems and electropotential-based systems to determine a positioning of a portion of a device in a bodily cavity. In some example embodiments, the described devices are part of a tissue ablation system capable of ablating tissue in a desired pattern within the bodily cavity using various techniques (e.g., via thermal ablation, PFA, etc., according to various embodiments).

In some example embodiments, the devices are capable of sensing various cardiac functions (e.g., electrophysiological activity including intracardiac voltages). In some example embodiments, the devices are capable of providing stimulation (e.g., electrical stimulation) to tissue within the bodily cavity. Electrical stimulation may include pacing.

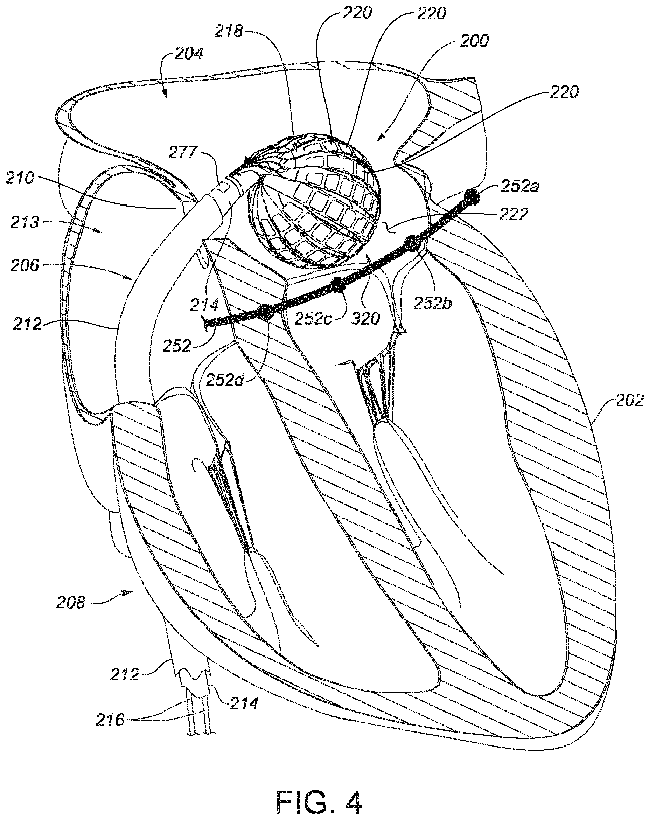

Figure 2 includes a partially schematic representation of some particular implementations of a catheter navigation system 260A implementing an electric-field-based location system, according to various example embodiments. According to some embodiments, the navigation system 260A is part of various tissue ablation systems described in this disclosure. Figure 2 illustrates a controller 324, which may be a particular implementation of the data processing device system 110 shown in Figure 1. Illustrated in Figure 2 is an input-output device system 320 communicatively connected to the controller 324 and may include a display device system 332, a mouse 335 or other pointing device system, a speaker device system 334 or other audio output device system, or a sensing device system 325, according to various embodiments. Possible contents of the input-output device system 320 are discussed in more detail below. The input-output device system 320 may be a particular implementation of the input-output device system 120 shown in Figure 1. Figure 2 also illustrates, in cut-out illustration window 250, a catheter or transducer-based device 200, 300, or 400, discussed in more detail below, which may be communicatively connected to the controller 324 via electrical conductors 216, cable 316, electrical leads 317 (discussed in more detail below; see, *e.g.*, at least Figures 4-6), or a combination thereof, according to various embodiments. According to various embodiments, catheter or transducer-based device 200, 300, or 400 may form part of a tissue ablation system. The electrical conductors 216, cable 316, or electrical leads 317 may reside, at least in part, within a catheter shaft 214 or 314 or within a catheter sheath 212 or 312 discussed in more detail below (see, *e.g.,* at least Figures 4-6). The catheter or transducer-based device 200, 300, or 400 may include one or more transducers (discussed in more detail below; see, e.g., at least Figures 4-7) and may be included in the input-output device system 320, according to some embodiments. In Figure 2, the catheter or transducer-based device 200, 300, or 400 is illustrated via cut-out illustration window 250 within a heart 202 of a patient 361, although the catheter or transducer-based device 200, 300, or 400 may instead be operated outside of any living being, e.g., in a quality-control, training, or testing environment. The single patient 361 is illustrated in two parts in Figure 2 merely to concurrently show the front-side 362 and the back-side 363 of the patient 361, although the various connections to the controller 324 are only fully shown via the illustrated front-side 362 of the patient 361 for purposes of clarity. The portion of the electrical conductors 216, cable 316, or electrical leads 317 that is outside the patient 361 is illustrated in thick solid line in Figure 2, and the portion of the electrical conductors 216, cable 316, or electrical leads 317 that is inside the patient 361 is illustrated in thick broken line in Figure 2.

Also illustrated in Figure 2 is an energy source device system 340 communicatively connected to the controller 324. The energy source device system 340 may be part of the input-output device system 320 and may be configured to provide energy to the transducers of the catheter or transducer-based device 200, 300, or 400 for sensing, tissue ablation, or both, according to various embodiments and as discussed in more detail below. According to various embodiments, energy delivered to catheter or transducer-based device 200, 300, or 400 for tissue ablation may be configured to cause thermal ablation or PFA. Electrode 326, shown on the lower back of the back-side 363 of patient 361 in Figure 2, for example, may be communicatively connected to energy source device system 340 via conductor 326a. Electrode 326 may be placed externally on the body of the patient 361, according to some embodiments. Electrode 326 may be an indifferent electrode, which may facilitate the performance of impedance sensing or ablation, particularly monopolar or blended monopolar ablation, according to some embodiments. Indifferent electrode 326 is discussed in more detail below.

Figure 2 also illustrates electrodes 256a, 256b, 256c, 256d, 256e, and 256f that are placed externally on the body of the patient 361, according to some embodiments. The electrodes 256a, 256b, 256c, 256d, 256e, and 256f may be included in the input-output device system 320 and may be communicatively connected to the controller 324 via respective electrical conductors 258a, 258b, 258c, 258d, 258e, and 258f partially inside cable 262, according to some embodiments. Although respective electrical conductors 258a, 258b, 258c, 258d, 258e, and 258f are shown within a same cable 262 for clarity of illustration, one or more of such electrical conductors may be in separate cables. According to some embodiments, electrodes 256a, 256b, 256c, 256d, 256e, and 256f are configured to generate electric fields that enable the controller 324 to determine, in conjunction with corresponding sensing performed by transducers of the catheter or transducer-based device 200, 300, or 400, X, Y, and Z coordinate axis location information of the catheter or transducer-based device 200, 300, or 400 within the heart 202 of the patient 361 or in a quality-control, training, or testing environment. In particular, electrodes 256a and 256b (a first pair of electrodes) may be configured to generate a first electric field at a first frequency or frequency range that the transducers of the catheter or transducer-based device 200, 300, or 400 are configured to sense as, e.g., respective X-axis locations of the respective transducers. Similarly, electrodes 256c and 256d (a second pair of electrodes) may be configured to generate a second electric field at a second frequency or frequency range that the transducers of the catheter or transducer-based device 200, 300, or 400 are configured to sense as, e.g., respective Y-axis locations of the respective transducers. Similarly, electrodes 256e and 256f (a third pair of electrodes) may be configured to generate a third electric field at a third frequency or frequency range that the transducers of the catheter or transducer-based device 200, 300, or 400 are configured to sense as, e.g., respective Z-axis locations of the respective transducers. The first, second, and third frequencies or frequency ranges may be mutually exclusive, according to some embodiments. In some embodiments, the first, second, and third electric fields may have a same frequency or frequency range and be time-multiplexed in coordination with time-multiplexed sensing by the transducers of the catheter or transducer-based device 200, 300, or 400, to facilitate repeated sequential sensing of respective X, Y, and Z-axis locations of the respective transducers. Electric field strength sensed by one or more transducers of the catheter or transducer-based device 200, 300, or 400 may be evaluated by the controller 324 or its data processing device system 310 to determine location information including respective three-dimensional X, Y, and Z-axis locations of the transducers with respect to the first, second, and third electric fields and with respect to reference device 252 (shown as including reference electrodes 252a, 252b, 252c, and 252d, although fewer or more may be provided), according to some embodiments. The reference device 252 (see, e.g., cut-out illustration window 250 in Figure 2 or see, e.g., Figure 4 for more detail) may be located within the body of the patient 361, preferably in a location that keeps its positioning relatively stable, such as in the coronary sinus, to factor out transitory movements of the transducer(s) of the transducer-based device 200, 300, or 400 due, e.g., to the beating of the heart. The one or more reference electrodes (e.g., reference electrodes 252a, 252b, 252c, and 252d) of the reference device 252 may be configured to also sense electric field strength of the first, second, and third electric fields, and the three-dimensional location of the transducer-based device 200, 300, or 400 is determined by the controller 324 or its data processing device system 310 with respect to the reference device 252 based on the measurements made by the transducers of the catheter or transducer-based device 200, 300, or 400 and the measurements made by the reference electrodes (e.g., reference electrodes 252a, 252b, 252c, and 252d) of the reference device 252, according to some embodiments.

The measurements made by the transducers of the catheter or transducer-based device 200, 300, or 400, the measurements made by the reference electrodes of the reference device 252 (or reference device 257z in some embodiments), or both may, in some embodiments, provide at least part of location information indicating locations of at least part of a transducer-based device 200, 300, or 400 in a bodily cavity or relative to a tissue surface in a bodily cavity. In some embodiments, even if (i) the measurements made by the transducers of the catheter or transducer-based device 200, 300, or 400, (ii) the measurements made by the reference electrodes of the reference device 252 (or reference device 257z in some embodiments), or both (i) and (ii) indicate locations of at least part of the transducer-based device 200, 300, or 400 with respect to an absolute reference frame associated with locations derived solely from the three-dimensional X, Y, and Z-axes, such location information may indicate (e.g., by derivation or by combination with tissue contact sensing information provided by electrodes of the transducer-based device in some embodiments) locations of at least part of the transducer-based device 200, 300, or 400 relative to a tissue surface in the bodily cavity, according to some embodiments. In some embodiments, measurements made by the transducers of the catheter or transducer-based device 200, 300, or 400 derived relatively to the measurements made by the reference electrodes of the reference device 252 or reference device 257z may indicate locations of at least part of a transducer-based device 200, 300, or 400 relative to a tissue surface in a bodily cavity. In this regard, a reference, such as reference device 252 or reference device 257z may, according to various embodiments, help define a coordinate frame that moves with an organ that includes the bodily cavity (e.g., movement of the organ resulting from the cardiac cycle or pulmonary cycle), and measurements made in this coordinate frame may accordingly indicate locations of at least part of a transducer-based device 200, 300, or 400 relative to a tissue surface in a bodily cavity, according to some embodiments. However, in some embodiments, the locations of at least part of a catheter or transducer-based device may be indicated by location information without necessarily being relative to a tissue surface. U.S. Patent No. 5,697,377, issued on December 16, 1997 to Frederik H. M. Wittkampf, provides examples of how to determine a three-dimensional location of a catheter (e.g., an electrode position).

In this regard, Figure 2 illustrates a catheter navigation system 260B that may include a catheter (e.g., transducer-based device 200, 300, or 400) including a plurality of transducers (discussed in more detail below), a catheter-device-location tracking system or navigation system, which may include one or more external electrodes (e.g., electrodes 256a, 256b, 256c, 256d, 256e, and 256f), one or more reference electrodes (e.g., reference electrodes 252a, 252b, 252c, 252d of reference device 252), the controller 324 or data processing system 310 or 110, the transducers of the catheter (e.g., transducer-based device 200, 300, or 400), and a display device system (e.g., display device system 332), according to various embodiments. In some embodiments, the display device system, the catheter, the navigation system, or a combination thereof may be included as part of an input-output device system (e.g., input-output device system 320 or input-output device system 120) of the catheter navigation system.

Figure 3 includes a partially schematic representation of some particular implementations of the catheter navigation system 260B implementing a magnetic-field-based location system, according to various example embodiments. In this regard, Figure 3 corresponds to Figure 2, except that a magnetic-field-based location system is illustrated instead of an electric-field-based location system. Instead of electrodes 256a, 256b, 256c, 256d, 256e, and 256f, Figure 3 illustrates three magnetic field generation sources 257w, 257x, and 257y, such as coils, each of which respectively generates a magnetic field, according to some embodiments. The magnetic field generation sources 257w, 257x, and 257y may be integrally formed within a package or frame 257 located beneath the patient 361. Magnetic field sources 257w, 257x, and 257y may respectively be connected to the controller 324 via a set of one or more conductors 259, which may or may not be located within the same cable 262. Similarly, the reference device 257z may be connected to the controller 324 via a set of one or more conductors 259z, which may or may not be included in conductor set 259, and which may or may not be located within the same cable 262. The transducer-based device 200, 300, or 400 may include one or more magnetic field transducers 277 (shown in the cut-out illustration window 250a in Figure 3) configured to sense the strengths of the magnetic fields generated by magnetic field sources 257w, 257x, and 257y. As with some embodiments associated with Figure 2, the magnetic field sources 257w, 257x, and 257y need not generate different magnetic fields, but may instead generate the same magnetic fields in a time-multiplexed manner that are sensed in sequence over time by the one or more magnetic field transducers 277. In some embodiments, the one or more magnetic field transducers 277 may sense the magnetic field strengths with respect to a reference device 257z, which may be akin to the reference device 252 in the electric field context of Figure 2. With the three magnetic field strengths detected by the one or more magnetic field transducers 277 for a given time or time period, the distance(s) between the one or more transducers 277 and the magnetic field generation sources 257w, 257x, and 257y may be determined, and the three-dimensional location of the one or more transducers 277 may be determined according to triangulation as per some embodiments. With the location of the one or more transducers 277 in three-dimensional space known, and the geometry of the transducer-based device 200, 300, or 400 (e.g., including the locations of the transducers on the transducer-based device) relative to transducers 277 also known, the locations of the transducers of the transducer-based device 200, 300, or 400 for the given time or time period may be determined, according to some embodiments. U.S. Patent Application Publication No. 2007/0265526 (Govari et al.), published on November 15, 2007, provides examples of how to determine a three-dimensional location of a catheter in a magnetic-field-based system.

Figure 4 is a representation of a transducer-based device 200 useful in investigating or treating a bodily organ, for example, a heart 202, according to some embodiments. In some embodiments, the transducer-based device 200 may form part of a tissue ablation system.

Transducer-based device 200 can be percutaneously or intravascularly inserted into a portion of the heart 202, such as an intracardiac cavity, like left atrium 204. In this example, the transducer-based device 200 is, or is part of a catheter 206 inserted via the inferior vena cava 208 and penetrating through a bodily opening in transatrial septum 210 from right atrium 213. (In this regard, transducer-based devices or device systems described herein that include a catheter may also be referred to as catheter device systems, catheter devices or device systems, or catheter-based devices or device systems, according to various embodiments.) In other embodiments, other paths may be taken.

Catheter 206 includes an elongated flexible rod or shaft member appropriately sized to be delivered percutaneously or intravascularly. Various portions of catheter 206 may be steerable. For example, a structure 218 supporting transducers 220 may be controlled via various manipulations to advance outwardly, to retract, to rotate clockwise, to rotate counterclockwise, and to have a particular deployment plane orientation (e.g., a plane in which the structure progresses from a delivery configuration (e.g., described below with respect to at least Figure 5) to or at least toward a deployed configuration (e.g., described below with respect to at least Figure 6), according to some embodiments. One or more other portions of the transducer-based device 200 may be steerable. For example, a catheter sheath 212, which encompasses or surrounds at least part of an elongate shaft member 214 to which the structure 218 is physically coupled, may be steerable. In some embodiments, the sheath 212 may be controlled via various manipulations to advance outwardly, retract, rotate clockwise, rotate counterclockwise, bend, release a bend, and have a particular bending plane orientation, according to some embodiments.

Catheter 206 may include one or more lumens. The lumen(s) may carry one or more communications or power paths, or both. For example, the lumens(s) may carry one or more electrical conductors 216 (two shown). Electrical conductors 216 provide electrical connections to transducer-based device 200 and transducers 220 thereof that are accessible externally from a patient in which the transducer-based device 200 is inserted.

Transducer-based device 200 may include a frame or structure 218 which assumes an unexpanded configuration for delivery to left atrium 204, according to some embodiments, such frame or structure supporting transducers. Structure 218 is expanded (e.g., shown in a deployed or expanded configuration in Figure 4) upon delivery to left atrium 204 to position a plurality of transducers 220 (three called out in Figure 4) proximate the interior surface formed by tissue 222 of left atrium 204. In some embodiments, at least some of the transducers 220 may be configured to sense a physical characteristic of a fluid (e.g., blood) or tissue 222, or both, that may be used to determine tissue contact. In some embodiments, at least some of the transducers 220 may be configured to selectively ablate portions of the tissue 222. For example, some of the transducers 220 may be configured to ablate a pattern around the bodily openings, ports, or pulmonary vein ostia, for instance, to reduce or eliminate the occurrence of atrial fibrillation. In some embodiments, at least some of the transducers 220 are configured to ablate cardiac tissue. In some embodiments, at least some of the transducers 220 are configured to sense or sample intracardiac voltage data or sense or sample intracardiac electrogram data. In some embodiments, at least some of the transducers 220 are configured to sense or sample intracardiac voltage data or sense or sample intracardiac electrogram data while at least some of the transducers 220 are concurrently ablating cardiac tissue. In some embodiments, at least one of the sensing or sampling transducers 220 is provided by at least one of the ablating transducers 220. In some embodiments, at least a first one of the transducers 220 senses or samples intracardiac voltage data or intracardiac electrogram data at a location at least proximate a tissue location ablated by at least a second one of the transducers 220. In some embodiments, the first one of the transducers 220 is other than the second one of the transducers 220.

Figures 5 and 6 include a catheter device system (e.g., a portion thereof shown schematically) that includes a transducer-based device 300, according to some embodiments. All or part of such catheter system may be all, or part of, a tissue ablation system, according to various embodiments. The transducer-based device 300 may be the same as the transducer-based device 200, although different sizes, numbers of transducers, or types of transducer-based devices, such as balloon catheters, may be implemented. In this regard, transducer-based device 300 includes a plurality of elongate members 304 (not all of the elongate members are called out in Figures 5 and 6) and a plurality of transducers 306 (not all of the transducers are called out in Figures 5 and 6; some of the transducers 306 are called out in Figure 6 as 306a, 306b, and 306c). Figure 5 includes a representation of a portion of the transducer-based device 300 in a delivery or unexpanded configuration. Figure 6 includes a representation of a portion of the transducer-based device 300 in an expanded or deployed configuration. It is noted that, for clarity of illustration, all of the elongate members shown in Figure 6 are not represented in Figure 5. As will become apparent, the plurality of transducers 306 is positionable within a bodily cavity, such as with the transducer-based device 200. For example, in some embodiments, the transducers 306 are able to be positioned in a bodily cavity by movement into, within, or into and within the bodily cavity, with or without a change in a configuration of the plurality of transducers 306. In some embodiments, the transducers of the plurality of transducers 306 are arranged to form a two- or three-dimensional distribution, grid or array of the transducers capable of mapping, ablating, or stimulating an inside surface of a bodily cavity or lumen without requiring mechanical scanning. As shown, for example, in Figure 5, the plurality of transducers 306 are arranged in a distribution receivable in a bodily cavity. In Figures 5 and 6, each of at least some of transducers 306 includes a respective electrode 315 (not all of the electrodes 315 are called out in Figures 5 and 6).

The elongate members 304 are arranged in a frame or structure 308 that is selectively movable between an unexpanded or delivery configuration (e.g., as shown in Figure 5) and an expanded or deployed configuration (e.g., as shown in at least Figure 6) that may be configured to position elongate members 304 against a tissue surface within the bodily cavity or position the elongate members 304 in the vicinity of the tissue surface. In some embodiments, structure 308 has a size in the unexpanded or delivery configuration suitable for delivery through a bodily opening (e.g., via catheter sheath 312) to the bodily cavity. In various embodiments, catheter sheath 312 typically includes a length sufficient to allow the catheter sheath to extend between a location at least proximate a bodily cavity into which the structure 308 is to be delivered and a location outside a body comprising the bodily cavity. In some embodiments, structure 308 has a size in the expanded or deployed configuration too large for delivery through a bodily opening (e.g., via catheter sheath 312) to the bodily cavity. The elongate members 304 may form part of a flexible circuit structure (e.g., also known as a flexible printed circuit board (PCB) circuit, examples of which are described with respect to Figure 7, below). The elongate members 304 may include a plurality of different material layers. Each of the elongate members 304 may include a plurality of different material layers. The structure 308 may include a shape memory material, for instance, Nitinol. The structure 308 may include a metallic material, for instance, stainless steel, or non-metallic material, for instance, polyimide, or both a metallic and non-metallic material by way of non-limiting example. The incorporation of a specific material into structure 308 may be motivated by various factors including the specific requirements of each of the unexpanded or delivery configuration and expanded or deployed configuration, the required position or orientation (e.g., pose), or both of structure 308 in the bodily cavity, the requirements for successful ablation of a desired pattern, or the effect that the material may have on electric or magnetic fields to be sensed by the device (e.g., by one or more transducers 306 or one or more magnetic field transducers 277).

Figure 7 is a schematic side elevation view of at least a portion of a transducer-based device 400 that includes a flexible circuit structure 401 that is configured to provide a plurality of transducers 406 (two called out), according to some embodiments. In some embodiments, the flexible circuit structure 401 may form part of a structure (e.g., structure 308) that is selectively movable between a delivery configuration sized for percutaneous delivery and expanded or deployed configurations sized too large for percutaneous delivery. In some embodiments, the flexible circuit structure 401 may be located on, or form at least part of, a structural component (e.g., elongate member 304) of a transducer-based device system.

The flexible circuit structure 401 may be formed by various techniques including flexible printed circuit techniques. In some embodiments, the flexible circuit structure 401 includes various layers including flexible layers 403a, 403b, and 403c (e.g., collectively flexible layers 403). In some embodiments, each of flexible layers 403 includes an electrical insulator material (e.g., polyimide). One or more of the flexible layers 403 may include a different material than another of the flexible layers 403. In some embodiments, the flexible circuit structure 401 includes various electrically conductive layers 404a, 404b, and 404c (collectively electrically conductive layers 404) that are interleaved with the flexible layers 403. In some embodiments, each of the electrically conductive layers 404 is patterned to form various electrically conductive elements. For example, electrically conductive layer 404a may be patterned to form a respective electrode 415 of each of the transducers 406. Electrodes 415 may have respective electrode edges 415-1 that form a periphery of an electrically conductive surface associated with the respective electrode 415. It is noted that other electrodes employed in other embodiments may have electrode edges arranged to form different electrode shapes (for example, as shown by electrode edge 315-1 in Figure 6).

Electrically conductive layer 404b is patterned, in some embodiments, to form respective temperature sensors 408 for each of the transducers 406, as well as various leads 410a arranged to provide electrical energy to the temperature sensors 408. In some embodiments, each temperature sensor 408 includes a patterned resistive member 409 (two called out) having a predetermined electrical resistance. In some embodiments, each resistive member 409 includes a metal having relatively high electrical conductivity characteristics (e.g., copper). In some embodiments, electrically conductive layer 404c is patterned to provide portions of various leads 410b arranged to provide an electrical communication path to electrodes 415. In some embodiments, leads 410b are arranged to pass though vias in flexible layers 403a and 403b to connect with electrodes 415. Although Figure 7 shows flexible layer 403c as being a bottommost layer, some embodiments may include one or more additional layers underneath flexible layer 403c, such as one or more structural layers, such as a steel or composite layer. These one or more structural layers, in some embodiments, are part of the flexible circuit structure 401 and can be part of, e.g., elongate member 304. In some embodiments, the one or more structural layers may include at least one electrically conductive surface (e.g., a metallic surface) exposed to blood flow. In addition, although Figure 7 shows only three flexible layers 403a-403c and only three electrically conductive layers 404a-404c, it should be noted that other numbers of flexible layers, other numbers of electrically conductive layers, or both, may be included.

In some embodiments, electrodes 415 are employed to selectively deliver thermal ablation energy (e.g., RF energy) to various tissue structures within a bodily cavity (not shown in Figure 7) (e.g., an intracardiac cavity or chamber). In some embodiments, the thermal energy may be delivered in the form of a continuous waveform. In some embodiments, the thermal ablation energy may be delivered in the form of plurality of discrete energy applications (e.g., in the form of a duty cycled waveform). The thermal energy delivered to the tissue may be delivered to cause monopolar thermal tissue ablation, bipolar thermal tissue ablation, or blended monopolar-bipolar thermal tissue ablation by way of non-limiting example.

In some embodiments, electrodes 415 are employed to selectively deliver discrete energy applications in the form of PFA high voltage pulses to various tissue structures within a bodily cavity (not shown in Figure 4) (e.g., an intracardiac cavity or chamber). The PFA high voltage pulses delivered to the tissue structures may be sufficient for ablating portions of the tissue structures. The PFA high voltage pulses delivered to the tissue may be delivered to cause monopolar pulsed field tissue ablation, bipolar pulsed field tissue ablation, or blended monopolar-bipolar pulsed field tissue ablation by way of non-limiting example. The energy that is delivered by each high voltage pulse may be dependent upon factors including the electrode location, size, shape, relationship with respect to another electrode (e.g., the distance between adjacent electrodes that deliver the PFA energy), the presence, or lack thereof, of various material between the electrodes, the degree of electrode-to-tissue contact, and other factors. In some cases, a maximum ablation depth resulting from the delivery of high voltage pulses by a relatively smaller electrode is typically shallower than that of a relatively larger electrode.

In some embodiments, each electrode 415 is configured to sense or sample an electric potential in the tissue proximate the electrode 415 at a same or different time than delivering energy sufficient for tissue ablation. In some embodiments, each electrode 415 is configured to sense or sample intracardiac voltage data in the tissue proximate the electrode 415. In some embodiments, each electrode 415 is configured to sense or sample data in the tissue proximate the electrode 415 from which an electrogram (e.g., an intracardiac electrogram) may be derived. In some embodiments, each resistive member 409 is positioned adjacent a respective one of the electrodes 415. In some embodiments, each of the resistive members 409 is positioned in a stacked or layered array with a respective one of the electrodes 415 to form a respective one of the transducers 406. In some embodiments, the resistive members 409 are connected in series to allow electrical current to pass through all of the resistive members 409. In some embodiments, leads 410a are arranged to allow for a sampling of electrical voltage in between each resistive member 409. This arrangement allows for the electrical resistance of each resistive member 409 to be accurately measured. The ability to accurately measure the electrical resistance of each resistive member 409 may be motivated by various reasons including determining temperature values at locations at least proximate the resistive member 409 based at least on changes in the resistance caused by convective cooling effects (e.g., as provided by blood flow). The resistance data can thus be correlated to the degree of presence of the flow between the electrode 415 and tissue, thereby allowing the degree of contact between the electrode 415 and the tissue to be determined. Other methods of detecting transducer-to-tissue contact or degrees of transducer-to-tissue contact may be employed according to various example embodiments.

Referring to Figures 5 and 6, transducer-based device 300 can communicate with, receive power from or be controlled by a transducer-activation device system 322 according to some embodiments. In some embodiments, the transducer-activation device system 322 represents one or more particular implementations of the system 100 illustrated in Figure 1. In some embodiments, elongate members 304 include transducers 306 that are communicatively connected to a data processing device system 310 via electrical connections running within elongate shaft member 314 that are communicatively connected to one or more of electrical leads 317 (e.g., control leads, data leads, power leads or any combination thereof) within elongated cable 316 (only a portion of which is shown in Figures 5 and 6 to reveal other structures) terminating at a connector 321 or other interface. The leads 317 may correspond to the electrical conductors 216 in Figure 4 in some embodiments and, although only two leads 317 are shown for clarity, more may be present. The transducer-activation device system 322 may include a controller 324 that includes the data processing device system 310 (e.g., which may be a particular implementation of data processing device system 110 from Figure 1) and a memory device system 330 (e.g., which may be a particular implementation of the memory device system 130 from Figure 1) that stores data and instructions that are executable by the data processing device system 310 to process information received from transducer-based device 300 or to control operation of transducer-based device 300, for example, activating various selected transducers 306 to ablate tissue and control a user interface (e.g., of input-output device system 320) according to various embodiments. Controller 324 may include one or more controllers.

Transducer-activation device system 322 includes an input-output device system 320 (e.g., which may be a particular implementation of the input-output device system 120 from Figure 1) communicatively connected to the data processing device system 310 (e.g., via controller 324 in some embodiments). Input-output device system 320 may include a user-activatable control that is responsive to a user action. Input-output device system 320 may include one or more user interfaces or input/output (I/O) devices, for example one or more display device systems 332, speaker device systems 334, one or more keyboards, one or more mice (e.g., mouse 335), one or more joysticks, one or more track pads, one or more touch screens or other transducers to transfer information to, from, or both to and from a user, for example a care provider such as a physician or technician. For example, output from a mapping process may be displayed by a display device system 332. Input-output device system 320 may include one or more user interfaces or input/output (I/O) devices, for example, one or more display device systems 332, speaker device systems 334, keyboards, mice, joysticks, track pads, touch screens or other transducers employed by a user to indicate a particular selection or series of selections of various graphical information. Input-output device system 320 may include a sensing device system 325 configured to detect various characteristics including, but not limited to, at least one of tissue characteristics (e.g., electrical characteristics such as tissue impedance, electric potential of a tissue surface, tissue conductivity, tissue type, tissue thickness) and thermal characteristics such as temperature. In this regard, the sensing device system 325 may include one, some, or all, of the transducers 306 (or 220 in Figure 4 or 406 of Figure 7) of the transducer-based device 300, including the internal components of such transducers shown in Figure 7, such as the electrodes 415 and temperature sensors 408.

Transducer-activation device system 322 may also include an energy source device system 340 including one or more energy source devices connected to transducers 306. In this regard, although Figures 5 and 6 show a communicative connection between the energy source device system 340 and the controller 324 (and its data processing device system 310), the energy source device system 340 may also be connected to the transducers 306 via a communicative connection that is independent of the communicative connection with the controller 324 (and its data processing device system 310). For example, the energy source device system 340 may receive control signals via the communicative connection with the controller 324 (and its data processing device system 310), and, in response to such control signals, deliver energy to, receive energy from, or both deliver energy to and receive energy from one or more of the transducers 306 via a communicative connection with such transducers 306 (e.g., via one or more communication lines through catheter body or elongate shaft member 314, elongated cable 316 or catheter sheath 312) that does not pass through the controller 324. In this regard, the energy source device system 340 may provide results of its delivering energy to, receiving energy from, or both delivering energy to and receiving energy from one or more of the transducers 306 to the controller 324 (and its data processing device system 310) via the communicative connection between the energy source device system 340 and the controller 324.

The energy source device system 340 may, for example, be connected to various selected transducers 306 to selectively provide energy in the form of electrical current or power, light or low temperature fluid to the various selected transducers 306 to cause ablation of tissue. The energy source device system 340 may, for example, selectively provide energy in the form of electrical current to various selected transducers 306 and measure a temperature characteristic, an electrical characteristic, or both at a respective location at least proximate each of the various transducers 306. The energy source device system 340 may include various electrical current sources or electrical power sources as energy source devices. In some embodiments, an indifferent electrode 326 is provided to receive at least a portion of the energy transmitted by at least some of the transducers 306. Consequently, although not shown in Figures 5 and 6, the indifferent electrode 326 may be communicatively connected to the energy source device system 340 via one or more communication lines in some embodiments. In addition, although shown separately in each of Figures 5 and 6, indifferent electrode 326 may be considered part of the energy source device system 340 in some embodiments. In various embodiments, indifferent electrode 326 is positioned on an external surface (e.g., a skin-based surface) of a body that comprises the bodily cavity into which at least transducers 306 are to be delivered.

It is understood that input-output device system 320 may include other systems. In some embodiments, input-output device system 320 may optionally include energy source device system 340, transducer-based device 300 or both energy source device system 340 and transducer-based device 300 by way of non-limiting example. Input-output device system 320 may include the memory device system 330 in some embodiments.

Structure 308 may be delivered and retrieved via a catheter member, for example, a catheter sheath 312. In some embodiments, a structure provides expansion and contraction capabilities for a portion of the medical device (e.g., an arrangement, distribution or array of transducers 306). The transducers 306 may form part of, be positioned or located on, mounted or otherwise carried on the structure and the structure may be configurable to be appropriately sized to slide within catheter sheath 312 in order to be deployed percutaneously or intravascularly. Figures 5 and 6 show one embodiment of such a structure. In some embodiments, each of the elongate members 304 includes a respective distal end 305 (only one called out in each of Figures 5 and 6), a respective proximal end 307 (only one called out in each of Figures 5 and 6) and a respective intermediate portion 309 (only one called out in each of Figures 5 and 6) positioned between the proximal end 307 and the distal end 305. The respective intermediate portion 309 of each elongate member 304 includes a first or front surface 318a that is positionable to face an interior tissue surface within a bodily cavity and a second or back surface 318b opposite across a thickness of the intermediate portion 309 from the front surface 318a. In some embodiments, each of the elongate members 304 is arranged front surface 318a-toward-back surface 318b in a stacked array during an unexpanded or delivery configuration similar to that described in International Publication No. WO 2012/100184, published July 26, 2012 and International Publication No. WO 2012/100185, published July 26, 2012. In many cases, a stacked array allows the structure 308 to have a suitable size for percutaneous or intravascular delivery. In some embodiments, the elongate members 304 are arranged to be introduced into a bodily cavity distal end 305 first. An elongate shaft member 314 is configured to deliver structure 308 through catheter sheath 312, according to some embodiments. According to various embodiments, the elongate shaft member 314 includes a proximal end portion 314a and a distal end portion 314b, the distal end portion 314b physically coupled to structure 308. According to various embodiments, the elongate shaft member 314 may include a length to position distal end portion 314b (and structure 308 in some embodiments) at a desired location within a patient's body while maintaining the proximal end portion 314a at a location outside the patient's body. In some embodiments, the proximal end portion 314a may be coupled to a housing 319. Housing 319 may include or enclose various actuators that may be configured to manipulate various portions of the catheter, including, but not limited to, (a) portions of the elongate shaft member 314, portions of structure 308, or both (a) and (b). According to various embodiments, housing 319 may take the form of a handle that is directly manipulable by a user. U.S. Patent No. 9,452,016, issued September 27, 2016, provides possible examples of a housing and accompanying actuators that may be utilized as housing 319.

The transducers 306 can be arranged in various distributions or arrangements in various embodiments. In some embodiments, various ones of the transducers 306 are spaced apart from one another in a spaced apart distribution in the delivery configuration shown in Figure 5. In some embodiments, various ones of the transducers 306 are arranged in a spaced apart distribution in the deployed configuration shown in at least Figure 6. In some embodiments, various pairs of transducers 306 are spaced apart with respect to one another. In some embodiments, various regions of space are located between various pairs of the transducers 306. For example, in Figure 6, the transducer-based device 300 includes at least a first transducer 306a, a second transducer 306b, and a third transducer 306c (all collectively referred to as transducers 306). In some embodiments, each of the first, the second, and the third transducers 306a, 306b, and 306c are adjacent transducers in the spaced apart distribution. In some embodiments, the first and the second transducers 306a, 306b are located on different elongate members 304, while the second and the third transducers 306b, 306c are located on a same elongate member 304. In some embodiments, a first region of space 350 is between the first and the second transducers 306a, 306b. In various embodiments, a first region of space 350 is between the respective electrodes 315a, 315b of the first and the second transducers 306a, 306b. In some embodiments, the first region of space 350 is not associated with any physical portion of structure 308. In some embodiments, a second region of space 360 associated with a physical portion of device 300 (e.g., a portion of an elongate member 304) is between the second and the third transducers 306b, 306c (and their respective electrodes 315b, 315c). In various embodiments, the second region of space 360 is between the respective electrodes 315b, 315c of the second and the third transducers 306b, 306c. In some embodiments, each of the first and the second regions of space 350, 360 does not include a transducer of transducer-based device 300. In some embodiments, each of the first and the second regions of space 350, 360 does not include any transducer. It is noted that other embodiments need not employ a group of elongate members 304 as employed in the illustrated embodiment. For example, other embodiments may employ a structure having one or more surfaces, at least a portion of the one or more surfaces defining one or more openings in the structure. In these embodiments, a region of space not associated with any physical portion of the structure may extend over at least part of an opening of the one or more openings. In some embodiments, the transducers of the plurality of transducers (e.g., at least a group of the transducers 306) may be circumferentially arranged about an axis (e.g., axis 323, Figure 6) of the structure 308 at least in the state in which the structure 308 is in the deployed configuration, the axis intersecting both the first portion of the structure (e.g., portion 308c in Figure 6) and the second portion of the structure (e.g., portion 308d in Figure 6) in the state in which the structure 308 is in the deployed configuration. According to various embodiments, portions 308c and 308d may each include a respective polar region of the structure 308 in the deployed configuration. In other example embodiments, other structures may be employed to support or carry transducers of a transducer-based device such as a transducer-based catheter. For example, an elongated catheter member may be used to distribute the transducers in a linear or curvilinear array. Basket catheters or balloon catheters may be used to distribute the transducers in a two-dimensional or three-dimensional array.

According to some embodiments, a system is provided that may include an input-output device system (e.g., 120, 320) that may, in some embodiments, include a catheter that includes a plurality of transducers (e.g., transducers 220, 306, 406). The catheter may include the catheter body to which the plurality of transducers (or the structure on which the transducers reside) is physically coupled (e.g., catheter 206, and elongate shaft member 314). In some embodiments, the catheter may also include other components such as catheter sheath 312. According to various embodiments, different portions of the catheter are manipulable to in turn manipulate various ones of the plurality of transducers (e.g., transducers 220, 306, 406) into various degrees of contact with a tissue wall within a patient's body (e.g., patient 361). According to various embodiments, at least some transducers (e.g., at least some of the transducers 220, 306, 406), such as a first set of transducers, of the plurality of transducers of the catheter device system are arranged in a first spatial distribution (e.g., the spaced apart distribution associated with the deployed configuration of Figure 6), the distribution positionable in a bodily cavity of a patient. The bodily cavity is defined by at least a tissue wall, and, according to various embodiments, each transducer of the at least some transducers, such as at least the first set of transducers of the plurality of transducers, is configured at least to sense a degree of contact between the transducer and the tissue wall. In some embodiments, each particular transducer of the at least some transducers (e.g., at least the first set of transducers) of the plurality of transducers of the catheter may be configured to sense or detect a degree of transducer-to-tissue contact between at least a portion of the particular transducer and the tissue wall. Various methods may be executed to determine the degree of transducer-to-tissue contact including, by way of non-limiting example, techniques including sensing impedance, sensing permittivity, sensing the presence or absence of flow of a fluid (e.g., a bodily fluid), or by sensing contact force or pressure. U.S. Patent No. 8,906,011, issued December 9, 2014, describes example transducer sensing techniques. In some embodiments, the tissue-contacting portion of the transducer itself directly senses the degree of tissue contact. In some embodiments, a portion of the transducer other than the tissue-contacting portion of the transducer is configured to sense the degree of contact between the tissue wall and the tissue-contacting portion of the transducer. In some embodiments, the tissue-contacting portion of the transducer is provided by an electrode.

According to some embodiments, the at least some transducers (e.g., at least the first set of transducers) of the plurality of transducers of the catheter (e.g., transducer-based device 200 or transducer-based device 300) may be configured to provide a plurality of contact signal sets to the controller 324 or its data processing device system 310. Each contact signal set may indicate a degree of transducer-to-tissue contact between each transducer (e.g., a transducer 220, 306, 406) and a tissue surface in the bodily cavity.

In some embodiments, at least some transducers (e.g., at least some of the transducers 220, 306, 406), such as a second set of transducers, of the plurality of transducers of the catheter are configured to sense one or more electrical properties or characteristics of or generated at least in part by a body (e.g., the body of the patient 361) including the bodily cavity. In some embodiments, such transducers (e.g., at least the second set of transducers) may be configured to provide a plurality of tissue-electrical-information signal sets to the controller 324 or its data processing device system 310. In some embodiments, such transducers (e.g., at least the second set of transducers) may be configured to provide a plurality of tissue-electrical-information signal sets (e.g., electrophysiological signal sets) to the controller 324 or its data processing device system 310 throughout movement of at least a portion of the catheter (e.g., transducer-based device 200 or transducer-based device 300) among a sequence of locations of the at least the portion of the catheter in the bodily cavity. In some embodiments, the plurality of tissue-electrical-information signal sets indicate an electrical property set of or associated at least in part with a body including the bodily cavity and detected by at least the second set of transducers. The electrical property set may be tissue electrical characteristics as discussed above, possibly including different electrical property types, such as electric potential or electrical impedance, e.g., as detected by the respective transducers (e.g., transducers 220, 306, 406). In some embodiments, the plurality of tissue-electrical-information signal sets are generated by and provided to (and consequently, are received by) the controller 324 or its data processing device system 310 at least in a state representative of the second set of transducers being located in the bodily cavity. The state associated with the second set of transducers being located in the bodily cavity may be a state in which the second set of transducers are actually located in the bodily cavity, or may be, e.g., a simulation state in which it is simulated, e.g., for quality-control, training, or testing, that the second set of transducers are located (but not actually located) in the bodily cavity. In some embodiments, the second set of transducers (which may be configured to sense one or more electrical properties or characteristics of or generated at least in part by a body) and the first set of transducers (which may be configured to sense or detect a degree of transducer-to-tissue contact between at least a portion of the respective transducer and the tissue wall) may be the same one or more transducers (e.g., transducers 220, 306, 406). In other embodiments, the first set of transducers, the second set of transducers, or the first and second sets of transducers include at least one transducer not included in the other set. Transducer-to-tissue contact between at least a portion of the respective transducer and the tissue wall may be determined via various techniques, including those described above in this disclosure.

In some embodiments, one or more devices of the catheter-device-location tracking system or navigation system shown in at least Figure 2 or Figure 3 is or are configured to provide location information derived from a plurality of location signal sets to (and consequently, received by) the controller 324 or its data processing device system 310. According to various embodiments, the location information may indicate a plurality of locations in a bodily cavity in response to movement of at least part of a transducer-based device (e.g., transducer-based device 200, 300, or 400 in some embodiments) in the bodily cavity. In some embodiments, each location signal set may be indicative of a respective location of the plurality of locations. In some embodiments, the location information may indicate movement of at least part of a transducer-based device (e.g., transducer-based device 200, 300, or 400 in some embodiments) through or between a plurality of locations in a bodily cavity. In some embodiments, each location signal set may be indicative of a respective location of the plurality of locations. In some embodiments, each location signal set may be indicative of a respective location in a sequence of locations at which at least a portion of a catheter has been sequentially located in a bodily cavity, according to some embodiments. For example, with respect to at least Figure 2 or Figure 3, at least a portion of the catheter or transducer-based device (e.g., transducer-based device 200, 300, or 400) may be moved or progressed through a sequence of locations in a chamber of the heart or other bodily cavity of the patient 361 (or through a quality-control, training, or testing environment) in the presence of an electric field set (e.g., one or more electric fields generated by the external electrodes 256a, 256b, 256c, 256d, 256e, 256f) or a magnetic field set (e.g., one or more magnetic fields generated by magnetic field generation sources 257w, 257x, 257y). As the portion of the catheter is moved through the sequence of locations, at least some of the catheter's transducers (e.g., transducers 220, 306, 406 (or, e.g., 277 in the case of magnetic-field-based systems)) may be configured to generate each location signal set as detected strengths of the respective field(s), which the controller 324 or its data processing device system 310 may then be configured to utilize to generate a three-dimensional location of the at least the portion of the catheter (e.g., transducer-based device 200, 300, or 400) or its transducers (e.g., transducers 220, 306, 406 (and, e.g., 277 in the case of magnetic-field-based systems)) for the respective location in the sequence of locations, according to some embodiments. In this regard, in some embodiments, the navigation system may be deemed to include the respective transducer(s) (e.g., transducers 220, 306, 406 (or, e.g., 277 in the case of some magnetic-field-based systems)) that detected the field strength(s), the field-generating devices (e.g., the external electrodes 256a, 256b, 256c, 256d, 256e, 256f in the case of electric field(s); and, e.g., magnetic field generation sources 257w, 257x, 257y in the case of magnetic field(s)), or both the respective transducers and the field-generating devices. In some embodiments, the controller 324 or its data processing device system 310 may be considered at least part of the navigation system.

At least in light of the above discussion, in some embodiments, the navigation system is configured to generate location information that may be derived from one or more location signal sets at least in response to one or more electric or magnetic fields producible by one or more devices of the navigation system. In some embodiments, the one or more devices that generate the one or more electric or magnetic fields may be configured to operate outside a body including the bodily cavity, such as the external electrodes 256a, 256b, 256c, 256d, 256e, 256f in the case of electric field(s), and magnetic field generation sources 257w, 257x, 257y in the case of magnetic field(s). According to some embodiments, the electric or magnetic field sensing devices of the catheter (e.g., transducers 220, 306, 406 or one or more magnetic field transducers 277) are configured to generate location information at least in response to the one or more electric or magnetic fields producible by one or more devices of the navigation system. In this regard, the navigation system, in some embodiments, may include the transducers 220, 306, 406 (or, e.g., 277 in the case of magnetic-field-based systems) of the catheter that sense the one or more electric or magnetic fields and consequently generate the plurality of location signal sets. According to some embodiments, each transducer of at least some of the transducers of the catheter (e.g., transducer-based device 200, 300, or 400 in some embodiments) is configured to not only sense an electric field for location determination purposes, but also to perform one or more other functions (e.g., ablation, pacing, tissue electric potential detecting or measuring, transducer-to-tissue contact detecting or measuring, etc.). In some embodiments, the navigation system may be configured to provide location information to (which is, consequently, received by) the controller 324 or its data processing device system 310, the location information indicating locations of at least part of a transducer-based device (e.g., transducer-based device 200, 300, or 400). For example, in some embodiments, the location information may be based at least on, or include (a) a location of the at least part of the transducer-based device from sensed electric or magnetic fields generated by the navigation system, and (b) transducer-to-tissue-contact sensing results provided by transducers of the transducer-based device. However, in some embodiments, a location of the at least part of the transducer-based device may be indicated at least by (a), and not (b), for example, when (a) is determined with respect to a 3D model of the bodily cavity. In some embodiments, the location information indicates locations of at least part of a transducer-based device (e.g., transducer-based device 200, 300, or 400) relative to a tissue surface in a bodily cavity. In some embodiments, the location information indicates locations of at least part of a transducer-based device (e.g., transducer-based device 200, 300, or 400) relative to a reference device (e.g., reference device 252 (Figure 2) or reference device 257z (Figure 3), in some embodiments) of a navigation system.

Various tissue ablation procedures may include having the transducer-based device (e.g., transducer-based device 200, 300, or 400, in some embodiments) transmit tissue ablation energy at each of a plurality of locations in a bodily cavity. In various embodiments, movement of at least part of the transducer-based device occurs between at least some of the plurality of locations in the bodily cavity. In various embodiments, movement of at least part of the transducer-based device in the bodily cavity occurs between, during, or between and during each of the transmission of tissue ablative energy at each of at least some of the plurality of locations in the bodily cavity. Movement of at least the part of the transducer-based device may be motivated for different reasons. For example, movement of at least the part of the transducer-based device between different locations can allow for the formation of a larger ablated region (e.g., a larger lesion) than would be possible if tissue ablative energy was transmitted while at least the part of the transducer-based device remained at a single location in the bodily cavity. In some embodiments, movement of at least the part of the transducer-based device between a plurality of locations may be employed to form relatively long, or relatively long and continuous lesions in bodily tissue under the effects of the transmitted tissue ablative energy. In some embodiments, the continuous lesions may take the form of closed circumferential lesions (e.g., circumferential lesions surrounding an anatomical feature, such a pulmonary vein). In some embodiments, the continuous lesions may take the form of continuous lesions connecting various anatomical features or connecting various ablated regions (for example, lesions connecting to circumferential lesions in a Cox-Maze procedure).

In attempting to complete continuous lesion lines, tension may arise between ensuring that the individual locations are ablated adequately to result in a lesion that is continuous or contiguous (and transmural in some embodiments), while minimizing the total energy applied to external anatomical structures that may be desired to not be exposed to certain levels of ablative energy.

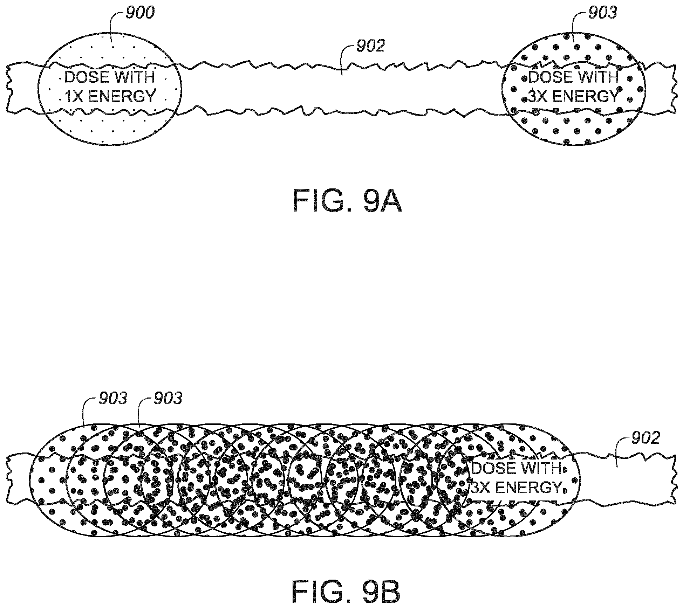

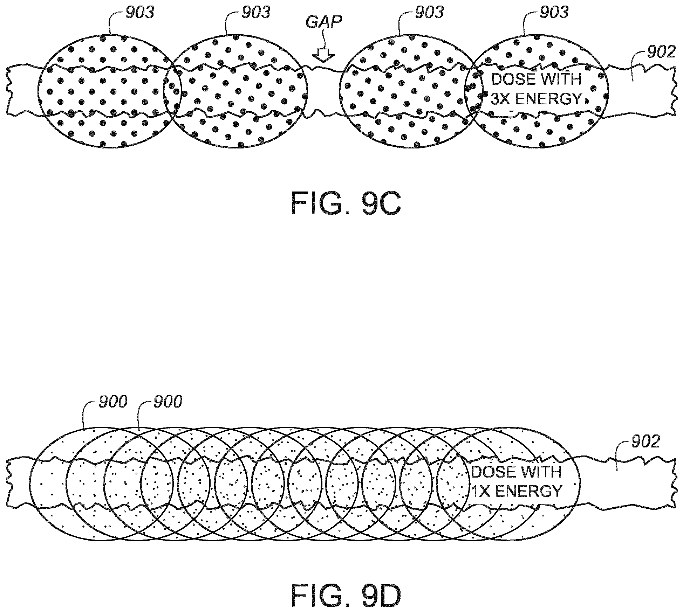

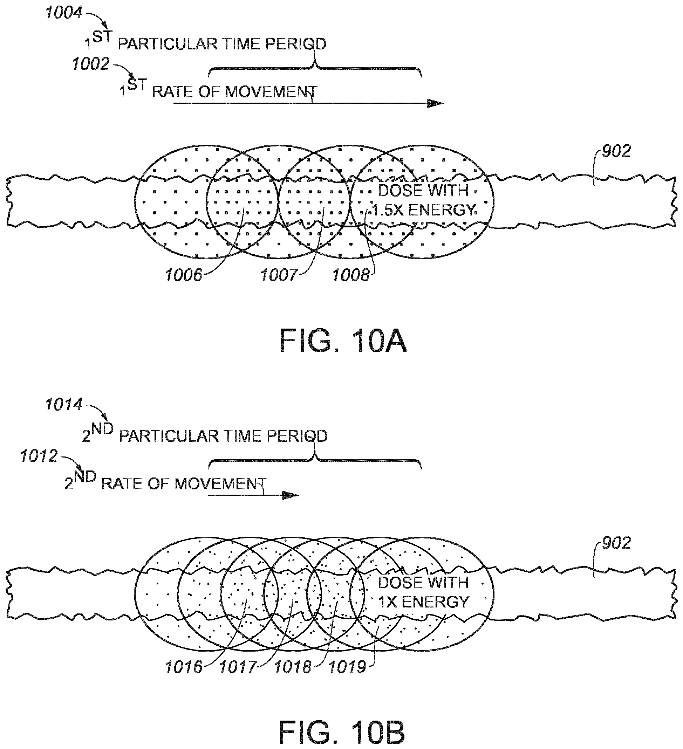

To illustrate this concept, in Figure 9A, a single "dose" 900 of delivered or transmitted tissue ablative energy is shown against a rectangular block representing a tissue layer 902 (e.g., a myocardium layer) that would be insufficient to form a transmural lesion due to the inadequacy of the amount of energy included in the dose (i.e., represented by the illustrated relatively small dot size of the dots in the oval representative of dose 900 representing the relatively low amount of energy in such dose). Figure 9A additionally shows an application of a dose 903 of delivered or transmitted tissue ablative energy that is equal to three times the energy included in dose 900 (i.e., dose 903 is represented in Figure 9A with larger dots compared to the dot size of the oval representative of dose 900). In the case of the delivery of the dose 903, a transmural lesion results in the tissue layer 902 at that location, according to various embodiments. In some embodiments, in the case of the delivery of the dose 903, a transmural lesion results in the tissue layer 902, while reducing occurrences of delivering additional energy that may damage a neighboring external anatomical structure. It is noted that the use of a "single dose" value versus a "three dose" value in the examples described with respect to at least Figures 9A, 9B, 9C, 9D, 10A, and 10B is simply to illustrate the principle in a way amenable to simple illustrations, and that the actual quantity or amount of energy delivered per dose (i.e., the amount of "X" and the coefficients thereof shown at least in Figures 9A, 9B, 9C, 9D, 10A, and 10B) will be dependent on the system, operating environment (e.g., including tissue or blood flow characteristics), and ablation regimen applied.

To make a contiguous elongated transmural lesion in the tissue layer 902, multiple applications of relatively high intensity doses may be required (e.g., multiple applications of dose 903 in the example described above with respect to Figure 9A). In some embodiments, repositioning of the transducer-based device (e.g., transducer-based device 200, 300, or 400, in some embodiments) occurs between the application of doses. In some embodiments, the transducer-based device may be activated to apply doses 903 during or after each of several relatively small position changes to avoid gaps in the resulting elongated lesion. Such gaps, as exemplified in Figure 9C, can break the continuity of a lesion. The left side of Figure 9C illustrates a relatively larger position change, such that the applied doses just merge with each other, according to some embodiments. This relatively-large-position-change approach may, in turn, require relatively precise positioning to just merge or just overlap the doses and, as such gaps may result if this positioning requirement is not achieved, as illustrated in Figure 9C. With the relatively-small-position-change approach, overlapping of the respective lesions formed by each dose 903 application can be more easily controlled, and such overlapping can more reliably result in a contiguous and transmural lesion in suitable contexts. Figure 9B shows an elongated contiguous and transmural lesion formed by the application of multiple applications of dose 903 (only two called out in Figure 9B). In some of these embodiments, each application of dose 903 forms a respective individual transmural lesion, the individual lesions combining to form the elongated contiguous and transmural lesion.

It is noted that, although a transmural lesion results at least in the example of Figure 9B, the contiguous elongated lesion receives relatively many (e.g., compared to the example of Figure 9C) of the relatively high energy doses 903 and, therefore, the risk of damage to other neighboring anatomical structures is relatively higher than it would be with the application of only a single one of the doses 903 set at a single position without overlapping (e.g., as shown by the right-hand side of Figure 9A). For example, excessive transmitted energy during thermal cardiac ablation (e.g., RF ablation) should be avoided in cardiac regions proximate the esophagus and phrenic nerve. Testing conducted by the inventors has also showed a similar dose-concentration-dependency risk to neighboring anatomical structures when producing pulsed field ablation ("PFA") lesions, i.e., that the application of excessive PFA doses raises the risk of non-specific damage to extra-cardiac structures (e.g., esophagus, phrenic nerve, etc.) in the case of ablating tissue in an intra-cardiac cavity. Although it was conventionally thought that these cardiac-adjacent structures are more resistant to PFA pulses than myocardial tissue, it is believed that these structures are vulnerable at higher voltage gradients or concentrations. For example, in one test performed by the Applicant, Kardium Inc., of one particular pulse design, a shallow esophageal lesion was observed in an acute test of direct esophageal ablation, but no lesion was created in a chronic test using approximately 25% fewer pulses.

In some embodiments, the transducer-based device (e.g., transducer-based device 200, 300, 400, in some embodiments) may be activated to apply doses while rigorously controlling the positioning of the transducer-based device, such that the individual transmural lesions that are formed by each dose 903 would just merge or just overlap. Again, each dose 903 is configured to be sufficient to form a respective individual transmural lesion (in this example), and the minimal overlapping (or overlapping within a threshold amount) of the individual lesions is configured to reduce the risk of damage to other neighboring anatomical structures not intended to be ablated or damaged, according to various embodiments.

In some embodiments, lower intensity doses (e.g., doses, such as dose 900 described above with respect to Figure 9A) are applied or delivered to form a contiguous elongated lesion that is transmural. In some embodiments, each of the lower intensity doses is configured to form an individual lesion that is not transmural (e.g., dose 900 described above). In some embodiments, each of the lower intensity doses is configured to form an individual lesion that is not transmural (e.g., dose 900 described above), but when combined in an overlapping arrangement with other lower intensity doses, is configured to form a combined lesion that is transmural. In some embodiments, repositioning of the transducer-based device occurs between the application of the relatively lower intensity doses (e.g., doses 900 in this example). In some embodiments, the transducer-based device may be activated to apply doses 900 after many relatively small position changes to avoid gaps in the elongated lesion.

For example, Figure 9D shows an elongated contiguous and transmural lesion formed by the application of multiple applications of dose 900 (only two called out with reference numerals in Figure 9D). In some of these embodiments, each application of dose 900 forms a respective individual lesion that is not transmural, with the individual lesions combining to form the elongated contiguous lesion that is transmural. It is noted that, although each individual lesion that is formed is not transmural, the doses 900 overlap in a manner that delivers sufficient energy to cause the resulting elongated continuous lesion to be transmural. Further, in Figure 9D, the overlapping doses 900 deliver sufficient energy to assure that the resulting elongated continuous lesion is transmural, while reducing over-energizing conditions that can potentially cause damage to neighboring anatomical structures not intended to be ablated or damaged. In particular, the concentration of energy applied in the example of Figure 9D is less than the concentration of energy applied in the example of Figure 9B. Therefore, the example of Figure 9B exhibits relatively lower risk of damage to other external anatomical structures. (It is noted that the illustration of particular numbers of doses in the figures, such as Figure 9B and Figure 9D, are merely examples and that other dosage numbers and applied energy amounts per dose (e.g., besides 1X (a single unit of energy) or 3X (three times the single unit of energy)) may be utilized in other embodiments. Similarly, other degrees of spatial dosage overlap than those illustrated in Figure 9B and Figure 9D may be utilized in other embodiments.

Further, applying relatively lower total dosage energy per location (e.g., Figure 9D compared to Figure 9B) can allow for greater degrees of overlap between dosage-application locations as compared to applying relatively higher total dosage energy per location (e.g., Figure 9B compared to Figure 9D), since energy application is less concentrated at each location. Allowance of greater overlap between dosage-application locations can allow for a greater margin of error in positional requirements when determining a next dosage-application location, since there is less of a concern of damage to non-targeted adjacent anatomical structures due to excessive energy. Since determining a precise location of a transducer or portion of a transducer-based device by a navigation system can be relatively difficult due to motion of the heart during the cardiac cycle, motion of the transducer-based device itself due to an inherent imprecision of movement caused by the physician or healthcare practitioner, and motion of the body of the patient caused by the pulmonary cycle, an increase in margin of error in positional requirements when determining a next dosage-application location can be beneficial.

The word "dose" referred to in this disclosure may have different meanings based on the type of ablation delivered. As it pertains to PFA, "dose" may refer to the high voltage output pulse count. In some PFA applications, the degree of tissue ablation increases cumulatively with the high voltage pulse count, even with increasing time intervals between successive doses. In some PFA applications, a dose may refer to a group of high voltage pulses delivered with a particular inter-pulse spacing or a particular pulse periodicity. In some embodiments, successive groups (e.g., doses) of the delivered pulses are separated by a time period that is different than the particular inter-pulse spacing or the particular pulse periodicity. In some embodiments, successive groups (e.g., doses) of the delivered pulses are separated by a time period that is greater than the particular inter-pulse spacing or the particular pulse periodicity. For example, in some embodiments, successive groups (e.g., doses) of the delivered pulses may be separated by a time period measured in seconds while the particular inter-pulse spacing or the particular pulse periodicity may be measured in milliseconds or nanoseconds. In some embodiments, each group (e.g., dose) of the delivered pulses are delivered during a respective one of a plurality of cardiac cycles. In some embodiments, delivery of each group (e.g., dose) of high voltage pulses is gated to a particular signal feature (e.g., a particular signal feature in an electrophysiological signal or a particular signal feature in a pacing signal). In some embodiments, the number of pulses in each group (e.g., dose) of the delivered pulses may be limited to a particular number to avoid an undesired physiological effect. For example, the number of pulses in each group (e.g., dose) of pulses may be limited to avoid exceeding a desired limit of micro-bubble formation. Micro-bubbles may occur in PFA applications due to electrolysis effects caused by the delivered current, and may lead to procedure complications. In some embodiments, the number of pulses in each group (e.g., dose) of pulses may be limited to avoid creating undesired thermal effects. In some embodiments, a time interval between successive groups (e.g., doses) of the delivered pulses may be selected to restore various physiological parameters to a desired level between the deliveries of successive groups (e.g., doses) of pulses. In some embodiments, a time interval between successive groups (e.g., doses) of the delivered pulses may be selected to allow a high voltage generation system to reset, or recharge to a desired level between the delivery of successive groups (e.g., doses) of pulses.

In the case of a thermal ablation transducer device (e.g., an RF catheter) that is being moved relatively quickly across a tissue surface when transmitting ablative energy, the thermal ablation transducer device may need to be operated at higher power in order to achieve the same ablation depth than when the thermal ablation transducer is moved relatively slowly. When required to move relatively quickly, the thermal ablation transducer device may also be run at higher temperature in order to achieve this thermal penetration in less time. Dose then in this context may refer to a temperature measured at a target depth, and power is then being adjusted as a function of the rate of movement in order to achieve this goal. In some embodiments, in thermal ablation applications, dose may refer to a targeted thermal damage integral at a desired ablation depth (e.g., calculated by an Arrhenius function or equivalent thermal model of time and temperature contributions to ablation). In some embodiments, a dose may refer to a particular amount of tissue ablative energy deliverable per unit time. In some embodiments, a dose may refer to a particular amount of tissue ablative energy deliverable per unit of tissue that is to be treated such as, by way of non-limiting example, length, area, or volume of tissue to be treated.

According to some embodiments, location information (e.g., provided by a catheter navigation system (e.g., as described above with respect to Figure 2 and Figure 3), in some embodiments) is processed by a data processing device system (e.g., 110, 310) or a controller (e.g., controller 324, in some embodiments) to control the activation of the transducer-based device (e.g., transducer-based device 200, 300, or 400, in some embodiments) to produce individual lesions zones in an overlapping manner. In various embodiments, the overlapping lesion zones result in the formation of an overall lesion zone (e.g., a contiguous lesion) that is transmural. In various embodiments, each of the individual lesion zones is not transmural, but when a group of the individual lesion zones are combined in an overlapping manner, an overall lesion zone that is transmural is produced. In some embodiments, the overall lesion zone is produced while reducing excessive transmitted energy that could undesirably adversely impact neighboring non-targeted anatomical structures.

According to various embodiments, location information (e.g., provided by a catheter navigation system (e.g., as described above with respect to Figure 2 and 3, in some embodiments)) is processed by a data processing device system (e.g., data processing device system 110 or 310, in some embodiments) or a controller (e.g., controller 324, in some embodiments) to control the transducer-based device (e.g., transducer-based device 200, 300, or 400, in some embodiments) in various manners to produce the overlapping lesions. For example, in some embodiments, the temporal rate of ablative energy delivery may be automatically varied (e.g., via various machine-based controls instituted by a data processing device system) based on the speed of movement of at least a part of a transducer-based device as indicated by location information (e.g., provided by a catheter navigation system).

Figures 8A-8C illustrate respective programmed configurations of a data processing device system (e.g., data processing device system 110 or 310), according to some embodiments of the present invention. For example, a programmed configuration may be implemented by the data processing device system being communicatively connected to an input-output device system (e.g., input-output device system 120 or 320) and a memory device system (e.g., memory device system 130 or 330), and being configured by a program stored by the memory device system at least to perform one or more actions (e.g., such as at least one, more, or all of the actions described in any one or more of Figures 8A-8C or otherwise herein). In some embodiments in which the one or more of the programmed configurations illustrated in Figures 8A-8C actually is or are executed at least in part by the data processing device system, such actual execution may be considered a respective method executed by the data processing device system. In this regard, Figures 8A-8C may be considered to represent one or more methods in some embodiments and, for ease of communication, such one or more methods may be referred to simply as 'the method of Figure 8A', 'the method of Figure 8B', and 'the method of Figure 8C' and the like. The blocks shown in each of Figures 8A-8C may be associated with computer-executable instructions of a program that configures the data processing device system to perform the actions described by the respective blocks. According to various embodiments, not all of the actions or blocks shown in each of Figures 8A-8C are required, and different orderings of the actions or blocks shown in each of Figures 8A-8C may exist. In this regard, in some embodiments, a subset of the blocks shown in each of Figures 8A-8C or additional blocks may exist. In some embodiments, a different sequence of various ones of the blocks in each of Figures 8A-8C or actions described therein may exist.

In some embodiments, a memory device system (e.g., memory device system 130 or 330, or a computer-readable medium system) stores the program(s) represented by each of Figures 8A-8C, and, in some embodiments, the memory device system is communicatively connected to the data processing device system as a configuration thereof. In this regard, in various example embodiments, a memory device system is communicatively connected to a data processing device system (e.g., data processing device systems 110 or 310) and stores a program executable by the data processing device system to cause the data processing device system to execute various actions described by, or otherwise associated with, the blocks illustrated in each of Figures 8A-8C for performance of some or all of the corresponding method(s) via interaction with at least, for example, a transducer-based device (e.g., transducer-based device devices 200, 300, or 400, in some embodiments). In these various embodiments, the program may include instructions configured to perform, or cause to be performed, various ones of the block actions described by or otherwise associated with one or more or all of the blocks illustrated in each of Figures 8A-8C for performance of some, or all, of the corresponding method(s).

Figure 8A shows configurations of the data processing device system to behave differently in association with different states, respectively referred to by blocks 804a, 804b, which are within broken-line block 804. In this regard, either or both of the states and corresponding actions set forth in blocks 804a, 804b may actually occur or be executed by the data processing device system (e.g., as in a method) in some embodiments, and, in the case where both states and corresponding actions referred to by blocks 804a, 804b actually occur or are executed by the data processing device system, they may occur in any order, as illustrated by the double-headed broken line arrow shown in Figure 8A between blocks 804a, 804b, according to various embodiments.

In Figure 8A, according to some embodiments, block 802 represents a configuration of the data processing device system (e.g., data processing device system 110 or 310) (e.g., according to a program) to receive, via an input-output device system (e.g., input-output device system 120 or 320), location information indicating locations of at least part of a transducer-based device (e.g., transducer-based device 200, 300, or 400, in some embodiments). In some embodiments, block 802 represents a configuration of the data processing device system (e.g., data processing device systems 110 or 310) (e.g., according to a program) to receive, via an input-output device system (e.g., input-output device system 120 or 320), location information indicating movement of at least part of a transducer-based device through a plurality of locations in a bodily cavity during a particular time period (e.g., particular time period 1004 in Figure 10A or particular time period 1014 in Figure 10B, in some embodiments). According to various embodiments, the location information may be provided by a catheter navigation system (e.g., as described above with respect to at least Figure 2 or Figure 3, in some embodiments). For example, the location information may be derived from a location signal set provided by a catheter navigation system in response to movement of at least part of transducer-based device (e.g., transducer-based device 200, 300, or 400, in some embodiments) between various locations (e.g., various locations in a bodily cavity) (for example, as described above in this disclosure). In some embodiments, the part of the transducer-based device includes a particular part of the transducer-based device that is configured to be deliverable to a bodily cavity. In some embodiments, the part of the transducer-based device includes one or more transducers configured to cause tissue ablation (e.g., transducers 220, 306, or 406, in some embodiments). In some embodiments, the part of the transducer-based device includes one or more transducers (e.g., transducers 220, 306, 406 (or, e.g., 277 in the case of magnetic-field-based systems, in some embodiments)) configured, as the part of the transducer-based device is moved through a sequence of locations, to generate various location signal sets as detected strengths of the respective field(s), which the controller or data processing device system may then be configured to utilize to generate three-dimensional location information of the part of the transducer-based device.

In Figure 8A, according to some embodiments, block 803 represents a configuration of the data processing device system (e.g., data processing device systems 110 or 310) (e.g., according to a program) to determine a rate of movement of the part of the transducer-based device (e.g., transducer-based device 200, 300, or 400, in some embodiments) in the bodily cavity based at least on an analysis of the locations indicated by the received location information. In some embodiments, however, such rate of movement information may merely be provided to or accessed by the data processing device system, e.g., if such information has been pre-determined by another system. According to various embodiments, a rate of movement of the part of the transducer-based device may be determined via a location signal set defining a plurality of locations of the transducer-based device over a period of time (for example, via a catheter navigation system described above with respect to at least Figure 2 or Figure 3). According to various embodiments, a rate of movement of the part of the transducer-based device may be determined when the location signal set is referenced to a reference device (e.g., reference device 252 (Figure 2) or reference device 257z (Figure 3), in some embodiments). According to various embodiments, a rate of movement of the part of the transducer-based device in the bodily cavity, may be determined by the data processing device system determining a net translation vector over a predetermined or determined window of time. In some embodiments, the net translation vector may be determined on a rolling basis, and the magnitude of that net translation vector may be divided by the duration of the window to provide the rate of movement. In some embodiments, the net translation vector may be determined based on the position of the part of the transducer-based device over time, or based on a smoothed estimate of position over time, using any one of a number of smoothing operations known in the art. Such smoothing operations may include by way of non-limiting example, a running average, a finite impulse response, or an infinite impulse response filter acting on the positions of the ablating element over time. Typical rates of movement for the part of the transducer-based device may, in some embodiments, be in a range in the order of 0 -10 mm/s, generating multiple plausible rates of motion over which ablative energy delivery may be modulated. This modulation may lead to meaningful changes in energy delivery from moment to moment, from ablation to ablation within a single procedure, and/or from one procedure to another.

In Figure 8A, according to some embodiments, block 804 represents a configuration of the data processing device system (e.g., data processing device systems 110 or 310) (e.g., according to a program) to cause, via the input-output device system (e.g., via a communicative connection between the input-output device system 120 or 320 and the transducer-based device 200, 300, or 400, in some embodiments), delivery of tissue-ablative energy (e.g., pulsed field ablation energy) during at least part of a particular time period. For example, according to some embodiments, each of block 804a and block 804b represents a possible implementation of at least part of block 804, according to some embodiments. Block 804a represents a configuration of the data processing device system (e.g., data processing device systems 110 or 310) (e.g., according to a program) to cause, via the input-output device system (e.g., input-output device system 120 or 320) and the transducer-based device (e.g., transducer-based device 200, 300, or 400, in some embodiments), delivery of first tissue-ablative energy during a duration of a first particular time period in accordance with a first energy waveform parameter set at least in response to a first state in which at least part of the location information indicates at least a first rate of movement of the part of the transducer-based device. In various embodiments, the first tissue-ablative energy caused to be delivered during the duration of the first particular time period in accordance with the first energy waveform parameter set is configured to cause tissue ablation.

For example, Figure 10A illustrates one example of such a first state in which the part of the transducer-based device exhibits a first rate of movement 1002. In this example, it is assumed, merely for illustration purposes, that approximately three times ("3X") the amount of energy of dose 900 delivered at a particular location is needed to produce a transmural lesion in tissue at that particular location. With this first rate of movement 1002, the data processing device system may be configured to utilize a first energy waveform parameter set that defines that each energy application (e.g., a dose in some embodiments) should include 1.5 times ("1.5X") the energy of dose 900, such that the data processing device system may be configured to cause delivery of four doses, each at such 1.5X energy, at the four corresponding locations shown in the example of Figure 10A during the duration of the first particular time period 1004. Such a pattern produces dosage-application overlap regions 1006, 1007, and 1008 that each are subjected, in total, to three times ("3X") the energy of dose 900. Accordingly, the dosage-application overlap regions 1006-1008 collectively produce a contiguous, transmural lesion at or close to the needed three doses of delivered energy to produce the transmural lesion, while limiting the delivery of excessive energy in an attempt to reduce the risk of damaging non-targeted neighboring anatomical structures. Again, it should be noted that the 1.5X, 3X, and other dosage energy values provided herein merely are for illustration purposes.

Returning to Figure 8A, according to some embodiments, block 804b represents a configuration of the data processing device system (e.g., data processing device systems 110 or 310) (e.g., according to a program) to cause, via the input-output device system (e.g., input-output device system 120 or 320) and the transducer-based device (e.g., transducer-based device 200, 300, or 400, in some embodiments), delivery of second tissue-ablative energy during a duration of a second particular time period in accordance with a second energy waveform parameter set at least in response to a second state in which the at least part of the location information indicates at least a second rate of movement of the part of the transducer-based device. According to some embodiments, the second tissue-ablative energy caused to be delivered during the duration of the second particular time period in accordance with the second energy waveform parameter set is configured to cause tissue ablation. In some embodiments, the second rate of movement is different than the first rate of movement. According to various embodiments, the second energy waveform parameter set is different than the first energy waveform parameter set.

For example, Figure 10B illustrates one example of such a second state in which the part of the transducer-based device exhibits a second rate of movement 1012 that is approximately 1/3 (one-third) of the first rate of movement 1002 shown in Figure 10A. In this example, it is also assumed, merely for illustration purposes, that approximately three times ("3X") the amount of energy of dose 900 delivered at a particular location is needed to produce a transmural lesion in tissue at that particular location. With this second rate of movement 1012, the data processing device system may be configured to utilize a second energy waveform parameter set that defines that each energy application (e.g., a dose in some embodiments) should include the same amount of energy ("1X") as dose 900, such that the data processing device system may be configured to cause delivery of six doses, each at such 1X energy, at the six corresponding locations shown in the example of Figure 10B during the duration of the second particular time period 1014. In this example, the second particular time period 1014 is the same as the first particular time period 1004. The applied dosage pattern shown in Figure 10B produces dosage-application overlap regions 1016, 1017, 1018, and 1019 that each are subjected, in total, to three times ("3X") the energy of dose 900. Accordingly, the dosage-application overlap regions 1016-1019 collectively produce a contiguous, transmural lesion at or close to the needed 3X of delivered energy to produce the transmural lesion (in this example), while limiting the delivery of excessive energy in an attempt to reduce the risk of damaging non-targeted neighboring anatomical structures.

In this regard, it can be seen that, in some embodiments, the rate of movement of at least part of the transducer-based device may be monitored to control energy delivery of one or more transducers of the transducer-based device, for instance, according to at least some of the principles described above with reference to Figures 9 to improve the likelihood of achieving a contiguous, transmural tissue lesion while reducing the risk of damage to neighboring non-targeted anatomical structures. In this regard, in some embodiments, the aforementioned first energy waveform parameter set and second energy waveform parameter set may be tailored to the respective rate of movement of the at least part of the transducer-based device experienced in the respective first and second states associated with blocks 804a and 804b in Figure 8A. In some embodiments, each energy waveform parameter set may define one or more parameters that may include, by way of non-limiting example, an amount or rate of energy applied or delivered (e.g., voltage, current, or both; a duty cycle, a pulse width or number of pulses; or dosage parameter(s) or numbers of dosages per location), a duration of energy application or delivery, a type of energy applied or delivered (e.g., thermal or PFA; monopolar, bipolar, or blended monopolar/bipolar), or a combination thereof. Reasons for differences in energy waveform parameter sets for different rates of movement or circumstances are provided in more detail below, such as the delivery of different amounts of energy not only for different rates of movement, but also, e.g., when applying a first or last energy application or dose in a sequence, as compared to an energy application within an interior of such sequence, in some embodiments.

According to various embodiments, the first particular time period is equal to the second particular time period, as with the examples of Figure 10A and Figure 10B. In some embodiments, the first particular time period and the second particular time period correspond to, or are provided by, a particular time period subsequent to the reception of at least part of the location information. In some embodiments, the first particular time period and the second particular time period correspond to, or are provided by, a particular time period subsequent to a time interval in which a determination, by the data processing device system (e.g., data processing device system 110 or 310), of a rate of movement of the part of the transducer-based device (e.g., transducer-based device 200, 300, or 400, in some embodiments) occurs. In some embodiments, as with the examples of Figure 10A and Figure 10B, in the first state, the part of the transducer-based device moves through at least some of the locations (e.g., corresponding to the ovals in Figure 10A) during the duration of the first particular time period, and in the second state, the part of the transducer-based device moves through at least some of the locations (e.g., corresponding to the ovals in Figure 10B) during the duration of the second particular time period. In some embodiments, the rate of movement of the part of the transducer-based device may be determined by the data processing device system based on at least part of the received location information (e.g., received per block 802 in Figure 8A) indicating a first subset of the plurality of locations. For instance, not all locations that a part of a transducer-based device moves through (e.g., as informed by a catheter navigation system) may be utilized in order to determine a rate of movement. According to various embodiments, in either the first state or the second state, the part of the transducer-based device moves through a second subset of the plurality of locations during the duration of the respective one of the first particular time period and the second particular time period. Accordingly, in some embodiments, a catheter navigation system (e.g., as described above with respect to Figure 2 and Figure 3) may provide location signals reflective of various locations of the plurality of locations through which the transducer-based device (or part thereof) moves, before, during, and sometimes, after the first particular time period or the second particular time period, and the rate of movement may be determined based on a subset of those locations, such as a subset of locations visited before or during the respective particular time periods. Accordingly, in some embodiments, determination of the first rate of movement or the second rate of movement may occur prior to the start of the first particular time period or the start of the second particular time period.

In some embodiments, movement of the part of the transducer-based device (e.g., transducer-based device 200, 300, or 400, in some embodiments) may occur during a determination of a rate of movement of the part of the transducer-based device, and during (a) the first particular time period, or (b) the second particular time period. In various embodiments, determination of the first rate of movement or the second rate of movement may be considered to be a predictive rate of movement of the part of the transducer-based device during a respective one of the first particular time period and the second particular time period. In some embodiments, in the first state (e.g., Figure 10A), the part of the transducer-based device moves through at least some of the locations during the duration of the first particular time period with the first rate of movement. In some embodiments, in the second state (e.g., Figure 10B), the part of the transducer-based device moves through at least some of the locations during the duration of the second particular time period with the second rate of movement.

Each of the first tissue-ablative energy (e.g., the energy delivered during the first state of Figure 10A, in some embodiments) and the second tissue-ablative energy (e.g., the energy delivered during the second state of Figure 10B, in some embodiments) may take different forms, according to various embodiments. For example, in some embodiments, each of the first tissue-ablative energy and the second tissue-ablative energy may be configured to be delivered with a continuous energy waveform. For example, an interrupted DC waveform or an uninterrupted AC waveform may be employed according to various embodiments. Thermal ablation delivering radiofrequency (RF) with continuous AC waveforms may be employed in some embodiments. In some embodiments, each of the first tissue-ablative energy and the second tissue-ablative energy may be configured to be delivered with an interrupted or discontinuous waveform. In some embodiments, at least in response to the first state, the data processing device system (e.g., data processing device system 110 or 310) may be configured at least by the program at least to cause delivery of the first tissue-ablative energy via a first plurality of discrete energy application sets during the duration of the first particular time period. In the example of Figure 10A, each discrete energy application set may correspond to a dose having 1.5 times ("1.5X") the energy of dose 900 (e.g., Figure 9A) corresponding to a respective one of the ovals shown in Figure 10A. The content, qualities, or parameters of a dose itself may be tailored to suit the needs of a particular application. In some embodiments, at least in response to the second state, the data processing device system may be configured at least by the program at least to cause delivery of the second tissue-ablative energy via a second plurality of discrete energy application sets during the duration of the second particular time period. In the example of Figure 10B, each discrete energy application set may correspond to an application of one dose having the same energy ("1X") as dose 900 corresponding to one of the ovals shown in Figure 10B. In some embodiments, the duration of the first particular time period is the same as the duration of the second particular time period (as shown, for instance, in the examples of Figure 10A and Figure 10B). In some embodiments, each of the first tissue-ablative energy and the second tissue-ablative energy is energy delivered via pulsed field ablation.

Each of the first and the second plurality of discrete energy application sets may take different forms, according to various applications and embodiments. For example, in thermal ablation applications, each discrete energy application set may take the form of an energy delivery via a duty cycled waveform. A duty cycle waveform includes a plurality of ON and OFF cycles. Duty cycle is usually expressed as the fraction of one period in which a signal is active (ON) with the period being the time it takes for a signal to complete an ON-and-OFF cycle. Duty cycle is commonly expressed as a percentage or a ratio. In PFA applications, each discrete energy application set may take form of a group of high voltage pulses configured to caused irreversible electroporation or pulsed field ablation of tissue. Such pulses may be monophasic or biphasic, in some embodiments, and may have varying pulse widths or pulse shapes and the same or varying inter-pulse gaps or spacing, according to various embodiments. In this regard, in some embodiments, the time interval between groups of high voltage pulses forming respective discrete energy application sets, and at least some of the respective embodiments, are typically orders of magnitude greater than the interval between pulses within any group of pulses, such that the difference between groups of pulses and pulses within a same group is easily determined.

According to various embodiments, the first energy waveform parameter set (e.g., such as that used to define each energy application in the example of Figure 10A, in some embodiments) defines one or more first parameters applicable to each discrete energy application set (e.g., corresponding to a respective oval in Figure 10A, in some embodiments) in the first plurality of discrete energy application sets, and the second energy waveform parameter set (e.g., such as that used to define each energy application in the example of Figure 10B, in some embodiments) defines one or more second parameters applicable to each discrete energy application set (e.g., corresponding to a respective oval in Figure 10B, in some embodiments) in the second plurality of discrete energy application sets. In some embodiments, each of at least one of the one or more first parameters is different than each of at least one of the one or more second parameters. In the examples of Figure 10A and Figure 10B, such a first parameter may define, or result in 1.5 times ("1.5X") the energy of dose 900 (e.g., Figure 9A) per dose or application in Figure 10A, and such a second parameter may define, or result in the 1 times ("1X") the energy of dose 900 per dose or application in Figure 10B, according to some embodiments. In some embodiments, the first energy waveform parameter set defines a first plurality of discrete energy application sets (e.g., corresponding to the four energy applications or doses (e.g., represented as ovals) in the example of Figure 10A, in some embodiments) to deliver the first tissue-ablative energy (e.g., the total energy of six times the energy of dose 900 applied in the example of Figure 10A, in some embodiments) during the duration of the first particular time period. In some embodiments, the second energy waveform parameter set defines a second plurality of discrete energy application sets (e.g., corresponding to the six energy applications or doses (represented as ovals) in the example of Figure 10B, in some embodiments) to deliver the second tissue-ablative energy (e.g., the total energy of six times the energy of dose 900 applied in the example of Figure 10B, in some embodiments) during the duration of the second particular time period. In some embodiments, each discrete energy application set of the first plurality of discrete energy application sets and each discrete energy application set of the second plurality of discrete energy application sets is configured to cause pulsed field ablation of tissue. In some embodiments associated with PFA applications in which delivery of a plurality of the discrete applications sets includes delivery of a plurality of high voltage pulse sets, the dose in some PFA-based embodiments may be considered to be the pulse count in a high voltage pulse set. For example, one dose 900 (e.g., Figure 9A) may apply 100 high voltage pulses, one dose at 1.5X the energy of dose 900 may apply 150 high voltage pulses, and one dose at 3X the energy of dose 900 may correspondingly apply 300 high voltage pulses, in some embodiments.

In some embodiments, each discrete energy application set in the first plurality of discrete energy application sets includes one or more discrete energy applications, and each discrete energy application set in the first plurality of discrete energy application sets includes the same total number of discrete energy applications as each of every other discrete energy application set in the first plurality of discrete energy application sets. For example, in some embodiments in which the first plurality of discrete energy application sets (e.g., corresponding to the four energy applications or doses shown as ovals in the example of Figure 10A, in some embodiments) is provided by a first plurality of PFA high voltage sets (each oval representing a dose shown in the example of Figure 10A being provided by a respective PFA high voltage set, in some embodiments), each high voltage pulse set that is transmitted includes the same number of pulses as every other high voltage pulse set that is transmitted. However, different numbers of pulses, different dose amounts, or other energy delivery characteristics may be different among the plurality of discrete energy application sets in some other embodiments. In this regard, in the example of Figure 10A, for instance, it may be preferable to increase the amount of energy delivered in the first discrete energy application set (e.g., corresponding to the leftmost dose shown in Figure 10A), the last discrete energy application set (e.g., corresponding to the right-most dose shown in Figure 10A), or both, as compared to the interior discrete energy application sets (e.g., corresponding to the two middle doses shown in Figure 10A), in some embodiments, since such first and last discrete energy application sets have less overlapping with adjacent discrete energy application sets (and, therefore, the corresponding tissue receives less total energy) compared to the interior discrete energy application sets.

Similarly, in some embodiments, each discrete energy application set in the second plurality of discrete energy application sets includes one or more discrete energy applications, and each discrete energy application set in the second plurality of discrete energy application sets includes the same total number of discrete energy applications as each of every other discrete energy application set in the second plurality of discrete energy application sets. For example, in some embodiments in which the second plurality of discrete energy application sets (e.g., corresponding to the six doses shown as ovals in the example of Figure 10B, in some embodiments) is provided by a second plurality of PFA high voltage sets (each dose shown in the example of Figure 10B being provided by a respective PFA high voltage set, in some embodiments), each high voltage pulse set that is transmitted includes the same number of pulses as every other high voltage pulse set that is transmitted. However, as discussed above, the numbers of pulses, the dose amounts, or other energy delivery characteristics may be different among the plurality of discrete energy application sets in some other embodiments.

In some embodiments, the first plurality of discrete energy application sets (e.g., a first plurality of PFA high voltage pulse sets) includes the same total number of discrete energy applications as the second plurality of discrete energy application sets (e.g., a second plurality of PFA high voltage pulse sets). Although the examples of Figure 10A and Figure 10B show four and six discrete energy application sets, respectively, applied over substantially the same time periods, some embodiments may provide the same total number of discrete energy applications for different rates of movement, e.g., in at least some embodiments in which the first particular time period (e.g., akin to first particular time period 1004 in Figure 10A) is different than the second particular time period (e.g., akin to the second particular time 1014 in Figure 10B). In some embodiments, the amount of energy delivered via the first plurality of discrete energy application sets during the duration of the first particular time period is substantially the same as the amount of energy delivered via the second plurality of discrete energy application sets during the duration of the second particular time period. For example, Figure 10A and Figure 10B each show a total amount of energy delivered of six times the energy of dose 900 (e.g., Figure 9A), although Figure 10A and Figure 10B show application of four and six doses, respectively. In some embodiments, delivery of the same amounts of energy may, in some embodiments, be achieved when the first plurality of discrete energy application sets includes the same total number of discrete energy applications as the second plurality of discrete energy application sets. In some embodiments, the amount of energy delivered via the first plurality of discrete energy application sets during the duration of the first particular time period is different than the amount of energy delivered via the second plurality of discrete energy application sets during the duration of the second particular time period. For example, although the examples of Figure 10A and Figure 10B show a same amount of total energy delivered, other embodiments may deliver different amounts of energy for different rates of movement. For example, in some embodiments, different total amounts of energy with different rates of motion may be employed in order to account for potentially non-linear phenomenon. In some examples, there may be a greater potential for greater risk of gaps between lesions at greater rates of motion, and the particular non-linear phenomenon relevant to these examples may be associated with one or more of the tolerance stack-up in tracking accuracy of the employed navigation system across different numbers of lesions, changes in tracking accuracy as a function of speed, or interaction effects related to a single direction of velocity being measured and the 2D geometry of the lesions being produced. Other examples of non-linear effects may include the temporal response of heating/cooling responses or physiological responses to applied doses that may, in some cases, be inherently non-linear.

In some embodiments, each discrete energy application set of at least one discrete energy application set in the first plurality of discrete energy application sets (e.g., a first plurality of PFA high voltage pulse sets) includes a different number of discrete energy applications compared to each discrete energy application set of at least one discrete energy application set in the second plurality of discrete energy application sets (e.g., a second plurality of PFA high voltage pulse sets). For instance, in the examples of Figure 10A and Figure 10B, in some embodiments, each dose (e.g., corresponding to a respective oval) in Figure 10A is associated with the application of 1.5 times ("1.5X") the energy of dose 900 (e.g., Figure 9A) (e.g., such 1.5X energy may be provided by a set of 150 discrete energy applications merely as an example in some embodiments), whereas in Figure 10B, each dose is associated with the application of 1 times ("1X") the energy of dose 900 (e.g., such 1X energy may be provided by a set of 100 discrete energy applications merely as an example in some embodiments).

In some embodiments, each discrete energy application set of at least one discrete energy application set in the first plurality of discrete energy application sets (e.g., a first plurality of PFA high voltage pulse sets) includes one or more discrete energy applications delivering a first particular amount of energy, and each discrete energy application set of at least one discrete energy application set in the second plurality of discrete energy application sets (e.g., a second plurality of PFA high voltage pulse sets) includes one or more discrete energy applications delivering a second particular amount of energy, the second particular amount of energy different than the first particular amount of energy. For example, referring back to the discussions above related to Figures 9, in PFA applications in which delivery of a plurality of the discrete energy application sets includes delivery of a plurality of high voltage pulse sets, various parameters can be varied to affect pulse energy in terms of tissue ablation. For instance, the high voltage pulse width duration (or a phase duration of a particular phase of a biphasic pulse when the high voltage pulses are biphasic in nature) may be made shorter (e.g., for the same pulse count and voltage) to produce a lower energy dose. The energy delivered by a high voltage pulse is dependent on the pulse width of the high voltage pulse. Similarly, PFA high voltage pulses employing a lower voltage (e.g., for the same pulse count) may be used to produce a lower energy dose. As described above with respect to Figure 9D, a catheter navigation system (e.g., at least Figures 2 or 3) may overlap 'lower energy' doses during movement of the transducer-based device (e.g., transducer-based device 200, 300, or 400, in some embodiments) to ensure enough additional pulses are applied at any given point on the tissue to still achieve a transmural lesion.

In some embodiments, (a) movement of the part of the transducer-based device (e.g., transducer-based device 200, 300, or 400, in some embodiments) occurs at least between the delivery of at least two discrete energy application sets in the first plurality of discrete energy application sets, (b) movement of the part of the transducer-based device occurs at least between the delivery of at least two discrete energy application sets in the second plurality of discrete energy application sets, or each of (a) and (b). For instance, in particular examples of Figure 10A and Figure 10B in which each dose is associated with at least a discrete energy application set, it is illustrated that movement of the part of the transducer-based device occurs between each of the discrete energy application sets (e.g., represented respectively by ovals), according to some embodiments. In some embodiments, movement of the part of the transducer-based device occurs during at least one of the deliveries of the at least two discrete energy application sets in the first plurality of discrete energy application sets. In some embodiments, movement of the part of the transducer-based device occurs during at least one of the deliveries of the at least two discrete energy application sets in the second plurality of discrete energy application sets. In some embodiments, in the event of (a), and as with a particular example of Figure 10A, the first energy waveform parameter set defines that each discrete energy application set of the at least two discrete energy application sets in the first plurality of discrete energy application sets includes a respective one or more particular discrete energy applications (e.g., one or more particular PFA high voltage pulses, in some embodiments). The respective one or more particular discrete energy applications of the at least the two discrete energy application sets in the first plurality of discrete energy application sets may be applied in an overlapping manner during the delivery of the first tissue-ablative energy (e.g., as illustrated by at least two overlapping ovals in a particular example of Figure 10A, in some embodiments). In some embodiments, in the event of (b), and as with a particular example of Figure 10B, the second energy waveform parameter set defines that each discrete energy application set of the at least two discrete energy application sets in the second plurality of discrete energy application sets includes a respective one or more particular discrete energy applications (e.g., one or more particular PFA high voltage pulses). The respective one or more particular discrete energy applications of the at least two discrete energy application sets in the second plurality of discrete energy application sets may be applied in an overlapping manner during the delivery of the second tissue-ablative energy (e.g., as illustrated by at least two overlapping ovals in a particular example of Figure 10B, in some embodiments).

For another particular example associated with Figure 9D in which each dose is associated with at least a discrete energy application set, at least two discrete energy application sets (e.g., two PFA high voltage pulse sets) may, in some embodiments, provide at least two of the overlapping doses 900. In some embodiments, in the event of (a), the first energy waveform parameter set defines that the at least two discrete energy application sets in the first plurality of discrete energy application sets include at least three discrete energy application sets in the first plurality of discrete energy application sets. In some embodiments, in the event of (b), the second energy waveform parameter set defines that the at least two discrete energy application sets in the second plurality of discrete energy application sets include at least three discrete energy application sets in the second plurality of discrete energy application sets. In some embodiments, each discrete energy application set of the at least three discrete energy application sets in the first plurality of discrete energy application sets includes a respective one or more particular discrete energy applications (e.g., one or more particular PFA high voltage pulses). In some embodiments, the respective one or more particular discrete energy applications of the at least three discrete energy application sets in the first plurality of discrete energy application sets may be applied in an overlapping manner during the delivery of the first tissue-ablative energy. In some embodiments, each discrete energy application set of the at least three discrete energy application sets in the second plurality of discrete energy application sets includes a respective one or more particular discrete energy applications (e.g., one or more particular PFA high voltage pulses). In some embodiments, the respective one or more particular discrete energy applications of the at least three discrete energy application sets in the second plurality of discrete energy application sets may be applied in an overlapping manner during the delivery of the second tissue-ablative energy.

In some embodiments, in the event of (a), (i.e., movement of the part of the transducer-based device between the delivery of at least two discrete energy application sets in the first plurality of discrete energy application sets) each discrete energy application set of the at least two discrete energy application sets (e.g., corresponding to at least two doses in the example of Figure 10A, in some particular embodiments) in the first plurality of discrete energy application sets is configured at least by the first energy waveform parameter set to deliver a respective amount of energy insufficient to produce a transmural tissue lesion in the bodily cavity. For example, each of at least two of the ovals in Figure 10A represents the delivery of 1.5 times the energy of dose 900 (e.g., Figure 9A), which may individually represent energy insufficient to produce a transmural tissue lesion. In some embodiments, in the event of (b), (i.e., movement of the part of the transducer-based device between the delivery of at least two discrete energy application sets in the first plurality of discrete energy application sets) each discrete energy application set of the at least two discrete energy application sets (e.g., corresponding to at least two doses in the example of Figure 10B, in some embodiments) in the second plurality of discrete energy application sets is configured at least by the second energy waveform parameter set to deliver a respective amount of energy insufficient to produce a transmural tissue lesion in the bodily cavity. For example, each of at least two of the ovals in Figure 10B represents the delivery of one (1) times ("1X") the energy of dose 900, which may individually represent energy insufficient to produce a transmural tissue lesion.

In some embodiments, in the event of (a), at least the at least two discrete energy application sets in the first plurality of discrete energy application sets are configured at least by the first energy waveform parameter set to collectively deliver energy sufficient to produce a transmural tissue lesion in the bodily cavity. For instance, in the example of Figure 10A in some particular embodiments, although each discrete energy application set applies 1.5 times the energy of dose 900, which may individually be insufficient to produce a transmural tissue lesion, the overlap regions 1006-1008 are subjected to three times the energy of dose 900, which may be sufficient to produce a transmural tissue lesion, according to some embodiments. Similarly, in some embodiments, in the event of (b), at least the at least two discrete energy application sets in the second plurality of discrete energy application sets are configured at least by the second energy waveform parameter set to collectively deliver energy sufficient to produce a transmural tissue lesion in the bodily cavity. For instance, in the example of Figure 10B in some particular embodiments, although each discrete energy application set applies 1 times the energy of dose 900, which may individually be insufficient to produce a transmural tissue lesion, the overlap regions 1016-1019 are subjected to three times the energy of dose 900, which may be sufficient to produce a transmural tissue lesion, according to some embodiments.

In this regard, in some embodiments, each discrete energy application set in the first plurality of discrete energy application sets (e.g., the plurality corresponding to four doses in the example of Figure 10A, in some particular embodiments) is configured at least by the first energy waveform parameter set to deliver a respective amount of energy (e.g., each dose in Figure 10A delivers 1.5 times the energy of dose 900, in some embodiments) insufficient to produce a transmural tissue lesion in the bodily cavity, and the discrete energy application sets of the first plurality of discrete energy application sets are configured at least by the first energy waveform parameter set to collectively deliver energy sufficient to produce a transmural tissue lesion in the bodily cavity (e.g., the collective energy delivered by the four discrete energy application sets in the example of Figure 10A in some particular embodiments produces a transmural tissue lesion, in some embodiments). Similarly, in some embodiments, each discrete energy application set in the second plurality of discrete energy application sets (e.g., the plurality corresponding to six doses in the example of Figure 10B, in some particular embodiments) is configured at least by the second energy waveform parameter set to deliver a respective amount of energy (e.g., each dose in Figure 10B delivers 1 times the energy of dose 900, in some embodiments) insufficient to produce a transmural tissue lesion in the bodily cavity, and the discrete energy application sets of the second plurality of discrete energy application sets are configured at least by the second energy waveform parameter set to collectively deliver energy sufficient to produce a transmural tissue lesion in the bodily cavity (e.g., the collective energy delivered by the six discrete energy application sets in the example of Figure 10B in some particular embodiments produces a transmural tissue lesion, in some embodiments).

Returning to Figure 8A, in some embodiments, broken line block 804 may be considered to represent a configuration of the data processing device system (e.g., data processing device system 110 or 310) (e.g., according to a program) to vary an energy waveform parameter set based at least on the rate of movement, determined according to block 803, of at least the part of the transducer-based device (e.g., transducer-based device 200, 300, or 400, in some embodiments). In some embodiments, this varying of the energy waveform parameter set may manifest as the different first and second energy waveform parameter sets referred to in blocks 804a, 804b, although other embodiments may vary such energy waveform parameter set in other manners.

In some embodiments, broken line block 804 may be considered to also represent a configuration of the data processing device system (e.g., data processing device system 110 or 310) (e.g., according to a program) to cause, via the input-output device system (e.g., input-output device system 120 or 320) and the transducer-based device (e.g., transducer-based device 200, 300, or 400, in some embodiments), delivery of tissue-ablative energy in accordance with the varied energy waveform parameter set, the tissue-ablative energy being configured to cause tissue ablation. In some embodiments, this delivery of tissue ablative energy according to block 804 may manifest as the delivery of the first tissue-ablative energy in accordance with block 804a or the delivery of the second tissue-ablative energy in accordance with block 804b. However, other embodiments may have such delivery of tissue ablative energy occur in one or more different manners.

In various embodiments, the rate of movement of the part of the transducer-based device (e.g., transducer-based device 200, 300, or 400, in some embodiments) may be dependent on specific user actions that control manipulation of the part of the transducer-based device (for example, manipulations required to deliver at least the part of the transducer-based device to the bodily cavity, and manipulations required to move at least the part of the transducer-based device in the bodily cavity). In some embodiments, the user may manually manipulate the transducer-based device to cause movement of at least the part of the transducer-based device. Variability in a rate of movement of at least the part of the transducer-based device may arise from these user-actions. In some embodiments, the variability in the rate of movement of at least the part of the transducer-based device may be undesired when a specific rate of movement is desired (for example, when targeting either the first rate of movement or the second rate of movement described above with respect to Figure 8A, blocks 804a, 804b). In some embodiments, the data processing device system (e.g., data processing device system 110 or 310) may provide user feedback regarding the rate of movement of at least the part of the transducer-based device, e.g., in order to inform the user of the present rate of movement or changes in the rate of movement so that appropriate adjustments or other remedies may be made.

In this regard, Figure 8B represents some alternate embodiments based on Figure 8A, such that Figure 8B is the same or similar to Figure 8A, except for the addition of blocks 805, 806, 807, and 808 in Figure 8B. Block 805 represents a configuration of the data processing device system (e.g., data processing device system 110 or 310) (e.g., according to a program) to provide, via the input-output device system (e.g., input-output device system 120 or 320), a user-feedback indication indicating the determined rate of movement (e.g., determined according to block 803 in some embodiments). The user-feedback indication indicating the determined rate of movement may take various forms according to various embodiments. For example, in some embodiments, speaker device system 334 or other audio output device system may provide audible feedback indicating the determined rate of movement. In some embodiments, display device system 332 may provide visual feedback indicating the determined rate of movement. Without limitation, various systems in the input-output device system may, according to various embodiments, provide the user-feedback indication indicating the determined rate of movement.

In some embodiments, the user-feedback referred to in block 805 may, in some embodiments, be provided in response to a first state in which the determined rate of movement of at least the part of the transducer-based device exceeds a first rate of movement threshold. Typical rates of motion for the ablating transducers of a cardiac catheter when manually manipulated inside a bodily cavity may range from 0-10 mm/s. In some embodiments, the first rate of movement threshold may correspond to a rate of movement value associated with the part of the transducer-based device (e.g., transducer-based device 200, 300, or 400, in some embodiments) that is desired to not exceed. In some embodiments, the first rate of movement threshold may correspond to an uppermost value in a range of rate of movement values associated with the transducer-based device (e.g., the uppermost value desired not to be exceeded). A desired rate of movement range associated with the transducer-based device may be, by way of non-limiting example, 4 mm/s to 6 mm/s in some embodiments, 3 mm/s to 7 mm/s in some embodiments, and 2 mm/s to 8 mm/s in some embodiments. A desire to not exceed the first rate of movement threshold may be motivated for various reasons. For example, exceeding the first rate of movement threshold during movement of at least the part of the transducer-based device relative to the tissue surface in the bodily cavity may lead to a lack of lesion transmurality, or in the extreme, to gaps in a desired contiguous lesion that is to be formed by the ablative energy delivered by the transducer-based device (for example, as described above with respect to Figure 9C. Maintaining the movement of at least the part of the transducer-based device at, or below (e.g., within a determined or predetermined amount) the first rate of movement threshold may, in various embodiments, allow the transducer-based device to deliver desired ablative energy doses during the movement while lessening the chance of lesion gaps and non-transmural lesions.

In some embodiments, the user-feedback referred to in block 805 may, in some embodiments, be provided in response to a second state in which the determined rate of movement of at least the part of the transducer-based device is below a second rate of movement threshold. In some embodiments, the second rate of movement threshold may correspond to a rate of movement value associated with the part of the transducer-based device (e.g., transducer-based device 200, 300, or 400, in some embodiments), that is desired to at least match, or exceed (e.g., within a determined or pre-determined amount). In some embodiments, the second rate of movement threshold may correspond to a lowermost value in a range of rates of movement values associated with the transducer-based device, (e.g., it being desired that the rate of movement of the part of the transducer-based device be not lower than the lowermost value). A desire that the rate of movement of the part of the transducer-based device be not lower than the second rate of movement threshold may be motivated for various reasons. For example, in some embodiments, having the rate of movement of at least the part of the transducer-based device relative to the tissue surface that is too slow may make it relatively difficult to balance application dosage with sufficient tissue ablation to ensure a transmural lesion, while limiting the risk of excessive energy concentration that may increase the risk of damage to non-targeted neighboring anatomical structures. In various embodiments, when the rate of movement of at least the part of the transducer-based device relative to the tissue surface is too slow, the chances of over-dosing increase. The user-feedback indication provided for this 'too slow' state may be considered a second user-feedback indication as compared to a user-feedback indication provided for the 'too fast' state, which may be referred to as a first user-feedback indication, and the second user-feedback indication may be provided in a manner that is the same, similar, or different from the provision of the first user-feedback indication.

Returning to Figure 8B, in some embodiments, broken line block 804 may be associated with a configuration of the data processing device system (e.g., data processing device system 110 or 310) (e.g., according to a program) at least to cause, during the movement, the transducer-based device (e.g., transducer-based device 200, 300, or 400, in some embodiments) to deliver tissue ablative energy (e.g., pulsed field ablation energy, in some embodiments) via the communicative connection between the input-output device system (e.g., input-output device system 120 or 320) and the transducer-based device. In this regard, in some embodiments, blocks 804a and 804b need not be present, and block 804 may merely be associated with the delivery of tissue ablative energy, e.g., in some embodiments, before, after, or contemporaneously with providing user-feedback according to one or more embodiments of block 805. For instance, block 805 may be associated with providing one or more user-feedback indications pertaining to the rate of movement of at least part of the transducer-based device (e.g., too fast or too slow), in order to assist the user in providing a proper rate of movement to facilitate balancing of the production of a continuous, transmural lesion with reducing risk of injury to neighboring non-targeted anatomical structures.

In some embodiments, the rate of movement of at least part of the transducer-based device may be monitored during the delivery of tissue ablative energy. In some embodiments, if it is determined that the rate of movement exceeds an upper bound threshold or is below a lower bound threshold during the delivery of tissue ablative energy, such delivery may be controlled or even stopped in situations where risk of excessive energy delivery is unacceptably high. In some embodiments, the user may be provided with an indication that at least some part of the intended-ablation region may need to be re-ablated (for example, if lesion transmurality is likely not to have occurred, or lesion gaps were likely to have occurred).

In this regard, in some embodiments, block 806 may be associated with a configuration of the data processing device system (e.g., data processing device system 110 or 310) (e.g., according to a program) at least to monitor (e.g., via location information received from a catheter navigation system via the input-output device system 120 or 320) the rate of movement of at least the part of the transducer-based device (e.g., transducer-based device 200, 300, or 400, in some embodiments), e.g., at least during the delivery of tissue-ablative energy according to block 804, in some embodiments. In some embodiments, block 807 in Figure 8B may be associated with a configuration of the data processing device system (e.g., according to a program) at least to control or modify, via the communicative connection between the input-output device system and the transducer-based device, the delivery of the pulsed field ablation energy based at least on the monitored rate of movement, e.g., in response to a state in which the determined rate of movement indicates a change in rate of movement beyond a threshold, e.g., in response to the first state in which the determined rate of movement (e.g., determined according to block 803) of at least the part of the transducer-based device exceeds the above-discussed first rate of movement threshold, or, e.g., in response to the second state in which the determined rate of movement (e.g., determined according to block 803) of at least the part of the transducer-based device is below the above-discussed second rate of movement threshold. In some embodiments, if the rate of movement is too fast, then the delivery of ablative energy may be stopped, e.g., to prevent gaps in continuity of a tissue lesion. In some embodiments, if the rate of movement is too slow, then the delivery of ablative energy may be stopped, e.g., to prevent excessive energy delivery conditions. In at least cases where ablative energy is stopped, a user re-ablate indication may be provided (e.g., per block 808 in Figure 8B) to indicate that a tissue region should be re-ablated. The user re-ablate indication may be communicated to a user via any system of the input-output device suitable for communicating the user re-ablate indication.

In some embodiments, location information (e.g., provided by a catheter navigation system (e.g., as described above with respect to Figure 2 and Figure 3)) may be used to control the transducer-based device (e.g., transducer-based device 200, 300, or 400, in some embodiments) in other manners to produce or otherwise control lesions. For example, a catheter navigation system (e.g., at least Figure 2 or Figure 3) may be employed to cause the transducer-based device to automatically deliver a dose of energy (e.g., at least dose 900, dose 903, or otherwise) when the catheter navigation system detects that at least part of the transducer-based device has reached a target. According to some embodiments, the term "automatically" in this context may refer to a data processing device system (e.g., data processing device system 110 or 310) initiating the delivery of energy from at least part of a transducer-based device in response to reaching of the target without requiring user instruction to do so at least at that time.

In this regard, Figure 8C represents a method or programmed configuration of the data processing device system, according to some embodiments, where block 802a in Figure 8C may be the same or similar to block 802 in Figure 8A or Figure 8B, in some embodiments. In some embodiments, block 802a represents a configuration of the data processing device system (e.g., data processing device systems 110 or 310) (e.g., according to a program) to receive, via an input-output device system (e.g., input-output device system 120 or 320), location information indicating a plurality of locations in a bodily cavity in response to movement of at least part of a transducer-based device (e.g., transducer-based device 200, 300, or 400, in some embodiments) in the bodily cavity. According to various embodiments, the location information may be provided by a catheter navigation system (e.g., as described above with respect to Figure 2 and 3). For example, the location information may be derived from a location signal set provided by a catheter navigation system in response to movement of at least part of transducer-based device, the location signal set indicating a plurality of locations (e.g., various locations in a bodily cavity). In some embodiments, the plurality of locations may be a plurality of locations that the part of the transducer-based device moves between. For example, in some embodiments, the part of the transducer-based device, may be a physical part of the transducer-based device (for example, a particular electrode or a particular transducer-based device, or a particular electrode set or transducer set of the transducer-based device), and the location information may indicate various locations that the part of the transducer-based device moves between.

In some embodiments, the part of the transducer-based device may be a non-transducer-based portion of the transducer-based device (e.g., transducer-based device 200, 300, or 400, in some embodiments), a non-electrode portion of the transducer-based device, or even a virtual or non-physical portion associated with the transducer-based device. For example, in the transducer-based device 300 of Figure 6, the part of the transducer-based device may be a calculated center or centroid of the quasi-spherical arrangement of transducers 306. Such center or centroid may be calculated by the data processing device system 110 or 310 based on the determined locations of various ones of the transducers 306 and pre-determined information related to their distance or radius from the center. In some embodiments, the location information received according to block 802a may indicate various locations that the non-transducer-based portion of the transducer-based device (e.g., transducer-based device 200, 300, or 400, in some embodiments), the non-electrode portion of the transducer-based device, or even the virtual or non-physical portion associated with the transducer-based device moves between. In some embodiments, the location information may indicate the locations of different parts or portions of the transducer-based device in response to movement of the part of the transducer-based device. For example, a first location of the plurality of locations may reflect a location of a first transducer of the transducer-based device at a first time during the movement of a part of the transducer-based device, and a second location of the plurality of locations may reflect a location of a second transducer of the transducer-based device at a second time during the movement of the part of the transducer-based device, the second transducer other than the first transducer, the second time other than the first time. In some embodiments, the location information may indicate the locations of different parts or portions of the transducer-based device that move in response to movement of the part of the transducer-based device.

Movement of at least the part of the transducer-based device (e.g., transducer-based device 200, 300, or 400, in some embodiments) may include translation of the transducer-based device, according to some embodiments. Movement of at least the part of the transducer-based device may include rotation of the transducer-based device, according to some embodiments. In some embodiments, each location of the plurality of locations is a location of the part of the transducer-based device determined relative to a tissue surface in the bodily cavity (for example, when the location signal set is referenced to a reference (e.g., reference device 252 (Figure 2) or reference device 257z (Figure 3)). In some embodiments, each of at least some of the plurality of locations corresponds to a respective one of a plurality of measured locations measured by a catheter navigation system (e.g., at least Figure 2 or Figure 3). In some embodiments, each of at least some of the plurality of locations does not directly correspond to a respective one of a plurality of measured locations directly measured by a catheter navigation system (e.g., at least Figure 2 or Figure 3). For example, at least one of the plurality of locations may correspond to a location interpolated from at least some of the plurality of measured locations measured by a catheter navigation system, or may correspond to an unmeasured location having some determined or predetermined spatial relationship with one or more of the plurality of measured locations.

In some embodiments, the part of the transducer-based device (e.g., transducer-based device 200, 300, or 400, in some embodiments) (e.g., referred to in block 802a) includes a particular part of the transducer-based device that is configured to be deliverable to a bodily cavity. In some embodiments, the part of the transducer-based device includes one or more transducers configured to cause ablation (e.g., transducers 220, 306, or 406, in some embodiments). In some embodiments, the part of the transducer-based device includes one or more transducers (e.g., transducers 220, 306, or 406 (or, e.g., 277 in the case of magnetic-field-based systems, in some embodiments)) configured, as the part of the transducer-based device is moved through a sequence of locations, to generate various location signal sets as detected strengths of the respective field(s), which the controller (e.g., controller 324, in some embodiments) or data processing device system (e.g., 110 or 310) may then be configured to utilize to generate three-dimensional location information of the part of the transducer-based device (e.g., transducer-based device 200, 300, or 400, in some embodiments).

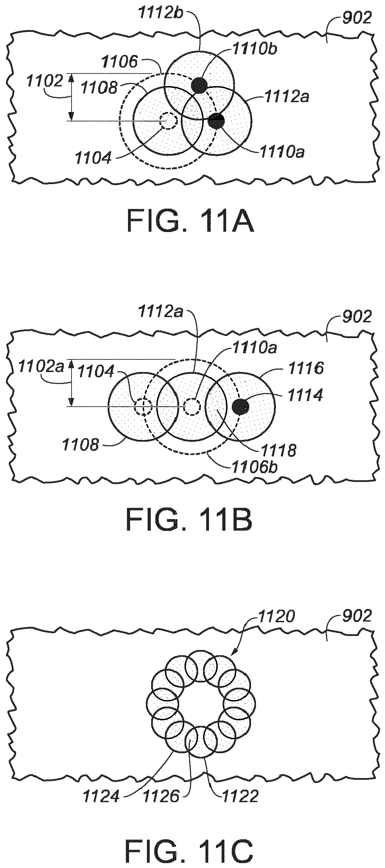

In Figure 8C, according to some embodiments, block 810 represents a configuration of the data processing device system (e.g., data processing device system 110 or 310) (e.g., according to a program) to determine, based at least on an analysis of at least part of the location information (e.g., received according to block 802a), target location information indicative of a target location set relative to a first particular location of the plurality of locations in the bodily cavity. In some embodiments, the target location information may represent a target distance, e.g., stored in memory device system 130 or 330, according to some embodiments. Figure 11A illustrates an example of such a target distance as a radius 1102 from a first particular location 1104. The first particular location 1104 may be a location that has been visited by the part of the transducer-based device, as determined, in some embodiments, from the location information received according to block 802a in Figure 8C. In some embodiments, the first particular location 1104 may be a location at which the part of the transducer-based device delivered energy, such delivery of energy being represented in the example of Figure 11A via circle 1108. From the target distance or radius 1102, a target location set, relative to the first particular location 1104, may be indicated or derived, such target location set represented in Figure 11A as a plurality of target locations along circumference 1106 having radius 1102, according to some embodiments.

Returning to Figure 8C, block 812 represents a configuration of the data processing device system (e.g., data processing device system 110 or, 310) (e.g., according to a program) to determine, based at least on an analysis of at least part of the location information, that at least a portion of the transducer-based device (e.g., transducer-based device 200, 300, or 400, in some embodiments) has reached a target location relative to the first particular location of the plurality of locations in the bodily cavity, the target location defined at least in part by the target location information and belonging to the target location set. For example, with input from a catheter navigation system (e.g., according to Figure 2 or Figure 3), the data processing device system may be configured to determine that the part of the transducer-based device has reached a target distance from the first particular location, according to some embodiments. In the context of Figure 11A, the data processing device system determines that the part of the transducer-based device has reached target location 1110a as being at a target distance corresponding to radius 1108 from first particular location 1104, according to some embodiments. In this regard, the target location 1110a is defined at least in part by the target location information, which, in the case of Figure 11A, includes target radius 1102.

According to various embodiments, the analysis of the at least part of the location information per block 810 may include determining of the first particular location as a particular location of the plurality of locations in the bodily cavity. For example, in some embodiments, the first particular location (e.g., first particular location 1104) may be determined as a particular location of a portion of the transducer-based device (e.g., transducer-based device 200, 300, or 400, in some embodiments) during which tissue ablation occurred (which may be represented by circle 1108). For example, in some embodiments, the first particular location may be determined as a particular location of the part of the transducer-based device during which tissue ablation occurred. In some embodiments, the first particular location may be determined as a particular location of the plurality of locations during which a delivery of tissue ablation energy by the transducer-based device occurred. In some embodiments, the first particular location (e.g., first particular location 1104) may be determined as a particular location of the plurality of locations during which a delivery of tissue ablation energy by the transducer-based device last occurred. In some embodiments, the first particular location may be determined as a particular location during which a delivery of tissue ablation energy by the transducer-based device is occurring. In some embodiments, the first particular location of the plurality of locations is a location of a previously ablated tissue region. For example, the first particular location may be a location of a particular transducer (e.g., transducer 206, 306, or 406, in some embodiments) that was employed to ablate tissue or at least deliver energy to such tissue, the particular transducer in contact with the tissue, according to some embodiments. In some embodiments, the first particular location is one of the plurality of locations in the bodily cavity corresponding to a previous delivery of tissue ablation energy by at least a portion of the transducer-based device prior to delivery of the particular tissue-ablative energy as per block 804c, discussed below, where tissue-ablative energy or other energy is delivered at the target location (e.g., target location 1110a).

In some embodiments, the first particular location of the plurality of locations may be determined as a particular location of the plurality of locations during which contact was detected between a portion of the transducer-based device (e.g., transducer-based device 200, 300, or 400, in some embodiments) and a tissue surface in the bodily cavity. For example, as a condition delivering tissue ablative energy or, in some embodiments, other energy, it may be desirable to ensure that the respective transducer(s) delivering such energy is or are in sufficient tissue contact. As discussed above, the respective transducer(s) themselves may provide such contact signals to the data processing device system (e.g., data processing device system 110 or 310) for determination of sufficient tissue contact. In some embodiments, the first particular location of the plurality of locations may be determined as a particular location of the plurality of locations during which contact was detected between the part of the transducer-based device and a tissue surface in the bodily cavity. In some embodiments, the first particular location of the plurality of locations may be determined as a particular location of the plurality of locations during which electrophysiological information was sensed by a transducer of the transducer-based device.

In some embodiments, the target location set determined as per block 810 may include at least one target location having a determined positioning relative to the first particular location of the plurality of locations. For example, the at least one target location, may in some embodiments, be defined by a determined, or predetermined distance from the first particular location of the plurality of locations, as with the radius 1102 and first particular location 1104 in the example of Figure 11A, which can provide any number of potential target locations along circumference 1106, including potential target location 1110a and potential target location 1110b, according to some embodiments. Some embodiments may be employed where relatively precise positioning is required (e.g., Figure 9C described above) and, may in some embodiments, be employed in various robotic medical instrument positioning systems. In some embodiments, the target location information indicative of a target location set relative to a first particular location of the plurality of locations in the bodily cavity defines a target distance from the first particular location of the plurality of locations in the bodily cavity. In some embodiments, unlike a pure radius or distance length, the defined target distance may specify a particular distance in a particular direction (i.e., a vector instead or merely a scalar value) relative to the first particular location of the plurality of locations. In some of these embodiments, a target location may be defined as a location reached when the particular distance in the particular direction relative to the first particular location of the plurality of locations has been traversed. In some embodiments, the defined target distance may specify a particular distance (e.g., irrespective of direction; i.e., a scalar value, such as the mere length of radius 1102, regardless of direction, in some embodiments) from the first particular location (e.g., first particular location 1104, in some embodiments) of the plurality of locations, the target location being defined as a location reached when the particular distance relative to the first particular location of the plurality of locations has been traversed. In some embodiments, the target location information defines the target location set as including at least one of a plurality of possible target locations, each of the possible target locations spaced from the first particular location of the plurality of locations in the bodily cavity by a target radius, such as with the radius 1102 and corresponding circumference 1106 defining the plurality of possible target locations in the example of Figure 11A. In some embodiments, the plurality of possible target locations may be a plurality of known possible locations that may be stored in a memory device system (e.g., memory device system 130 or 330), each of the possible target locations spaced from the first location of the plurality of locations by a determined or predetermined distance (e.g., radius). For example, instead of storing in memory the length of the radius 1102 in the example of Figure 11A, particular locations on the circumference 1106 may be stored in the memory, in some embodiments. In some embodiments, the plurality of possible target locations may be a plurality of possible locations that may not be known in advance of, or during, a movement of the portion of the transducer-based device (e.g., transducer-based device 200, 300, or 400, in some embodiments), but one of such possible target locations may be determined to have been reached when, e.g., the portion of the transducer-based device has moved by a magnitude of the radius from the first location of the plurality of locations. For example, if merely the radius 1102 and location 1104 are stored in the memory device system, the possible target locations along circumference 1106 are not necessarily known in advance, but one of such possible target locations is merely deemed to be reached when movement by a magnitude of the radius 1102 from location 1104 is determined to have been achieved, according to some embodiments. In some embodiments, a target location of the target location set is determined to have been reached when the portion of the transducer-based device has moved by a magnitude of the radius from the first location of the plurality of locations. In some embodiments, movement of the portion of the transducer-based device is essentially planar (e.g., movement in two-dimensional space) and the radius is a two-dimensional entity, e.g., as illustrated in the example of Figure 11A. In some embodiments, however, movement of the portion of the transducer-based device is in three-dimensional space and the radius or other definition of possible target location(s) is a three-dimensional entity (e.g., a spherical radius).

In some embodiments, the target location may be a second particular location of the plurality of locations in the bodily cavity, the second particular location other than the first particular location. In the example of Figure 11A, the target location 1110a may be a second particular location at which the part of the transducer-based device is, or has been located, and it is other than the first particular location 1104 according to some embodiments. In some embodiments, the target location may be a second particular location of the plurality of particular locations in the bodily cavity spaced by at least the target distance from the first particular location of the plurality of locations in the bodily cavity. For example, the target location 1110a in Figure 11A is spaced from the first particular location 1104 by the radius 1102. In some embodiments, the target location is not defined or assigned until the location information indicates that a second location of the plurality of locations has been reached, the second location of the plurality of locations spaced by at least the target distance from the first particular location of the plurality of locations. In some embodiments, the data processing device system (e.g., data processing device system 110 or 310) is configured at least by the program at least to determine the target location as a second particular location of the plurality of locations in the bodily cavity in response to the determination that at least the portion of the transducer-based device (e.g., transducer-based device 200, 300, or 400, in some embodiments) has reached the target distance (e.g., radius 1102 in the example of Figure 11A) from the first particular location (e.g., first particular location 1104 in the example of Figure 11A) of the plurality of locations in the bodily cavity. In some embodiments, the portion of the transducer-based device determined (e.g., per block 812 in Figure 8C) to reach the target location is the part of the transducer-based device associated with the received location information (e.g., per block 802a in Figure 8C). In some embodiments associated with block 802a in Figure 8C, the part of the transducer-based device may be a first particular transducer (e.g., a transducer 220, 306, 406 (or, e.g., 277 in the case of magnetic-field-based systems), in some embodiments). In some embodiments, at least the first particular location (e.g., first particular location 1104 in the example of Figure 11A) of the plurality of locations corresponds to a location, indicated by the location information, of the first particular transducer. In some embodiments associated with block 812 in which the portion of the transducer-based device is the part of the transducer-based device (e.g., the first particular transducer), the data processing device system (e.g., data processing device system 110 or 310) may be configured at least by the program at least to determine the target location as a second particular location (e.g., location 1110a in the example of Figure 11A) of the plurality of locations in the bodily cavity in response to the determination that the first particular transducer has reached the target distance from the first particular location of the plurality of locations in the bodily cavity.

In some embodiments, the portion of the transducer-based device (e.g., transducer-based device 200, 300, or 400, in some embodiments) determined (e.g., per block 812 in Figure 8C) to reach the target location is other than the part of the transducer-based device associated with the received location information (e.g., per block 802a in Figure 8C). For example, in some embodiments, the part of the transducer-based device associated with at least block 802a may be a first particular transducer (e.g., a transducer 220, 306, 406 (or, e.g., 277 in the case of magnetic-field-based systems), in some embodiments), and the portion of transducer-based device associated with at least block 812 may be second particular transducer (e.g., a transducer 220, 306, 406 (or, e.g., 277 in the case of magnetic-field-based systems), in some embodiments). In some embodiments associated with block 812 in which the portion of the transducer-based device is the second particular transducer, the data processing device system (e.g., data processing device system 110 or 310) may be configured at least by the program at least to determine the target location as a second particular location (e.g., location 1110a in the example of Figure 11A) of the plurality of locations in the bodily cavity in response to the determination that the second particular transducer has reached the target distance (e.g., radius 1102 in the example of Figure 11A) from the first particular location of the plurality of locations in the bodily cavity. In some embodiments, the first particular location (e.g., first particular location 1104 in the example of Figure 11A) of the plurality of locations is a location of the first particular transducer.

In some embodiments, the target location may correspond to one of a plurality of measured locations measured by a catheter navigation system (e.g., at least Figure 2 or Figure 3). In some embodiments, the target location does not directly correspond to any particular one of a plurality of measured locations measured by a catheter navigation system (e.g., at least Figure 2 or Figure 3). For example, the target location may correspond to a location interpolated from at least some of the plurality of measured locations measured by a catheter navigation system, or may in some embodiments, correspond to an unmeasured location having some determined or predetermined spatial relationship with one of the plurality of measured locations. In some embodiments, the portion of the transducer-based device determined (e.g., per block 812 in Figure 8C) to reach the target location is a first portion of the transducer-based device (e.g., transducer-based device 200, 300, or 400, in some embodiments), and the target location is a first target location. In some embodiments, the data processing device system (e.g., data processing device system 110 or 310) is configured at least by the program at least to determine that at least a second portion of the transducer-based device has reached a second target location, e.g., in response to or after the determination that at least the first portion of the transducer-based device has reached the first target location. For instance, if the first portion of the transducer-based device is considered a mathematical centroid of the shape of the spherical head of the transducer-based device 300 in Figure 6, it may be determined that such centroid has reached a first target location, and based on known geometries of the spherical head of the transducer-based device 300, it may further be determined that a second portion (e.g., a transducer of the transducer-based device 300) has reached a corresponding second target location, which may be relative to the first target location based on such known geometries.

In some embodiments, the data processing device system (e.g., data processing device system 110 or 310) may be configured, e.g., at least by program instructions associated with block 804c in Figure 8C, at least to cause, in response to determining that at least the first portion, the second portion, or both the first portion and the second portion of the transducer-based device (e.g., transducer-based device 200, 300, or 400, in some embodiments) has reached the respective target location(s), the transducer-based device to deliver at least a portion of particular tissue-ablative energy via the communicative connection between the input-output device system and the transducer-based device. In some embodiments, the first portion of the transducer-based device may include a first transducer (e.g., a transducer 220, 306, or 406, in some embodiments) of the transducer-based device and the second portion of the transducer-based device may include a second transducer (e.g., a transducer 220, 306, or 406, in some embodiments) of the transducer-based device. In some embodiments, in response to or after the determination that the first transducer has reached the first target location, the determination that the second transducer has reached the second target location is made. For example, similar to the above-discussed centroid example, the second transducer may have a known spatial positioning relative to the first transducer, according to some embodiments, and the determination that the first transducer has reached the first target location (e.g., location 1110a in the example of Figure 11A) may in turn lead to the determination that the second transducer has reached the second target location (e.g., location 1110b in the example of Figure 11A) according to some embodiment in which the second target location is related to the first target location by the known spatial positioning of the second transducer relative to the first transducer. In some embodiments, the second target location and the first target distance are spaced by a same distance from the first particular location of the plurality of locations (e.g., the distance associated with radius 1102 in some embodiments associated with Figure 11A). For another example, with respect to Figure 11B, the first portion of the transducer-based device may correspond to a first transducer determined to be at location 1110a at a first time, and then the second portion of the transducer-based device may correspond to a second transducer determined to be at location 1114 at a later second time after the first time. In some embodiments, the second transducer may be the first transducer (i.e., they may be the same transducer), but in other embodiments, they may be different transducers. In some embodiments, the second target location is determined based at least on an analysis of the location information (e.g., which may be received according to block 802a in some embodiments). In some embodiments, the second target location corresponds to a second particular location of the plurality of locations (e.g., per location information associated with block 802a, in some embodiments). In some embodiments, the second portion of the transducer-based device (e.g., the second transducer) delivers the at least the portion of the particular tissue-ablative energy at the second target location. In some embodiments, the first portion of the transducer-based device (e.g., the first transducer) delivers at least some of the particular tissue-ablative energy at the first target location. For instance, in some embodiments, the first transducer may deliver energy at the first target location 1110a in the example of Figure 11B, as illustrated by circle 1112a, and the second transducer may deliver energy at the second target location 1114, as illustrated by circle 1116. In some embodiments, each of the first portion of the transducer-based device (e.g., the first transducer) and the second portion of the transducer-based device (e.g., the second transducer) delivers at least some of the particular tissue-ablative energy (e.g., delivered as per block 804 in Figure 8C and illustrated by circles 1112a and 1116 in the example of Figure 11B, according to some embodiments). In some embodiments, the second portion of the transducer-based device (e.g., the second transducer) delivers all of the particular tissue-ablative energy. For instance, at least in some embodiments in which the first portion of the transducer-based device and the second portion of the transducer-based device are the same (e.g., are the same transducer, in some embodiments), the same portion may deliver all of the particular tissue-ablative energy (e.g., the same transducer may provide the energy associated with at least circle 1112a and circle 1116, in the example of Figure 11B, in some embodiments). In some other embodiments in which the first portion of the transducer-based device and the second portion of the transducer-based device are different portions (e.g., different transducers, in some embodiments), the different portions may deliver their own parts of particular tissue-ablative energy at respective target locations.

In some embodiments, the data processing device system (e.g., data processing device system 110 or 310) may be configured at least by the program at least to determine, as at least part of the determination (e.g., according to block 812 in Figure 8C, in some embodiments) that at least the portion of the transducer-based device (e.g., transducer-based device 200, 300, or 400, in some embodiments) has reached the target location (e.g., location 1110a in at least the examples of Figures 11A and 11B, in some embodiments) relative to the first particular location (e.g., location 1104 in at least the examples associated with Figures 11A and 11B, in some embodiments) of the plurality of locations in the bodily cavity, a presence of contact between the transducer-based device and a tissue surface in the bodily cavity. In this regard, in some embodiments, the delivery of energy (e.g., energy that may result, at least in part, in tissue ablation) may be conditioned upon the detection (e.g., by one or more transducers of the transducer-based device that is to deliver the energy, according to some embodiments) of sufficient contact with the target tissue. The presence of tissue contact may be determined in various ways including techniques described above in this disclosure.

In some embodiments, the data processing device system (e.g., data processing device system 110 or 310) may be configured at least by the program at least to determine that at least the portion of the transducer-based device has reached a target distance (e.g., the distance associated with radius 1102 or 1102a, in some embodiments) from a location of at least the part of the transducer-based device during a previous delivery of tissue ablation energy or a portion thereof. For example, in some embodiments, the part of the transducer-based device may be provided by a transducer (e.g., a transducer 220, 306, or 406, in some embodiments) configured to deliver tissue ablation energy, and the location of at least the part of the transducer-based device during the previous delivery of tissue ablation energy or a portion thereof is the location of the transducer during the previous delivery of tissue ablation energy or a portion thereof. In some embodiments, the location of at least the part of the transducer-based device during the previous delivery of tissue ablation energy or a portion thereof is the first particular location (e.g., location 1104 in at least the example associated with Figures 11A and 11B, in some embodiments) of the plurality of locations. In some embodiments, the location of at least the part of the transducer-based device during the previous delivery of tissue ablation energy or a portion thereof is other than the first particular location (e.g., location 1104 in at least the examples associated with Figures 11A and 11B, in some embodiments) of the plurality of locations. For instance, although the examples of Figure 11A and Figure 11B utilize a radius 1102 or radius 1102a to illustrate a target distance from first particular location 1104, respectively, other embodiments may base the target distance off of a different location or region of space associated with the transducer-based device (for example location 1110a in Figure 11B, which may be a location associated with a delivery of ablative energy other than (e.g., subsequent to) a previous delivery of ablative energy at the first particular location 1102 according to some embodiments). In some embodiments, the portion of the transducer-based device is the part of the transducer-based device (e.g., transducer-based device 200, 300, or 400, in some embodiments).

Referring back to Figure 8C, block 804c, which may be an example implementation of at least part of block 804 (in contrast to, e.g., blocks 804a and 804b in Figures 8A and 8B that may be another example implementation in some embodiments), represents, according to some embodiments, a configuration of the data processing device system (e.g., data processing device system 110 or 310) (e.g., according to a program) to cause, in response to the determination (e.g., per block 812) that at least the portion of the transducer-based device (e.g., transducer-based device 200, 300, or 400, in some embodiments) has reached the target location relative to the first particular location of the plurality of locations in the bodily cavity, the transducer-based device to deliver particular tissue-ablative energy via a communicative connection between the input-output device system (e.g., input-output device system 120 or 320) and the transducer-based device. In some embodiments, such delivery of the particular tissue-ablative energy is represented in at least the example of Figure 11B with circle 1112a. As discussed above at least with respect to Figure 9 and Figure 10, such energy may be in an amount that individually does or does not produce a transmural lesion, but multiple applications of energy (e.g., energy represented by circle 1108, circle 1112a, and circle 1116 in combination in at least the example of Figure 11B, in some embodiments) may collectively produce a transmural lesion, depending on embodiment. In some embodiments, the particular tissue-ablative energy is delivered after the determination that at least the portion of the transducer-based device has reached the target location relative to the first particular location of the plurality of locations in the bodily cavity has been made. In some embodiments, the particular tissue-ablative energy is delivered while the at least the portion of the transducer-based device is moving. In some embodiments, the data processing device system is configured at least by the program at least to cause the transducer-based device to deliver the particular tissue-ablative energy at the target location (e.g., location 1110a in the example of Figure 11B, in some embodiments) in response to determining that at least the portion of the transducer-based device has reached the target location. For example, the particular tissue-ablative energy may be delivered by a transducer (e.g., a transducer 220, 306, or 406, in some embodiments) of the transducer-based device, the transducer positioned at the target location. In some embodiments, the particular tissue-ablative energy is energy delivered via pulsed field ablation (e.g., pulsed field ablation pulses).

In some embodiments, the data processing device system (e.g., data processing device system 110 or 310) is configured at least by the program at least to cause the transducer-based device (e.g., transducer-based device 200, 300, or 400, in some embodiments) to deliver the particular tissue-ablative energy via a discrete energy application set, e.g., as discussed above with respect to at least Figure 10A and Figure 10B. In this regard, various discrete energy applications sets that may be employed in these embodiments have been described by way of non-limiting example above in this disclosure. In some embodiments, the discrete energy application set may be configured to cause pulsed field ablation of tissue.

In some embodiments, the portion of the transducer-based device (e.g., transducer-based device 200, 300, or 400, in some embodiments) is a first portion of the transducer-based device, the target location is a first target location (e.g., location 1110a at least in the example of Figure 11B, in some embodiments), and the discrete energy application set is a first discrete energy application set (e.g., represented by circle 1112a at least in the example of Figure 11B, in some embodiments). In some embodiments, the data processing device system (e.g., data processing device system 110 or 310) is configured at least by the program at least to determine, after at least the first portion of the transducer-based device has reached the first target location, and based at least on an analysis of at least part of the location information, that at least a second portion of the transducer-based device has reached a second target location (e.g., location 1114 in the example of Figure 11B, in some embodiments) relative to the first target location. The second portion of the transducer-based device may or may not be the same portion as the first portion of the transducer-based device, depending on embodiment. In some embodiments, the data processing device system is configured at least by the program at least to cause, in response to determining that at least the second portion of the transducer-based device has reached the second target location relative to the first target location, the transducer-based device to deliver a second discrete energy application set (e.g., represented by circle 1116 in the example of Figure 11B, in some embodiments) via the communicative connection between the input-output device system and the transducer-based device. For another example, with reference to Figure 9D, in some embodiments, a catheter navigation system (e.g., at least Figure 2 or Figure 3, in some embodiments) may be employed to cause the transducer-based device to automatically deliver a dose (e.g., dose 900, in some embodiments) when the catheter navigation system detects that at least part of the transducer-based device has reached a target location. In this regard, the first discrete energy application set may correspond to a first one of the doses delivered when the first target location is reached, and the second discrete energy application set may correspond to a second one of the delivered doses, the second dose delivered upon a determination that a second target location has been reached. In some embodiments, the second target location is determined relative to the first target location.

In some embodiments, the data processing device system (e.g., data processing device system 110 or 310) may be configured at least by the program at least to determine, as at least part of the determination that at least the second portion of the transducer-based device (e.g., transducer-based device 200, 300, or 400, in some embodiments) has reached the second target location (e.g., target location 1114 at least in the example of Figure 11B) relative to the first target location, that at least the second portion of the transducer-based device has reached a particular target distance (e.g., the length of radius 1102a in the example of Figure 11B, in some embodiments) from the first target location (e.g., target location 1110a at least in the example of Figure 11B).

In some embodiments, the data processing device system (e.g., data processing device system 110 or 310) may be configured at least by the program at least to (a) determine, as at least part of the determination that at least the first portion of the transducer-based device (e.g., transducer-based device 200, 300, or 400, in some embodiments) has reached the first target location relative to the first particular location of the plurality of locations in the bodily cavity, that at least the first portion of the transducer-based device has reached a first target distance (e.g., length of radius 1102, in some embodiments) from the first particular location of the plurality of locations in the bodily cavity, and (b) determine, as at least part of the determination that at least the second portion of the transducer-based device has reached the second target location relative to the first target location, that at least the second portion of the transducer-based device has reached a second target distance (e.g., length of radius 1102a, forming circumference 1106b when rotated 360 degrees, in some embodiments) from the first target location. In this regard, Figure 11B illustrates, according to some embodiments, a further movement of the transducer-based device beyond the state illustrated by the example of Figure 11A, with like references in Figure 11B corresponding to those in Figure 11A. Figure 11B adds a third location 1114 where a third application of energy represented by circle 1116 is applied. In this regard, in some embodiments, the data processing device system may be configured at least by the program at least to (a) determine, as at least part of the determination that at least the first portion of the transducer-based device has reached the first target location (e.g., location 1110a at least in the example of Figure 11B, in some embodiments) relative to the first particular location (e.g., location 1104 at least in the example of Figure 11B, in some embodiments) of the plurality of locations in the bodily cavity, that at least the first portion of the transducer-based device has reached a first target distance (e.g., the length of radius 1102, in some embodiments) from the first particular location (e.g., location 1104 at least in the example of Figure 11B, in some embodiments) of the plurality of locations in the bodily cavity, and (b) determine, as at least part of the determination that at least the second portion of the transducer-based device has reached the second target location (e.g., location 1114 in the example of Figure 11B, in some embodiments) relative to the first target location (e.g., location 1110a, in some embodiments), that at least the second portion of the transducer-based device has reached a second target distance (e.g., the length of radius 1102a, in some embodiments) from the first target location (e.g., location 1110a, in some embodiments). In some embodiments, the second target distance is the same as the first target distance (e.g., both distances being the length of radius 1102 or radius 1102a in the example of Figure 11B, according to some embodiments). However, this need not be the case and, in some embodiments, the second target distance may be different than the first target distance, e.g., depending on energy applied or tissue topography, in some embodiments. In some embodiments, a same target distance separates successive target locations associated with successive deliveries of tissue ablation energy. In some embodiments, the use of same target distances may be employed to establish a same, or substantially the same, degree of overlap between successively delivered doses.

In some embodiments, the data processing device system (e.g., data processing device system 110 or 310) may be configured at least by the program at least to cause the transducer-based device (e.g., transducer-based device 200, 300, or 400, in some embodiments) to deliver the first discrete energy application set (e.g., which may correspond to the ablation energy represented by circle 1112a in the example of Figure 11B, in some embodiments) at the first target location in response to determining that at least the first portion of the transducer-based device has reached the first target location. In some embodiments, the data processing device system may be configured at least by the program at least to cause the transducer-based device to deliver the second discrete energy application set (e.g., which may correspond to the ablation energy represented by circle 1116 in the example of Figure 11B, in some embodiments) at the second target location in response to determining that at least the second portion of the transducer-based device has reached the second target location.

In some embodiments, the second portion of the transducer-based device (e.g., transducer-based device 200, 300, or 400, in some embodiments) is the first portion of the transducer-based device. For example, both the first portion of the transducer-based device and the second portion of the transducer-based device may be provided by a same transducer (e.g., a transducer 220, 306, or 406, in some embodiments) of the transducer-based device. In such a case, Figure 11B may represent a sequence of three locations 1104, 1110, 1114 visited by the same transducer, in some embodiments. In some embodiments, the second portion of the transducer-based device is other than the first portion of the transducer-based device. For example, the first portion of the transducer-based device and the second transducer-based device may be provided by different transducers of the transducer-based device. In some embodiments, the location information indicates a plurality of locations in a bodily cavity in response to movement of at least part of a transducer-based device in the bodily cavity. In some embodiments, the part of the transducer-based device may be the second portion of the transducer-based device. In some embodiments, the part of the transducer-based device also may be the first portion of the transducer-based device.

In some embodiments, each of the first discrete energy application set (e.g., corresponding to the ablation energy represented by circle 1112a in the example of Figure 11B, in some embodiments) and the second discrete energy application set (e.g., corresponding to the ablation energy represented by circle 1116 in the example of Figure 11B, in some embodiments) is provided by one or more respective particular discrete energy applications, the one or more respective particular discrete energy applications of the first discrete energy application set and the one or more respective particular discrete energy applications of the second discrete energy application set applied to the same particular tissue region. For example, in some embodiments, each one or more energy application set in each of the first discrete energy application set and the second discrete energy application set may correspond to one or more PFA pulses, and each of the first discrete energy application set and the second discrete energy application energy set may be applied in an overlapping manner, such that the one or more PFA pulses of each of the first discrete energy application set and the second discrete energy application energy set are applied to a same particular tissue region. Such a same particular region may be represented by overlap region 1118 in the example of Figure 11B, in some embodiments. In this regard, the particular tissue region may be a particular region of the tissue surface of the bodily cavity that is exposed to the one or more PFA pulses from each of the first discrete energy application set and the second discrete energy application energy set, according to some embodiments.

In some embodiments, the data processing device system (e.g., data processing device system 110 or 310) may be configured at least by the program at least to cause the transducer-based device (e.g., transducer-based device 200, 300, or 400, in some embodiments) to deliver the first discrete energy application set (e.g., corresponding to the ablation energy represented by circle 1112a in the example of Figure 11B, in some embodiments) during a first time interval, and to cause the transducer-based device to deliver the second discrete energy application set (e.g., corresponding to the ablation energy represented by circle 1116 in the example of Figure 11B, in some embodiments) during a second time interval. In some embodiments, a duration of the second time interval is the same as a duration of the first time interval. In some embodiments, an amount of energy delivered by the first discrete energy application set during the duration of the first time interval is the same, or substantially the same, as an amount of energy delivered by the second discrete energy application set during the duration of the second time interval (e.g., as is the case in the examples of each of Figure 10A and Figure 10B, in some embodiments). For example, in some embodiments, it may be desired that respective doses delivered by the first discrete energy application set during the duration of the first time interval and the second discrete energy application set during the duration of the second time interval be part of a series of overlapping doses that are each equal in form, and whose overlapping is uniform in form. In some embodiments, the first discrete energy application set includes one or more discrete energy applications each delivering a first particular amount of energy, and the second discrete energy application set includes one or more discrete energy applications each delivering a second particular amount of energy, the second amount of energy the same as the first particular amount of energy (e.g., as is the case in the examples of each of Figure 10A and Figure 10B, in some embodiments). For example, in some embodiments, each one or more energy applications in each of the first discrete energy application set the second discrete energy application set may correspond to one or more PFA pulses, each of the pulses configured to deliver a same amount of energy. It is noted that various differences may exist among the pulse characteristics of various ones of the pulses (e.g., pulse width, amplitude), but that various combinations of the different characteristics may be employed to produce a delivery of a same amount of energy.

However, in some embodiments, an amount of energy delivered by the first discrete energy application set during the duration of the first time interval is different than an amount of energy delivered by the second discrete energy application set during the duration of the second time interval. For example, as discussed above with respect to at least Figure 10A, there may be a desire to deliver more energy in a first energy application, a last energy application, or both, as compared to an interior energy application, since the first and/or last energy application may not be overlapped on both sides by other energy applications like an interior energy application, according to some embodiments. Differences in the amount of energy delivered by each of the first discrete energy application set and the second discrete energy application set may be achieved in various manners. In some embodiments, the first discrete energy application set may include a different total number of discrete energy applications than the second discrete energy application set. In some embodiments, the first discrete energy application set may include one or more discrete energy applications (e.g., one or more PFA pulses) each delivering a first particular amount of energy, and the second discrete energy application set may include one or more discrete energy applications (e.g., one or more PFA pulses) each delivering a second particular amount of energy, the second particular amount of energy different than the first particular amount of energy.

In some embodiments, the data processing device system (e.g., data processing device system 110 or 310) may be configured at least by the program at least to cause the transducer-based device (e.g., transducer-based device 200, 300, or 400, in some embodiments) to deliver each of the first discrete energy application set and the second discrete energy application set to form at least part of a circumferential ablated tissue region in the bodily cavity. Figure 11C shows a contiguous circumferential ablated tissue region formed by a plurality of circumferential overlapping energy applications 1120, each of the overlapping lesions formed by the delivery of some dosage of ablative energy (e.g., provided by a discrete energy application set in some embodiments). According to various embodiments, each of the first discrete energy application set and the second discrete energy application set forms a respective part of a group of discrete energy application sets, each discrete application set in the group of discrete energy application sets configured to deliver a respective amount of energy insufficient to produce a transmural tissue lesion in the bodily cavity, but collectively, the discrete energy application sets of the group of the discrete energy application sets are configured to form a transmural tissue lesion, as discussed above at least with respect to Figures 10.

In some embodiments, the data processing device system (e.g., data processing device system 110 or 310) may be configured at least by the program at least to cause the transducer-based device (e.g., transducer-based device 200, 300, or 400, in some embodiments) to deliver a third discrete energy application set (e.g., represented by circle 1124 in the example of Figure 11C, in some embodiments) to form at least part of the circumferential ablated tissue region. In some embodiments, the data processing device system (e.g., data processing device system 110 or 310) may be configured at least by the program at least to cause the transducer-based device (e.g., transducer-based device 200, 300, or 400, in some embodiments) to deliver the first discrete energy application set (e.g., corresponding to the ablation energy represented by circle 1122 in the example of Figure 11C, in some embodiments) to start formation of the circumferential ablated tissue region in the bodily cavity, and to cause the transducer-based device to deliver the third discrete energy application set (e.g., represented by circle 1124 in the example of Figure 11C, in some embodiments) to conclude formation of the circumferential ablated tissue region in the bodily cavity. (The second discrete energy application set may be any of the other circles represented in the example of Figure 11C.) In some embodiments, the delivery of the first and the third discrete energy application sets are applied to a same tissue region of the bodily cavity. For example, in some embodiments, in which the first discrete energy application set is delivered to start formation of a circumferential ablated tissue region in the bodily cavity and the third discrete energy application set is delivered to conclude formation of the circumferential ablated tissue region in the bodily cavity, the third discrete energy application set may be delivered to a same tissue region of a tissue surface of the bodily cavity that at least the first discrete energy application set was applied to. Such same tissue region may be represented as overlap region 1126 in the example of Figure 11C, in some embodiments. In some embodiments, multiple discrete energy application sets (at least the first discrete energy application set and the second discrete energy application set) may have been applied to the region of the tissue surface prior to the delivery of the third discrete energy application set. In some embodiments, the delivery of the third discrete energy application set may deliver an amount of energy to the region of the tissue that the multiple discrete energy application sets were previously delivered to, that may result in undesired excessive energy (e.g., over-dosing) being applied to the region of the tissue surface (for example, as described above in this disclosure with various ones of Figures 9).

It should be noted that, although the overlap region 1126 and each of the other overlap regions shown in Figure 11C (as well as in Figure 11A and Figure 11B) have a particular size and are relatively smaller than the overlap regions shown in, e.g., Figure 10A and Figure 10B for purposes of clarity of illustration, other embodiments may have greater or otherwise different sizes of overlap regions. It should be noted that, although the overlap region 1126 and each of the other overlap regions shown in Figure 11C (as well as in Figure 11A and Figure 11B) are illustrated as pertaining to two overlapping doses, other embodiments may include various overlap regions pertaining to three or more overlapping doses.

Returning to Figure 11C, in some embodiments, delivery of the third discrete energy application set (e.g., corresponding to the ablation energy represented by circle 1124 in the example of Figure 11C, in some embodiments) delivers less energy than the energy delivered by at least the first discrete energy application set (e.g., corresponding to the ablation energy represented by circle 1122 in the example of Figure 11C, in some embodiments), for instance, depending on the amount of energy delivered by the first discrete energy application set, the time elapsed since the delivery of the first discrete energy application set, or the amount of overlap between the first and third discrete energy application sets. In some embodiments, the energy delivered by at least the first discrete energy application set (e.g., represented by circle 1122 in the example of Figure 11C, in some embodiments) may be reduced, e.g., when the data-processing device system 110, 310 determines that the third discrete energy application set (e.g., corresponding to the ablation energy represented by circle 1124 in the example of Figure 11C, in some embodiments) will eventually be applied to a same region of the tissue surface of the bodily cavity. Accordingly, it can be seen that respective energy deliveries may be balanced in various embodiments. Differences between the energy delivered by the first discrete energy application set and the energy delivered by the third discrete energy application set may be achieved in various manners according to various embodiments. In some embodiments, the first discrete energy application set may include a different total number of discrete energy applications than the third discrete energy application set. In some embodiments, the first discrete energy application set includes one or more discrete energy applications delivering a first particular amount of energy, and the third discrete energy application set includes one or more discrete energy applications delivering a second particular amount of energy, the second particular amount of energy different than the first particular amount of energy.

While some of the embodiments disclosed above are described with examples of cardiac mapping, ablation, or both, the same or similar embodiments may be used for mapping, ablating, or both, other bodily organs, for example with respect to the intestines, the bladder, or any bodily organ to which the devices of the present invention may be introduced.

Subsets or combinations of various embodiments described above can provide further embodiments.

These and other changes can be made to the invention in light of the above-detailed description. In general, in the following claims, the terms used should not be construed to limit the invention to the specific embodiments disclosed in the specification and the claims, but should be construed to include other transducer-based device systems including all medical treatment device systems and all medical diagnostic device systems in accordance with the claims. Accordingly, the invention is not limited by the disclosure.

Advantageous examples of the present disclosure can be phrased as follows:
1. A tissue ablation system comprising:
   an input-output device system;
   a memory device system storing a program; and
   a data processing device system communicatively connected to the input-output device system and the memory device system, the data processing device system configured at least by the program at least to:
      receive, via the input-output device system, location information indicating locations of at least part of a transducer-based device in a bodily cavity;
      cause, via the input-output device system and the transducer-based device, delivery of first tissue-ablative energy during a duration of a first particular time period in accordance with a first energy waveform parameter set at least in response to a first state in which at least part of the location information indicates at least a first rate of movement of the part of the transducer-based device in the bodily cavity, wherein the first tissue-ablative energy caused to be delivered during the duration of the first particular time period in accordance with the first energy waveform parameter set is configured to cause tissue ablation; and
      cause, via the input-output device system and the transducer-based device, delivery of second tissue-ablative energy during a duration of a second particular time period in accordance with a second energy waveform parameter set at least in response to a second state in which the at least part of the location information indicates at least a second rate of movement of the part of the transducer-based device in the bodily cavity, wherein the second tissue-ablative energy caused to be delivered during the duration of the second particular time period in accordance with the second energy waveform parameter set is configured to cause tissue ablation, the second energy waveform parameter set different than the first energy waveform parameter set, and the second rate of movement different than the first rate of movement.
2. The tissue ablation system of Example 1, wherein the data processing device system is configured at least by the program at least to determine a rate of movement of the part of the transducer-based device in the bodily cavity based at least on an analysis of the locations indicated by the received location information.
3. The tissue ablation system of Example 1 or 2, wherein the duration of the first particular time period is the same as the duration of the second particular time period.
4. The tissue ablation system of any one of Examples 1 to 3,
   wherein, in the first state, the part of the transducer-based device moves through at least some of the locations during the duration of the first particular time period, and
   wherein, in the second state, the part of the transducer-based device moves through at least some of the locations during the duration of the second particular time period.
5. The tissue ablation system of any one of Examples 1 to 4,
   wherein, in the first state, the part of the transducer-based device moves through at least some of the locations during the duration of the first particular time period with the first rate of movement, and
   wherein, in the second state, the part of the transducer-based device moves through at least some of the locations during the duration of the second particular time period with the second rate of movement.
6. The tissue ablation system of any one of Examples 1 to 5,
   wherein, at least in response to the first state, the data processing device system is configured at least by the program at least to cause delivery of the first tissue-ablative energy via a first plurality of discrete energy application sets during the duration of the first particular time period, and
   wherein, at least in response to the second state, the data processing device system is configured at least by the program at least to cause delivery of the second tissue-ablative energy via a second plurality of discrete energy application sets during the duration of the second particular time period.
7. The tissue ablation system of Example 6,
   wherein the first energy waveform parameter set defines one or more first parameters applicable to each discrete energy application set in the first plurality of discrete energy application sets,
   wherein the second energy waveform parameter set defines one or more second parameters applicable to each discrete energy application set in the second plurality of discrete energy application sets, and
   wherein each of at least one of the one or more first parameters is different than each of at least one of the one or more second parameters.
8. The tissue ablation system of Example 6 or 7, wherein the duration of the first particular time period is the same as the duration of the second particular time period.
9. The tissue ablation system of any one of Examples 1 to 8,
   wherein the first energy waveform parameter set defines a first plurality of discrete energy application sets to deliver the first tissue-ablative energy during the duration of the first particular time period, and
   wherein the second energy waveform parameter set defines a second plurality of discrete energy application sets to deliver the second tissue-ablative energy during the duration of the second particular time period.
10. The tissue ablation system of Example 9, wherein each discrete energy application set of the first plurality of discrete energy application sets and each discrete energy application set of the second plurality of discrete energy application sets is configured to cause pulsed field ablation of tissue.
11. The tissue ablation system of any one of Examples 6 to 10, wherein the first plurality of discrete energy application sets includes the same total number of discrete energy applications as the second plurality of discrete energy application sets.
12. The tissue ablation system of any one of Examples 6 to 11, wherein each discrete energy application set of at least one discrete energy application set in the first plurality of discrete energy application sets includes a different number of discrete energy applications compared to each discrete energy application set of at least one discrete energy application set in the second plurality of discrete energy application sets.
13. The tissue ablation system of any one of Examples 6 to 12,
   wherein each discrete energy application set in the first plurality of discrete energy application sets includes one or more discrete energy applications, and
   wherein each discrete energy application set in the first plurality of discrete energy application sets includes the same total number of discrete energy applications as each of every other discrete energy application set in the first plurality of discrete energy application sets.
14. The tissue ablation system of Example 13,
   wherein each discrete energy application set in the second plurality of discrete energy application sets includes one or more discrete energy applications, and
   wherein each discrete energy application set in the second plurality of discrete energy application sets includes the same total number of discrete energy applications as each of every other discrete energy application set in the second plurality of discrete energy application sets.
15. The tissue ablation system of any one of Examples 6 to 14, wherein each discrete energy application set of at least one discrete energy application set in the first plurality of discrete energy application sets includes one or more discrete energy applications, each delivering a first particular amount of energy, and each discrete energy application set of at least one discrete energy application set in the second plurality of discrete energy application sets includes one or more discrete energy applications, each delivering a second particular amount of energy, the second particular amount of energy different than the first particular amount of energy.
16. The tissue ablation system of any one of Examples 6 to 15, wherein (a) movement of the part of the transducer-based device in the bodily cavity occurs at least between the delivery of at least two discrete energy application sets in the first plurality of discrete energy application sets, (b) movement of the part of the transducer-based device in the bodily cavity occurs at least between the delivery of at least two discrete energy application sets in the second plurality of discrete energy application sets, or each of (a) and (b).
17. The tissue ablation system of Example 16, wherein:
   in the event of (a), the first energy waveform parameter set defines that each discrete energy application set of the at least two discrete energy application sets in the first plurality of discrete energy application sets includes a respective one or more particular discrete energy applications, the respective one or more particular discrete energy applications of the at least the two discrete energy application sets in the first plurality of discrete energy application sets applied in an overlapping manner during the delivery of the first tissue-ablative energy; and
   in the event of (b), the second energy waveform parameter set defines that each discrete energy application set of the at least two discrete energy application sets in the second plurality of discrete energy application sets includes a respective one or more particular discrete energy applications, the respective one or more particular discrete energy applications of the at least two discrete energy application sets in the second plurality of discrete energy application sets applied in an overlapping manner during the delivery of the second tissue-ablative energy.
18. The tissue ablation system of Example 17, wherein:
   in the event of (a), the first energy waveform parameter set defines that the at least two discrete energy application sets in the first plurality of discrete energy application sets comprise at least three discrete energy application sets in the first plurality of discrete energy application sets, and
   in the event of (b), the second energy waveform parameter set defines that the at least two discrete energy application sets in the second plurality of discrete energy application sets comprise at least three discrete energy application sets in the second plurality of discrete energy application sets.
19. The tissue ablation system of Example 17 or 18, wherein:
   in the event of (a), each discrete energy application set of the at least two discrete energy application sets in the first plurality of discrete energy application sets is configured at least by the first energy waveform parameter set to deliver a respective amount of energy insufficient to produce a transmural tissue lesion in the bodily cavity; and
   in the event of (b), each discrete energy application set of the at least two discrete energy application sets in the second plurality of discrete energy application sets is configured at least by the second energy waveform parameter set to deliver a respective amount of energy insufficient to produce a transmural tissue lesion in the bodily cavity.
20. The tissue ablation system of Example 19, wherein:
   in the event of (a), at least the at least two discrete energy application sets in the first plurality of discrete energy application sets are configured at least by the first energy waveform parameter set to collectively deliver energy sufficient to produce a transmural tissue lesion in the bodily cavity, and
   in the event of (b), at least the at least two discrete energy application sets in the second plurality of discrete energy application sets are configured at least by the second energy waveform parameter set to collectively deliver energy sufficient to produce a transmural tissue lesion in the bodily cavity.
21. The tissue ablation system of any one of Examples 6 to 20,
   wherein each discrete energy application set in the first plurality of discrete energy application sets is configured at least by the first energy waveform parameter set to deliver a respective amount of energy insufficient to produce a transmural tissue lesion in the bodily cavity, and the discrete energy application sets of the first plurality of discrete energy application sets are configured at least by the first energy waveform parameter set to collectively deliver energy sufficient to produce a transmural tissue lesion in the bodily cavity; and
   wherein each discrete energy application set in the second plurality of discrete energy application sets is configured at least by the second energy waveform parameter set to deliver a respective amount of energy insufficient to produce a transmural tissue lesion in the bodily cavity, and the discrete energy application sets of the second plurality of discrete energy application sets are configured at least by the second energy waveform parameter set to collectively deliver energy sufficient to produce a transmural tissue lesion in the bodily cavity.
22. The tissue ablation system of any one of Examples 1 to 21, wherein each of the first tissue-ablative energy and the second tissue-ablative energy is energy delivered via pulsed field ablation.
23. The tissue ablation system of any one of Examples 1 to 22, wherein the location information indicates the locations of the part of a transducer-based device relative to a tissue surface in the bodily cavity.
24. The tissue ablation system of any one of Examples 1 to 23, wherein the location information indicates the locations of the part of a transducer-based device relative to a reference device of a navigation system.
25. A method executed by a data processing device system according to a program stored by a communicatively connected memory device system, the data processing device system also communicatively connected to an input-output device system, and the method comprising:
   receiving, via the input-output device system, location information indicating locations of at least part of a transducer-based device;
   causing, via a communicative connection between the input-output device system and the transducer-based device, delivery of first tissue-ablative energy during a duration of a first particular time period in accordance with a first energy waveform parameter set at least in response to a first state in which at least part of the location information indicates at least a first rate of movement of the part of the transducer-based device, wherein the first tissue-ablative energy caused to be delivered during the duration of the first particular time period in accordance with the first energy waveform parameter set is configured to cause tissue ablation; and
   causing, via a communicative connection between the input-output device system and the transducer-based device, delivery of second tissue-ablative energy during a duration of a second particular time period in accordance with a second energy waveform parameter set at least in response to a second state in which the at least part of the location information indicates at least a second rate of movement of the part of the transducer-based device, wherein the second tissue-ablative energy caused to be delivered during the duration of the second particular time period in accordance with the second energy waveform parameter set is configured to cause tissue ablation, the second energy waveform parameter set different than the first energy waveform parameter set, and the second rate of movement different than the first rate of movement.
26. One or more computer-readable storage mediums storing a program executable by a data processing device system communicatively connected to an input-output device system, the program comprising:
   reception instructions configured to cause reception, via the input-output device system, of location information indicating locations of at least part of a transducer-based device in a bodily cavity;
   first delivery instructions configured to cause, via a communicative connection between the input-output device system and the transducer-based device, delivery of first tissue-ablative energy during a duration of a first particular time period in accordance with a first energy waveform parameter set at least in response to a first state in which at least part of the location information indicates at least a first rate of movement of the part of the transducer-based device in the bodily cavity, wherein the first tissue-ablative energy caused to be delivered during the duration of the first particular time period in accordance with the first energy waveform parameter set is configured to cause tissue ablation; and
   second delivery instructions configured to cause, via a communicative connection between the input-output device system and the transducer-based device, delivery of second tissue-ablative energy during a duration of a second particular time period in accordance with a second energy waveform parameter set at least in response to a second state in which the at least part of the location information indicates at least a second rate of movement of the part of the transducer-based device in the bodily cavity, wherein the second tissue-ablative energy caused to be delivered during the duration of the second particular time period in accordance with the second energy waveform parameter set is configured to cause tissue ablation, the second energy waveform parameter set different than the first energy waveform parameter set, and the second rate of movement different than the first rate of movement.
27. The one or more computer-readable storage mediums of Example 26, wherein the one or more computer-readable storage mediums are one or more non-transitory computer-readable storage mediums.
28. A tissue ablation system comprising:
   an input-output device system;
   a memory device system storing a program; and
   a data processing device system communicatively connected to the input-output device system and the memory device system, the data processing device system configured at least by the program at least to:
      receive, via the input-output device system, location information indicating a plurality of locations in a bodily cavity in response to movement of at least part of a transducer-based device in the bodily cavity;
      determine, based at least on an analysis of at least part of the location information, target location information indicative of a target location set relative to a first particular location of the plurality of locations in the bodily cavity;
      determine, based at least on an analysis of at least part of the location information, that at least a portion of the transducer-based device has reached a target location relative to the first particular location of the plurality of locations in the bodily cavity, the target location defined at least in part by the target location information and belonging to the target location set; and
      cause, in response to the determination that at least the portion of the transducer-based device has reached the target location relative to the first particular location of the plurality of locations in the bodily cavity, the transducer-based device to deliver particular tissue-ablative energy via a communicative connection between the input-output device system and the transducer-based device.
29. The tissue ablation system of Example 28, wherein the data processing device system is configured at least by the program at least to determine, as at least part of the determination that at least the portion of the transducer-based device has reached the target location relative to the first particular location of the plurality of locations in the bodily cavity, that at least the portion of the transducer-based device has reached a target distance from the first particular location of the plurality of locations in the bodily cavity.
30. The tissue ablation system of Example 28 or 29, wherein the target location information defines a target distance from the first particular location of the plurality of locations in the bodily cavity.
31. The tissue ablation system of Example 29 or 30, wherein the target location is a second particular location of the plurality of particular locations in the bodily cavity spaced by at least the target distance from the first particular location of the plurality of locations in the bodily cavity.
32. The tissue ablation system of any one of Examples 29 to 31, wherein the data processing device system is configured at least by the program at least to determine the target location as a second particular location of the plurality of locations in the bodily cavity in response to the determination that at least the portion of the transducer-based device has reached the target distance from the first particular location of the plurality of locations in the bodily cavity.
33. The tissue ablation system of any one of Examples 28 to 32, where the portion of the transducer-based device is the part of the transducer-based device.
34. The tissue ablation system of any one of Examples 28 to 33, wherein the target location information defines the target location set as a plurality of possible target locations, each of the possible target locations spaced from the first particular location of the plurality of locations in the bodily cavity by a target radius.
35. The tissue ablation system of any one of Examples 28 to 34, wherein the data processing device system is configured at least by the program at least to determine, as at least part of the determination that at least the portion of the transducer-based device has reached the target location relative to the first particular location of the plurality of locations in the bodily cavity, a presence of contact between the transducer-based device and a tissue surface in the bodily cavity.
36. The tissue ablation system of Example 35, wherein the data processing device system is configured at least by the program at least to cause the transducer-based device to deliver the particular tissue-ablative energy also in response to determining the presence of the contact between the transducer-based device and the tissue surface in the bodily cavity.
37. The tissue ablation system of any one of Examples 28 to 36, wherein the first particular location of the plurality of locations is a location of a previously ablated tissue region.
38. The tissue ablation system of any one of Examples 28 to 37, wherein the first particular location is one of the plurality of locations in the bodily cavity corresponding to a previous delivery of tissue ablation energy by the transducer-based device prior to delivery of the particular tissue-ablative energy.
39. The tissue ablation system of any one of Examples 28 to 38, wherein the first particular location is one of the plurality of locations in the bodily cavity corresponding to a previous delivery of tissue ablation energy by the portion of the transducer-based device prior to delivery of the particular tissue-ablative energy.
40. The tissue ablation system of any one of Examples 28 to 39, wherein the data processing device system is configured at least by the program at least to determine that at least the portion of the transducer-based device has reached a target distance from a location of at least the part of the transducer-based device during a previous delivery of tissue ablation energy.
41. The tissue ablation system of Example 40, wherein the portion of the transducer-based device is the part of the transducer-based device.
42. The tissue ablation system of any one of Examples 28 to 41, wherein the target location is a second particular location of the plurality of locations in the bodily cavity, the second particular location other than the first particular location.
43. The tissue ablation system of any one of Examples 28 to 42, wherein the data processing device system is configured at least by the program at least to cause the transducer-based device to deliver the particular tissue-ablative energy at the target location in response to determining that at least the portion of the transducer-based device has reached the target location.
44. The tissue ablation system of any one of Examples 28 to 43, wherein the data processing device system is configured at least by the program at least to cause the transducer-based device to deliver the particular tissue-ablative energy via a discrete energy application set.
45. The tissue ablation system of Example 44, wherein the discrete energy application set is configured to cause pulsed field ablation of tissue.
46. The tissue ablation system of Example 44 or 45, wherein the portion of the transducer-based device is a first portion of the transducer-based device, wherein the target location is a first target location, and the discrete energy application set is a first discrete energy application set, and wherein the data processing device system is configured at least by the program at least to:
   determine, after at least the first portion of the transducer-based device has reached the first target location, and based at least on an analysis of at least part of the location information, that at least a second portion of the transducer-based device has reached a second target location relative to the first target location; and
   cause, in response to determining that at least the second portion of the transducer-based device has reached the second target location relative to the first target location, the transducer-based device to deliver a second discrete energy application set via the communicative connection between the input-output device system and the transducer-based device.
47. The tissue ablation system of Example 46, wherein the second portion of the transducer-based device is the first portion of the transducer-based device.
48. The tissue ablation system of Example 47, wherein the part of the transducer-based device is the second portion of the transducer-based device, which also is the first portion of the transducer-based device.
49. The tissue ablation system of any one of Examples 46 to 48, wherein the data processing device system is configured at least by the program at least to:
   cause the transducer-based device to deliver the first discrete energy application set at the first target location in response to determining that at least the first portion of the transducer-based device has reached the first target location, and
   wherein the data processing device system is configured at least by the program at least to cause the transducer-based device to deliver the second discrete energy application set at the second target location in response to determining that at least the second portion of the transducer-based device has reached the second target location.
50. The tissue ablation system of any one of Examples 46 to 49, wherein the data processing device system is configured at least by the program at least to cause the transducer-based device to deliver the first discrete energy application set during a first time interval, and to cause the transducer-based device to deliver the second discrete energy application set during a second time interval, a duration of the second time interval being the same as a duration of the first time interval.
51. The tissue ablation system of any one of Examples 46 to 50, wherein the first discrete energy application set includes a different total number of discrete energy applications than the second discrete energy application set.
52. The tissue ablation system of any one of Examples 46 to 51, wherein the first discrete energy application set includes one or more discrete energy applications, each delivering a first particular amount of energy, and the second discrete energy application set includes one or more discrete energy applications, each delivering a second particular amount of energy, the second particular amount of energy the same as the first particular amount of energy.
53. The tissue ablation system of any one of Examples 46 to 52, wherein the first discrete energy application set includes one or more discrete energy applications, each delivering a first particular amount of energy, and the second discrete energy application set includes one or more discrete energy applications, each delivering a second particular amount of energy, the second particular amount of energy different than the first particular amount of energy.
54. The tissue ablation system of any one of Examples 46 to 53, wherein each of the first discrete energy application set and the second discrete energy application set includes one or more respective particular discrete energy applications, the one or more respective particular discrete energy applications of the first discrete energy application set and the one or more respective particular discrete energy applications of the second discrete energy application set applied to the same particular tissue region.
55. The tissue ablation system of Example 54, wherein each of the first discrete energy application set and the second discrete energy application set forms a respective part of a group of discrete energy application sets, each discrete energy application set in the group of discrete energy application sets configured to deliver a respective amount of energy insufficient to produce a transmural tissue lesion in the bodily cavity, and wherein the discrete energy application sets in the group of the discrete energy application sets are configured to collectively deliver energy sufficient to produce a transmural tissue lesion in the bodily cavity.
56. The tissue ablation system of Example 55, wherein the data processing device system is configured at least by the program at least to cause the transducer-based device to deliver each of the first discrete energy application set and the second discrete energy application set to form at least part of a circumferential ablated tissue region in the bodily cavity.
57. The tissue ablation system of Example 56, wherein the data processing device system is configured at least by the program at least to cause the transducer-based device to deliver a third discrete energy application set to form at least part of the circumferential ablated tissue region, and wherein the data processing device system is configured at least by the program at least to cause the transducer-based device to deliver the first discrete energy application set to start formation of the circumferential ablated tissue region in the bodily cavity, and to cause the transducer-based device to deliver the third discrete energy application set to conclude formation of the circumferential ablated tissue region in the bodily cavity.
58. The tissue ablation system of Example 57, wherein the first discrete energy application set includes a different total number of discrete energy applications than the third discrete energy application set.
59. The tissue ablation system of Example 57 or 58, wherein the first discrete energy application set includes one or more discrete energy applications, each delivering a first particular amount of energy, and the third discrete energy application set includes one or more discrete energy applications, each delivering a second particular amount of energy, the second particular amount of energy different than the first particular amount of energy.
60. The tissue ablation system of any one of Examples 28 to 59, wherein the particular tissue-ablative energy is energy delivered via pulsed field ablation.
61. A method executed by a data processing device system according to a program stored by a communicatively connected memory device system, the data processing device system also communicatively connected to an input-output device system, and the method comprising:
   receiving, via the input-output device system, location information indicating a plurality of locations in response to movement of at least part of a transducer-based device;
   determining, based at least on an analysis of at least part of the location information, target location information indicative of a target location set relative to a first particular location of the plurality of locations;
   determining, based at least on an analysis of at least part of the location information, that at least a portion of the transducer-based device has reached a target location relative to the first particular location of the plurality of locations, the target location defined at least in part by the target location information and belonging to the target location set; and
   causing, in response to the determination that at least the portion of the transducer-based device has reached the target location relative to the first particular location of the plurality of locations, the transducer-based device to deliver particular tissue-ablative energy via a communicative connection between the input-output device system and the transducer-based device.
62. One or more computer-readable storage mediums storing a program executable by a data processing device system communicatively connected to an input-output device system, the program comprising:
   reception instructions configured to cause reception, via the input-output device system, of location information indicating a plurality of locations in a bodily cavity in response to movement of at least part of a transducer-based device in the bodily cavity;
   first determination instructions configured to cause a determination, based at least on an analysis of at least part of the location information, of target location information indicative of a target location set relative to a first particular location of the plurality of locations in the bodily cavity;
   second determination instructions configured to cause a determination, based at least on an analysis of at least part of the location information, that at least a portion of the transducer-based device has reached a target location relative to the first particular location of the plurality of locations in the bodily cavity, the target location defined at least in part by the target location information and belonging to the target location set; and
   delivery instructions configured to cause, in response to the determination that at least the portion of the transducer-based device has reached the target location relative to the first particular location of the plurality of locations in the bodily cavity, the transducer-based device to deliver particular tissue-ablative energy via a communicative connection between the input-output device system and the transducer-based device.
63. The one or more computer-readable storage mediums of Example 62, wherein the one or more computer-readable storage mediums are one or more non-transitory computer-readable storage mediums.
64. A pulsed field ablation system comprising:
   an input-output device system;
   a memory device system storing a program; and
   a data processing device system communicatively connected to the input-output device system and the memory device system, the data processing device system configured at least by the program at least to:
      receive, via the input-output device system, location information indicating movement of at least part of a transducer-based device through a plurality of locations in a bodily cavity during a particular time period;
      cause the transducer-based device, via a communicative connection between the input-output device system and the transducer-based device, to deliver pulsed field ablation energy at least during part of the particular time period;
      determine, based at least on an analysis of at least part of the location information, a rate of movement of at least the part of the transducer-based device; and
      provide, via the input-output device system, a first user-feedback indication in response to a first state in which the determined rate of movement of at least the part of the transducer-based device exceeds a first rate of movement threshold.
65. The pulsed field ablation system of Example 64, wherein the data processing device system is configured at least by the program at least to provide, via the input-output device system, a second user-feedback indication in response to a second state in which the determined rate of movement of at least the part of the transducer-based device is below a second rate of movement threshold.
66. The pulsed field ablation system of Example 64 or 65, wherein the data processing device system is configured at least by the program at least to cause, during the movement, the transducer-based device to deliver the pulsed field ablation energy via the communicative connection between the input-output device system and the transducer-based device.
67. The pulsed field ablation system of Example 65 or 66, wherein the data processing device system is configured at least by the program at least to control or modify, via the communicative connection between the input-output device system and the transducer-based device, the delivery of the pulsed field ablation energy in response to a state in which the determined rate of movement indicates a change in rate of movement beyond a threshold.
68. The pulsed field ablation system of any one of Examples 64 to 67, wherein the data processing device system is configured at least by the program at least to provide, via the input-output device system and at least in response to the first state in which the determined rate of movement of at least the part of the transducer-based device exceeds the first rate of movement threshold, a user re-ablate indication indicating that a tissue region should be re-ablated.
69. A method executed by a data processing device system according to a program stored by a communicatively connected memory device system, the data processing device system also communicatively connected to an input-output device system, and the method comprising:
   receiving, via the input-output device system, location information indicating movement of at least part of a transducer-based device through a plurality of locations during a particular time period;
   causing the transducer-based device, via a communicative connection between the input-output device system and the transducer-based device, to deliver pulsed field ablation energy at least during part of the particular time period;
   determining, based at least on an analysis of at least part of the location information, a rate of movement of at least the part of the transducer-based device; and
   providing, via the input-output device system, a first user-feedback indication in response to a first state in which the determined rate of movement of at least the part of the transducer-based device exceeds a first rate of movement threshold.
70. One or more computer-readable storage mediums storing a program executable by a data processing device system communicatively connected to an input-output device system, the program comprising:
   reception instructions configured to cause reception, via the input-output device system, of location information indicating movement of at least part of a transducer-based device through a plurality of locations in a bodily cavity during a particular time period;
   delivery instructions configured to cause the transducer-based device, via a communicative connection between the input-output device system and the transducer-based device, to deliver pulsed field ablation energy at least during part of the particular time period;
   determination instructions configured to cause determination, based at least on an analysis of at least part of the location information, of a rate of movement of at least the part of the transducer-based device; and
   user-feedback instructions configured to cause provision, via the input-output device system, of a first user-feedback indication in response to a first state in which the determined rate of movement of at least the part of the transducer-based device exceeds a first rate of movement threshold.
71. The one or more computer-readable storage mediums of Example 70, wherein the one or more computer-readable storage mediums are one or more non-transitory computer-readable storage mediums.
72. A pulsed field ablation system comprising:
   an input-output device system;
   a memory device system storing a program; and
   a data processing device system communicatively connected to the input-output device system and the memory device system, the data processing device system configured at least by the program at least to:
      receive, via the input-output device system, location information indicating movement of at least part of a transducer-based device through a plurality of locations in a bodily cavity during a particular time period;
      cause the transducer-based device, via a communicative connection between the input-output device system and the transducer-based device, to deliver pulsed field ablation energy at least during part of the particular time period;
      determine, based at least on an analysis of the location information, a rate of movement of at least the part of the transducer-based device; and
      provide, via the input-output device system, a user-feedback indication indicating the determined rate of movement.
73. A method executed by a data processing device system according to a program stored by a communicatively connected memory device system, the data processing device system also communicatively connected to an input-output device system, and the method comprising:
   receiving, via the input-output device system, location information indicating movement of at least part of a transducer-based device through a plurality of locations during a particular time period;
   causing the transducer-based device, via a communicative connection between the input-output device system and the transducer-based device, to deliver pulsed field ablation energy at least during part of the particular time period;
   determining, based at least on an analysis of the location information, a rate of movement of at least the part of the transducer-based device; and
   providing, via the input-output device system, a user-feedback indication indicating the determined rate of movement.
74. One or more computer-readable storage mediums storing a program executable by a data processing device system communicatively connected to an input-output device system, the program comprising:
   reception instructions configured to cause reception, via the input-output device system, of location information indicating movement of at least part of a transducer-based device through a plurality of locations in a bodily cavity during a particular time period;
   delivery instructions configured to cause the transducer-based device, via a communicative connection between the input-output device system and the transducer-based device, to deliver pulsed field ablation energy at least during part of the particular time period;
   determination instructions configured to cause determination, based at least on an analysis of the location information, of a rate of movement of at least the part of the transducer-based device; and
   user-feedback instructions configured to cause provision, via the input-output device system, of a user-feedback indication indicating the determined rate of movement.
75. The one or more computer-readable storage mediums of Example 74, wherein the one or more computer-readable storage mediums are one or more non-transitory computer-readable storage mediums.
76. A computer program product comprising program code portions for performing the steps of a method according to any one of Examples 25, 61, 69 and 73, when the computer program product is executed by a computing device.
77. The computer program product of Example 76, stored on one or more computer readable storage mediums.

## Claims

1. A tissue ablation system comprising:
an input-output device system;
a memory device system storing a program; and
a data processing device system communicatively connected to the input-output device system and the memory device system, the data processing device system configured at least by the program at least to:
receive, via the input-output device system, location information indicating a plurality of locations in a bodily cavity in response to movement of at least part of a transducer-based device in the bodily cavity;
determine, based at least on an analysis of at least part of the location information, target location information indicative of a target location set relative to a first particular location of the plurality of locations in the bodily cavity;
determine, based at least on an analysis of at least part of the location information, that at least a portion of the transducer-based device has reached a target location relative to the first particular location of the plurality of locations in the bodily cavity, the target location defined at least in part by the target location information and belonging to the target location set; and
cause, in response to the determination that at least the portion of the transducer-based device has reached the target location relative to the first particular location of the plurality of locations in the bodily cavity, the transducer-based device to deliver particular tissue-ablative energy via a communicative connection between the input-output device system and the transducer-based device.

2. The tissue ablation system of Claim 1, wherein the data processing device system is configured at least by the program at least to determine, as at least part of the determination that at least the portion of the transducer-based device has reached the target location relative to the first particular location of the plurality of locations in the bodily cavity, that at least the portion of the transducer-based device has reached a target distance from the first particular location of the plurality of locations in the bodily cavity.

3. The tissue ablation system of Claim 1 or 2, wherein the target location information defines a target distance from the first particular location of the plurality of locations in the bodily cavity.

4. The tissue ablation system of any one of Claims 1 to 3, where the portion of the transducer-based device is the part of the transducer-based device.

5. The tissue ablation system of any one of Claims 1 to 4, wherein the target location information defines the target location set as a plurality of possible target locations, each of the possible target locations spaced from the first particular location of the plurality of locations in the bodily cavity by a target radius.

6. The tissue ablation system of any one of Claims 1 to 5, wherein the data processing device system is configured at least by the program at least to determine, as at least part of the determination that at least the portion of the transducer-based device has reached the target location relative to the first particular location of the plurality of locations in the bodily cavity, a presence of contact between the transducer-based device and a tissue surface in the bodily cavity.

7. The tissue ablation system of Claim 6, wherein the data processing device system is configured at least by the program at least to cause the transducer-based device to deliver the particular tissue-ablative energy also in response to determining the presence of the contact between the transducer-based device and the tissue surface in the bodily cavity.

8. The tissue ablation system of any one of Claims 1 to 7, wherein the first particular location is one of the plurality of locations in the bodily cavity corresponding to a previous delivery of tissue ablation energy by the transducer-based device prior to delivery of the particular tissue-ablative energy.

9. The tissue ablation system of any one of Claims 1 to 8, wherein the data processing device system is configured at least by the program at least to cause the transducer-based device to deliver the particular tissue-ablative energy at the target location in response to determining that at least the portion of the transducer-based device has reached the target location.

10. The tissue ablation system of any one of Claims 1 to 9, wherein the data processing device system is configured at least by the program at least to cause the transducer-based device to deliver the particular tissue-ablative energy via a discrete energy application set.

11. The tissue ablation system of Claim 10, wherein the portion of the transducer-based device is a first portion of the transducer-based device, wherein the target location is a first target location, and the discrete energy application set is a first discrete energy application set, and wherein the data processing device system is configured at least by the program at least to:
determine, after at least the first portion of the transducer-based device has reached the first target location, and based at least on an analysis of at least part of the location information, that at least a second portion of the transducer-based device has reached a second target location relative to the first target location; and
cause, in response to determining that at least the second portion of the transducer-based device has reached the second target location relative to the first target location, the transducer-based device to deliver a second discrete energy application set via the communicative connection between the input-output device system and the transducer-based device.

12. The tissue ablation system of Claim 11, wherein the data processing device system is configured at least by the program at least to:
cause the transducer-based device to deliver the first discrete energy application set at the first target location in response to determining that at least the first portion of the transducer-based device has reached the first target location, and
wherein the data processing device system is configured at least by the program at least to cause the transducer-based device to deliver the second discrete energy application set at the second target location in response to determining that at least the second portion of the transducer-based device has reached the second target location.

13. The tissue ablation system of Claim 11 or 12, wherein the first discrete energy application set includes one or more discrete energy applications, each delivering a first particular amount of energy, and the second discrete energy application set includes one or more discrete energy applications, each delivering a second particular amount of energy, the second particular amount of energy the same as the first particular amount of energy.

14. The tissue ablation system of Claim 11 or 12, wherein the first discrete energy application set includes one or more discrete energy applications, each delivering a first particular amount of energy, and the second discrete energy application set includes one or more discrete energy applications, each delivering a second particular amount of energy, the second particular amount of energy different than the first particular amount of energy.

15. The tissue ablation system of any one of Claims 11 to 14, wherein each of the first discrete energy application set and the second discrete energy application set includes one or more respective particular discrete energy applications, the one or more respective particular discrete energy applications of the first discrete energy application set and the one or more respective particular discrete energy applications of the second discrete energy application set applied to the same particular tissue region,
wherein each of the first discrete energy application set and the second discrete energy application set forms a respective part of a group of discrete energy application sets, each discrete energy application set in the group of discrete energy application sets configured to deliver a respective amount of energy insufficient to produce a transmural tissue lesion in the bodily cavity, and wherein the discrete energy application sets in the group of the discrete energy application sets are configured to collectively deliver energy sufficient to produce a transmural tissue lesion in the bodily cavity, and
wherein the data processing device system is configured at least by the program at least to cause the transducer-based device to deliver each of the first discrete energy application set and the second discrete energy application set to form at least part of a circumferential ablated tissue region in the bodily cavity.
